# EUROPEAN PATENT SPECIFICATION

(11) **EP 1 594 878 B1**
(45) Date of publication and mention of the grant of the patent: **04.06.2008**
(21) Application number: 04706630.3
(22) Date of filing: 30.01.2004
(51) Int. Cl.: C07D 493/22, A01N 43/00

(54) **AVERMECTIN MONOSACCHARIDE DERIVATIVES HAVING PESTICIDAL PROPERTIES**
AVERMECTINMONOSACCHARIDDERIVATE MIT PESTIZIDEN EIGENSCHAFTEN
DERIVES MONOSACCHARIDES D'AVERMECTINE PRESENTANT DES PROPRIETES PESTICIDES

(30) Priority: 31.01.2003 GB 0302309
(43) Date of publication of application: 16.11.2005
(73) Proprietor: Merial Limited, Harlow, Essex CM19 5TG (GB)
(72) Inventor: PITTERNA, Thomas,Syngenta Crop Protection AG, CH-4053 Basel (CH); MAIENFISCH, Peter,Syngenta Crop Protection AG, CH-4058 Basel (CH); MURPHY KESSABI, Fiona,Syngenta Corp Protection AG, CH-4058 Basel (CH); TOBLER, Hans,Syngenta Crop Protection AG, CH-4058 Basel (CH); CASSAYRE, Jérôme,Syngenta Corp Protection, CH-4058 Basel (CH); QUARANTA, Laura,Syngenta Crop Protection AG, CH-4058 Basel (CH)
(74) Representative: Clyde-Watson, Zöe
(86) International application number: PCT/EP2004/000899
(87) International publication number: WO 2004/067534

(56) References cited:
- EP-A- 0 259 688
- EP-A- 0 266 131
- EP-A- 0 340 849
- WO-A-93/15099
- WO-A-02/068442

## Description

The present invention provides: (1) a compound of the general formula (I) 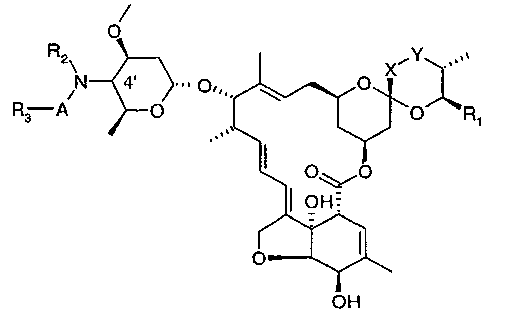 in which
A is -C(=Z)-, -O-C(=Z)-, -S-C(=Z)-, -NR₄-C(=Z)-, -SO₂-, -O-SO₂-, -NR₄-SO₂- or a bond,
X-Y is -CH=CH- or -CH₂-CH₂-;
Z is O or S;
R₁ is C₁-C₁₂alkyl, C₃-C₈cycloalkyl or C₂-C₁₂alkenyl;
R₂ and R₃:
   (a) are independently from each other, selected from H, C₁-C₁₂alkyl, C₂-C₁₂alkenyl, C₂-C₁₂alkynyl, C₃-C₁₂cycloalkyl, C₅-C₁₂cycloalkenyl, aryl, heterocyclyl and 2-cyano-2-C₁-C₁₂alkoxyimino; wherein the C₁-C₁₂alkyl, C₂-C₁₂alkenyl, C₂-C₁₂alkynyl, C₃-C₁₂cycloalkyl, C₅-C₁₂cycloalkenyl, aryl, heterocyclyl and C₁-C₁₂alkoxy radicals may be unsubstituted or mono- to pentasubstituted, depending on the substitution possibilities; or
   (b) together are a three- to seven membered alkylene or alkenylene bridge, which is unsubstituted or mono to tri-substituted; and wherein, optionally, one of the methylene groups of the three- to seven membered alkylene or alkenylene bridge is replaced by O, NR₄, S, S(=O) or SO₂; or
   (c) when A is a bond, together are =N⁺=N⁻;
R₄ is H, C₁-C₈alkyl, hydroxy-C₁-C₈alkyl, C₃-C₈cycloalkyl, C₂-C₈alkenyl, C₂-C₈alkynyl, phenyl, benzyl -C(=O)R₅, or -CH₂-C(=O)-R₅;
in which the substituents of the alkyl, alkenyl, alkynyl, alkylene, alkenylene, cycloalkyl, cycloalkenyl, aryl, heterocyclyl and alkoxy radicals as defined for R₁, R₂, R₃ and R₄ are selected from the group consisting of OH; =O; halogen; C₁-C₂haloalkyl; -N₃; CN; SCN; NO₂; C₃-C₈cycloalkyl that is unsubstituted or substituted by one to three of any of methyl groups, =O, OH, =S, or SH; norbornylenyl; C₃-C₈halocycloalkyl; C₁-C₁₂alkoxy, which may be substituted with a substituent selected from hydroxy, -N₃, -N(R₈)₂ wherein the two R₈ are independent of each other, and hydroxy; halo-C₁-C₁₂alkoxy; C₃-C₈cycloalkoxy; C₁-C₁₂alkylthio; C₃-C₈cycloalkylthio; C₁-C₁₂haloalkylthio; C₁-C₁₂alkyl-sulfinyl; C₃-C₈cycloalkylsulfinyl; C₁-C₁₂haloalkylsulfinyl; C₃-C₈halocycloalkylsulfinyl; C₁-C₁₂alkylsulfonyl; C₃-C₈cycloalkylsulfonyl; C₁-C₁₂haloalkylsulfonyl; C₃-C₈halocycloalkylsulfonyl; C₂-C₈alkenyl; C₂-C₈alkynyl; -N(R₈)₂, wherein the two R₈ are independent of each other; -C(=O)R₅; -O-C(=O)R₆; -NHC(=O)R₅; -N(CH₃)C(=O)R₅; -S-C(=S)R₆; -P(=O)(OC₁-C₆alkyl)₂; -S(=O)₂R₉; -NH-S(=O)₂R₉; -OC(=O)-C₁-C₆alkyl-S(=O)₂R₉; Si(R₈)₃; aryl; benzyl; heterocyclyl; aryloxy; benzyloxy; heterocyclyloxy; arylthio; benzylthio; and heterocyclylthio; wherein the aryl, heterocyclyl, aryloxy, benzyloxy, heterocyclyloxy, arylthio, benzylthio and heterocyclylthio radicals are unsubstituted or, depending on the possibilities of the substitution on the ring, are mono- to pentasubstituted by substituents selected from the group consisting of OH, halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, dimethylamino-C₁-C₆alkoxy, C₂-C₈alkenyl, C₂-C₈alkynyl, phenoxy, phenyl-C₁-C₆alkyl, methylenedioxy, -C(=O)R₅, -O-C(=O)-R₆, -NH-C(=O)R₆, -N(R₈)₂ wherein the two R₈ are independent of each other, C₁-C₆alkylsulfinyl, C₃-C₈cycloalkylsulfinyl, C₁-C₆haloalkylsulfinyl, C₃-C₈halocycloalkylsulfinyl, C₁-C₆alkylsulfonyl, C₃-C₈cycloalkylsulfonyl, C₁-C₆haloalkylsulfonyl and C₃-C₈halocycloalkylsulfonyl;
R₅ is H, OH, SH, -N(R₈)₂wherein the two R₈ are independent of each other, C₁-C₂₄alkyl, C₂-C₁₂alkenyl, C₁-C₈hydroxyalkyl, C₁-C₁₂haloalkyl, C₁-C₁₂alkoxy, C₁-C₁₂haloalkoxy, C₁-C₆alkoxy-C₁-C₆alkyl, C₁-C₆alkoxy-C₁-C₆alkoxy, phenoxy-C₁-C₆alkoxy, C₁-C₆alkoxy-C₁-C₆alkoxy-C₁-C₆alkyl, C₁-C₁₂alkylthio, C₂-C₈alkenyloxy, C₂-C₈alkynyloxy, C₁-C₆cycloalkoxy, NH-C₁-C₆alkyl-C(=O)R₇, -N(C₁-C₆alkyl)-C₁-C₆alkyl-C(=O)-R₇, -O-C₁-C₂alkyl-C(=O)R₇, -C₁-C₆alkyl-S(=O)₂R₉, aryl, benzyl, heterocyclyl, aryloxy, benzyloxy, or heterocyclyloxy; or aryl, benzyl, heterocyclyl, aryloxy, benzyloxy or heterocyclyloxy, each of which are mono- to trisubstituted in the ring independently of one another by halogen, nitro, C₁-C₆alkyl, C₁-C₆alkoxy, C₁-C₆haloalkyl or C₁-C₆haloalkoxy;
R₆ is H, C₁-C₂₄alkyl, C₁-C₁₂haloalkyl, C₁-C₁₂hydroxyalkyl, C₂-C₈alkenyl, C₂-C₈alkynyl, C₁-C₆alkoxy-C₁-C₆alkyl, (NR₈)₂ wherein the two R₈ are independent of each other, -C₁-C₆alkyl-C(=O)R₈, -C₁-C₆alkyl-S(=O)₂R₉, aryl, benzyl, and heterocyclyl; or aryl, benzyl or heterocyclyl which, depending on the possibilities of the substitution on the ring, are mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio and C₁-C₁₂haloalkylthio;
R₇ is H, OH, C₁-C₂₄alkyl that is optionally substituted with OH or -S(=O)₂-C₁-C₆alkyl, C₁-C₁₂alkenyl, C₂-C₁₂alkynyl, C₁-C₁₂alkoxy, C₁-C₆ alkoxy-C₁-C₆alkyl, C₁-C₆alkoxy-C₁-C₆alkoxy, C₂-C₈alkenyloxy, aryl, aryloxy, benzyloxy, heterocyclyl, heterocyclyloxy or -N(R₈)₂ wherein the two R₈ are independent of each other;
R₈ H, C₁-C₆alkyl that is optionally substituted with one to five substituents selected from the group consisting of halogen, C₁-C₆alkoxy, hydroxy and cyano, C₁-C₈-cycloalkyl, aryl, benzyl or heteroaryl; or aryl, benzyl or heteroaryl, which, depending on the possibilities of the substitution on the ring, are mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio and C₁-C₁₂haloalkylthio; and
R₉ is H, C₁-C₆alkyl that is optionally substituted with one to five substituents selected from the group consisting of halogen, C₁-C₆alkoxy, hydroxy and cyano, aryl, benzyl or heteroaryl; or aryl, benzyl or heteroaryl, which, depending on the possibilities of the substitution on the ring, are mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio and C₁-C₁₂haloalkylthio;
or, if appropriate, an E/Z isomer, E/Z isomer mixture and/or tautomer thereof, in each case in free form or in salt form, provided that R₃ is C₁-C₁₂alkyl, C₂-C₁₂alkenyl, C₂-C₁₂alkynyl, C₃-C₁₂cycloalkyl, C₅-C₁₂cycloalkenyl, aryl, heterocyclyl and 2-cyano-2-C₁-C₁₂alkoxyimino; each of which are unsubstituted or mono- to pentasubstituted, depending on the substitution possibilities, when the compound has a configuration of *(R)* at the 4'-position, X-Y is -CH₂-CH₂-, R₁ is sec-butyl or isopropyl, R₂ is H and A is a bond; or R₃ is H, C₂-C₁₂alkyl, C₂-C₁₂alkenyl, C₂-C₁₂alkynyl, C₃-C₁₂cycloalkyl, C₅-C₁₂cycloalkenyl, aryl, heterocyclyl and 2-cyano-2-C₁-C₁₂alkoxyimino; each of which are unsubstituted or mono- to pentasubstituted, depending on the substitution possibilities, when the compound has a configuration of *(R)* at the 4'-position, X-Y is -CH₂-CH₂-, R₁ is sec-butyl or isopropyl, R₂ is H and A is -C(=O); a process for preparing these compounds, their isomers and tautomers and the use of these compounds, their isomers and tautomers; pesticidal compositions whose active compound is selected from these compounds, their isomers and tautomers; intermediates for the preparation of the said compounds of the formula (I); methods for the preparation of the compounds of the formula (I); and a method for controlling pests using these compositions.

Hereinbefore and hereinafter, the configuration at the 4'-position, in the instance it is not specified, of the compounds of the present invention formulae can be *(S)* as well as *(R)*.

The literature proposes certain macrolide compounds for controlling pests. However, the biological properties of these known compounds are not entirely satisfactory, and, as a consequence, there is still a need for providing further compounds having pesticidal properties, in particular for the control of insects and representatives of the order Acarina. According to the invention, this object is achieved by providing the present compounds of the formula (I).

The compounds claimed according to the invention are derivatives of Avermectin. Avermectins are known to the person skilled in the art. They are a group of structurally closely related pesticidally active compounds which are obtained by fermenting a strain of the microorganism Streptomyces avermitilis. Derivatives of Avermectins can be obtained by conventional chemical syntheses.

The Avermectins which can be obtained from Streptomyces avermitilis are referred to as A1a, A1b, A2a, A2b, B1a, B1b, B2a and B2b. The compounds referred to as "A" and "B" have a methoxy radical and an OH group, respectively, in the 5-position. The "a" series and the "b" series are compounds in which the substituent R₁ (in position 25) is a sec-butyl radical and an isopropyl radical, respectively. The number 1 in the name of the compounds means that atoms 22 and 23 are linked by double bonds; the number 2 means that they are linked by a single bond and that the C atom 23 carries an OH group. The above nomenclature is adhered to in the description of the present invention to denote the specific structure type in the not naturally occurring Avermectin derivatives according to the invention which corresponds to the naturally occurring Avermectin. What is for instance claimed according to the invention are monosaccharide derivatives of compounds of the B1 series, in particular mixtures of monosaccharide derivatives of Avermectin B1, especially B1a and B1b, and also related compounds having a single bond between atoms 22 and 23, along with derivatives having other substituents in the 25-position.

Some of the compounds of the formula (I) can be present as tautomers. Accordingly, hereinabove and hereinbelow, the compounds of the formula (I) are, if appropriate, also to be understood as including the corresponding tautomers, even if the latter are not specifically mentioned in each case.

The compounds of formula (I) and, where applicable, their tautomers can form salts, for example acid addition salts. These acid addition salts are formed, for example, with strong inorganic acids, such as mineral acids, for example sulfuric acid, a phosphoric acid or a hydrohalic acid, with strong organic carboxylic acids, such as unsubstituted or substituted, for example halo-substituted, C₁-C₄alkanecarboxylic acids, for example acetic acid, unsaturated or saturated dicarboxylic acids, for example oxalic acid, malonic acid, maleic acid, fumaric acid or phthalic acid, hydroxycarboxylic acids, for example ascorbic acid, lactic acid, malic acid, tartaric acid or citric acid, or benzoic acid, or with organic sulfonic acids, such as unsubstituted or substituted, for example halo-substituted, C₁-C₄alkane- or arylsulfonic acids, for example methane- or p-toluene-sulfonic acid. Compounds of formula (I) that have at least one acidic group can furthermore form salts with bases. Suitable salts with bases are, for example, metal salts, such as alkali metal salts or alkaline earth metal salts, for example sodium, potassium or magnesium salts, or salts with ammonia or with an organic amine, such as morpholine, piperidine, pyrrolidine, a mono-, di- or tri-lower alkylamine, for example ethylamine, diethylamine, triethylamine or dimethylpropylamine, or a mono-, di- or trihydroxy-lower alkylamine, for example mono-, di- or tri-ethanolamine. Corresponding internal salts may also be formed where appropriate. The free form is preferred. Among the salts of the compounds of formula (I), the agrochemically advantageous salts are preferred. Hereinbefore and hereinafter, any reference to the free compounds of formula (I) or their salts is to be understood as including, where appropriate, also the corresponding salts or the free compounds of formula (I), respectively. The same applies to tautomers of compounds of formula (I) and salts thereof.

Unless defined otherwise, the general terms used hereinabove and hereinbelow have the meanings given below.

Carbon-containing groups and compounds containing in each case 1 up to and including 6, preferably 1 up to and including 4, in particular 1 or 2, carbon atoms.

Halogen- as a group per se and also as a structural element of other groups and compounds, such as haloalkyl, haloalkoxy and haloalkylthio - is fluorine, chlorine, bromine or iodine, in particular fluorine, chlorine or bromine, especially fluorine or chlorine.

Alkyl - as a group per se and also as a structural element of other groups and compounds, such as haloalkyl, alkoxy and alkylthio - is, in each case taking into account the number of carbon atoms contained in each case in the group or compound in question, either straight-chain, i.e. methyl, ethyl, propyl, butyl, pentyl, hexyl, heptyl or octyl, or branched, for example isopropyl, isobutyl, sec-butyl, tert-butyl, isopentyl, neopentyl or isohexyl.

Cycloalkyl - as a group per se and also as a structural element of other groups and compounds, such as, for example, of halocycloalkyl, cycloalkoxy and cycloalkylthio - is, in each case taking into account the number of carbon atoms contained in each case in the group or compound in question, cyclopropyl, cyclobutyl, cyclopentyl, cyclohexyl, cycloheptyl or cyclooctyl.

Alkenyl - as a group per se and also as a structural element of other groups and compounds - is, taking into account the number of carbon atoms and conjugated or isolated double bonds contained in the group, either straight-chain, for example vinyl, allyl, 2-butenyl, 3-pentenyl, 1-hexenyl, 1-heptenyl, 1,3-hexadienyl or 1,3-octadienyl, or branched, for example isopropenyl, isobutenyl, isoprenyl, tert-pentenyl, isohexenyl, isoheptenyl or isooctenyl. Preference is given to alkenyl groups having 3 to 12, in particular 3 to 6, especially 3 or 4, carbon atoms.

Alkynyl - as a group per se and also as a structural element of other groups and compounds - is, in each case taking into account the number of carbon atoms and conjugated or isolated double bonds contained in the group or compound in question, either straight-chain, for example ethynyl, propargyl, 2-butynyl, 3-pentynyl, 1-hexynyl, 1-heptynyl, 3-hexen-1-ynyl or 1,5-heptadien-3-ynyl, or branched, for example 3-methylbut-1-ynyl, 4-ethylpent-1-ynyl, 4-methylhex-2-ynyl or 2-methylhept-3-ynyl. Preference is given to alkynyl groups having 3 to 12, that is CH₂-C₂-C₁₁alkynyl, in particular 3 to 6, especially 3 or 4, carbon atoms.

Alkylene and alkenylene are straight-chain or branched bridge members; they are in particular -CH₂-CH₂-CH₂-, -CH₂-CH₂-CH₂-CH₂-, -CH₂-CH₂-CH₂-CH₂-CH₂-, -CH₂(CH₃)CH₂-CH₂-, -CH₂C(CH₃)₂-CH₂-, -CH₂-CH=CH-, -CH₂-CH=CH-CH₂- or -CH₂-CH=CH-CH₂-CH₂-.

Halogen-substituted carbon-containing groups and compounds, such as, for example, halogen-substituted alkyl, alkenyl, alkynyl, cycloalkyl, alkoxy or alkylthio, can be partially halogenated or perhalogenated, where in the case of polyhalogenation the halogen substituents can be identical or different. Examples of haloalkyl - as a group per se and also as a structural element of other groups and compounds, such as haloalkoxy or haloalkylthio - are methyl which is mono- to trisubstituted by fluorine, chlorine and/or bromine, such as CHF₂ or CF₃; ethyl which is mono- to pentasubstituted by fluorine, chlorine and/or bromine, such as CH₂CF₃, CF₂CF₃, CF₂CCl₃, CF₂CHCl₂, CF₂CHF₂, CF₂CFCl₂, CF₂CHBr₂, CF₂CHClF, CF₂CHBrF or CClFCHClF; propyl or isopropyl which is mono- to heptasubstituted by fluorine, chlorine and/or bromine, such as CH₂CHBrCH₂Br, CF₂CHFCF₃, CH₂CF₂CF₃, CF(CF₃)₂, or CH(CF₃)₂; butyl or one of its isomers, mono- to nonasubstituted by fluorine, chlorine and/or bromine, such as CF(CF₃)CHFCF₃ or CH₂(CF₂)₂CF₃; pentyl or one of its isomers, mono- to undecasubstituted by fluorine, chlorine and/or bromine, such as CF(CF₃)(CHF₂)CF₃ or CH₂(CF₂)₃CF₃; and hexyl or one of its isomers, mono- to tridecasubstituted by fluorine, chlorine and/or bromine, such as (CH₂)₄CHBrCH₂Br, CF₂(CHF)₄CF₃, CH₂(CF₂)₄CF₃ or C(CF₃)₂(CHF)₂CF₃.

Aryl is in particular phenyl, naphthyl, anthracenyl, phenanthrenyl, perylenyl or fluorenyl, preferably phenyl.

Heterocyclyl is understood as being a three- to seven-membered monocyclic ring, which may be saturated or unsaturated, and that contains from one to three hetero atoms selected from the group consisting of N, O and S, especially N and S; or a bicyclic ringsystem having from 8 to 14 ring atoms, which may be saturated or unsaturated, and that may contain either in only one ring or in both rings independently of one another, one or two hetero atoms selected from N, O and S.

Heterocyclyl is in particular piperidinyl, piperazinyl, oxiranyl, morpholinyl, thiomorpholinyl, pyridyl, N-oxidopyridinio, pyrimidyl, pyrazinyl, s-triazinyl, 1,2,4-triazinyl, thienyl, furanyl, dihydrofuranyl, tetrahydrofuranyl, pyranyl, tetrahydropyranyl, pyrrolyl, pyrrolinyl, pyrrolidinyl, pyrazolyl, imidazolyl, imidazolinyl, thiazolyl, isothiazolyl, triazolyl, oxazolyl, thiadiazolyl, thiazolinyl, thiazolidinyl, oxadiazolyl, phthalimidoyl, benzothienyl, quinolinyl, quinoxalinyl, benzofuranyl, benzimidazolyl, benzpyrrolyl, benzthiazolyl, indolinyl, isoindolinyl, cumarinyl, indazolyl, benzothiophenyl, benzofuranyl, pteridinyl or purinyl, which are preferably attached via a C atom; thienyl, benzofuranyl, benzothiazolyl, tetrahydropyranyl or indolyl is preferred; in particular pyridyl or thiazolyl. The said heterocyclyl radicals may preferably be unsubstituted or - depending on the substitution possibilities on the ring system - substituted by 1 to 3 substituents selected from the group consisting of halogen, =O, -OH, =S, SH, nitro, C₁-C₆alkyl, C₁-C₆hydroxyalkyl, C₁-C₆alkoxy, C₁-C₆haloalkyl, C₁-C₆haloalkoxy, phenyl, benzyl, -C(=O)-R₆ and -CH₂-C(=O)-R₆.

In the frame of the present invention, that in the bridging members -O-C(=Z)-, -S-C(=Z)-, and -NR₄-C(=Z)-, as defined under structural element A, it is understood that the carbon atom of the said bridging member A is bound to the N-atom which is adjacent to the 4'-position. For the bridging groups -O-SO₂- and -NR₄-SO₂-, it is understood, that the S-atom is bound to the N-atom adjacent to the 4'-position.

In the context of the present invention, preference is given to
(2) compounds according to group (1) of the formula (I) in which R₁ is isopropyl or sec-butyl, preferably to those in which a mixture of the isopropyl and the sec-butyl derivative is present;
(3) compounds according to group (1) of the formula (I) in which R₁ is cyclohexyl;
(4) compounds according to group (1) of the formula (I) in which R₁ is 1-methyl-butyl;
(5) compounds according to anyone of groups (1) to (4) of the formula (I) in which A is -C(=O)-;
(6) compounds according to anyone of groups (1) to (4) of the formula (I) in which A is -C(=S)-;
(7) compounds according to anyone of groups (1) to (4) of the formula (I) in which A is -O-C(=O)-;
(8) compounds according to anyone of groups (1) to (4) of the formula (I) in which A is -O-C(=S)-;
(9) compounds according to anyone of groups (1) to (4) of the formula (I) in which A is -S-C(=O)-;
(10) compounds according to anyone of groups (1) to (4) of the formula (I) in which A is -S-C(=S)-;
(11) compounds according to anyone of groups (1) to (4) of the formula (I) in which A is -NR₄-C(=O)-;
(12) compounds according to anyone of groups (1) to (4) of the formula (I) in which A is -NR₄-C(=S)-;
(13) compounds according to anyone of groups (1) to (4) of the formula (I) in which A is -SO₂-;
(14) compounds according to anyone of groups (1) to (4) of the formula (I) in which A is -O-SO₂-;
(15) compounds according to anyone of groups (1) to (4) of the formula (I) in which A is -NR₄-SO₂-;
(16) compounds according to anyone of groups (1) to (4) of the formula (I) in which A is a bond;
(17) compounds according to anyone of groups (1) to (16) of the formula (I) in which the carbon atom 4' has the configuration *(R)*;
(18) compounds according to anyone of groups (1) to (16) of the formula (I) in which the carbon atom 4' has the configuration *(S)*;
(19) compounds according to anyone of groups (1) to (18) of the formula (I) in which X-Y is -CH=CH-;
(20) compounds according to anyone of groups (1) to (18) of the formula (I) in which X-Y is -CH₂-CH₂-;
(21) compounds according to anyone of groups (1) to (20) of the formula (I) in which R₂ is H;
(22) compounds according to anyone of groups (1) to (20) of the formula (I) in which R₂ is C₁-C₁₂alkyl, especially methyl;
(23) compounds according to anyone of groups (1) to (20) of the formula (I) in which R₂ is unsubstituted C₇-C₁₂alkyl;
(24) compounds according to anyone of groups (1) to (20) of the formula (I) in which R₂ is mono- to pentasubstituted C₁-C₆alkyl;
(25) compounds according to anyone of groups (1) to (20) of the formula (I) in which R₂ is mono- to pentasubstituted C₇-C₁₂alkyl;
(26) compounds according to anyone of groups (1) to (25) of the formula (I) in which R₃ is H;
(27) compounds according to anyone of groups (1) to (25) of the formula (I) in which R₃ is C₁-C₁₂alkyl, C₂-C₁₂alkenyl, C₂-C₁₂alkynyl, C₃-C₁₂cycloalkyl, C₅-C₁₂cycloalkenyl, aryl, or heterocyclyl; wherein the C₁-C₁₂alkyl, C₂-C₁₂alkenyl, C₂-C₁₂alkynyl, C₃-C₁₂cycloalkyl, C₅-C₁₂cycloalkenyl, aryl and heterocyclyl radicals may be unsubstituted or mono- to pentasubstituted;
(28) compounds according to anyone of groups (1) to (25) of the formula (I) in which R₃ is unsubstituted or mono- to pentasubstituted, depending on the substitution possibilities, C₁-C₁₂alkyl, especially unsubstituted C₁-C₁₂alkyl;
(29) compounds according to anyone of groups (1) to (25) of the formula (I) in which R₃ is unsubstituted or monosubstituted C₁-C₆alkyl;
(30) compounds according to anyone of groups (1) to (20) of the formula (I) in which R₂ and R₃ together are a three- to seven membered alkylene or alkenylene bridge, which is unsubstituted or mono to tri-substituted, preferably unsubstituted; and in which one of the methylene groups of the bridge is replaced by O, NR₄, S, S(=O) or SO₂;
(31) compounds according to anyone of groups (1) to (25) of the formula (I) in which R₃ is unsubstituted or mono- to pentasubstituted C₆-C₁₂alkyl;
(32) compounds according to anyone of groups (1) to (25) of the formula (I) in which R₃ is unsubstituted or mono- to pentasubstituted C₂-C₁₂alkenyl;
(33) compounds according to anyone of groups (1) to (25) of the formula (I) in which R₃ is unsubstituted or mono- to pentasubstituted C₂-C₁₂alkynyl;
(34) compounds according to anyone of groups (1) to (25) of the formula (I) in which R₃ is unsubstituted or mono- to pentasubstituted C₃-C₁₂cycloalkyl;
(35) compounds according to anyone of groups (1) to (25) of the formula (I) in which R₃ is unsubstituted or mono- to pentasubstituted C₃-C₆-cycloalkyl;
(36) compounds according to anyone of groups (1) to (25) of the formula (I) in which R₃ is unsubstituted or monosubstituted C₃-C₆-cycloalkyl;
(37) compounds according to anyone of groups (1) to (25) of the formula (I) in which R₃ is unsubstituted or mono- to pentasubstituted C₁-C₁₂cycloalkyl;
(38) compounds according to anyone of groups (1) to (25) of the formula (I) in which R₃ is unsubstituted or mono- to pentasubstituted C₅-C₁₂cycloalkenyl;
(39) compounds according to anyone of groups (1) to (25) of the formula (I) in which R₃ is unsubstituted or mono- to pentasubstituted aryl;
(40) compounds according to anyone of groups (1) to (25) of the formula (I) in which R₃ is unsubstituted or mono- to pentasubstituted phenyl;
(41) compounds according to anyone of groups (1) to (25) of the formula (I) in which R₃ is unsubstituted or monosubstituted phenyl;
(42) compounds according to anyone of groups (1) to (25) of the formula (I) in which R₃ is unsubstituted or mono- to pentasubstituted naphthyl, anthracenyl, phenanthrenyl, perylenyl or fluorenyl;
(43) compounds according to anyone of groups (1) to (25) of the formula (I) in which R₃ is unsubstituted or mono- to pentasubstituted heterocyclyl;
(44) compounds according to anyone of groups (1) to (20) of the formula (I) in which R₂ and R₃ together are a three- to seven membered alkylene or alkenylene bridge, which is unsubstituted or mono to tri-substituted;
(45) compounds according to anyone of groups (1) to (20) of the formula (I) in which R₂ and R₃ together are a three membered alkylene or alkenylene bridge, which is unsubstituted or mono to tri-substituted;
(46) compounds according to anyone of groups (1) to (20) of the formula (I) in which R₂ and R₃ together are a four membered alkylene or alkenylene bridge, which is unsubstituted or mono to tri-substituted;
(47) compounds according to anyone of groups (1) to (20) of the formula (I) in which R₂ and R₃ together are a five membered alkylene or alkenylene bridge, which is unsubstituted or mono to tri-substituted;
(48) compounds according to anyone of groups (1) to (20) of the formula (I) in which R₂ and R₃ together are a six membered alkylene or alkenylene bridge, which is unsubstituted or mono to tri-substituted;
(49) compounds according to anyone of groups (1) to (20) of the formula (I) in which R₂ and R₃ together are a seven membered alkylene or alkenylene bridge, which is unsubstituted or mono to tri-substituted;
(50) compounds according to anyone of groups (1) to (20) of the formula (I) in which R₂ and R₃ together are a three- to seven membered alkylene or alkenylene bridge, which is unsubstituted;
(51) compounds according to anyone of groups (1) to (20) of the formula (I) in which R₂ and R₃ together are a three- to seven membered alkylene or alkenylene bridge, which is mono to tri-substituted;
(52) compounds according to anyone of groups (1) to (20) of the formula (I) in which R₂ and R₃ and A together are =N⁺=N⁻;

In each embodiment of the present invention, the total number of carbon atoms in at least one of R₂ and R₃ is at least 6, preferably at least 7, such as 8 to 12.

Special preference is given within the scope of the invention to the compounds of formula (I) listed in Tables A1 to A9 and in Tables 1 to 144 and, where applicable, their isomers and tautomers thereof, mixtures of tautomers, their E/Z isomers and mixtures of E/Z isomers.

The invention also provides a process for preparing the compounds of the formula (I), their isomers and, if appropriate, tautomers thereof, wherein
(A) for the preparation of a compound of the formula 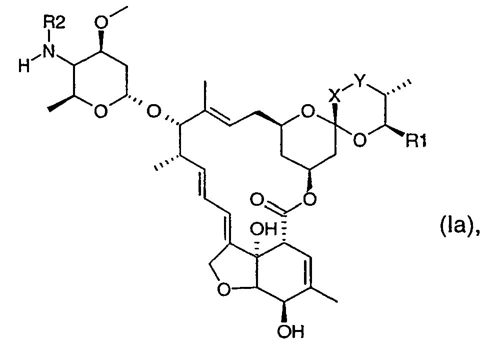 wherein X-Y, R₁ and R₂ have the same meanings as given above under (1) for formula (I), a compound of the formula 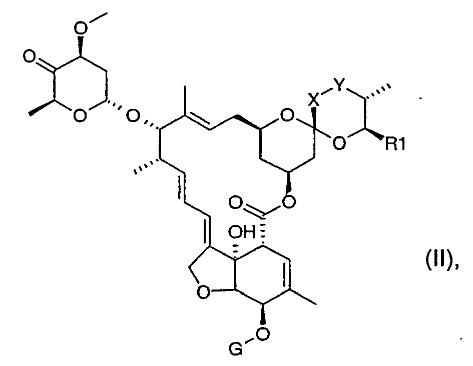 wherein X-Y and R₁ have the same meanings as given above under (1) for formula (I) and G is a protecting group, for example a trialkylsilyl group or an ester group, and which is known and which can be prepared by methods known per se, is reacted with a compound R₂-N(G₁)₂, in which R₂ has the same meaning as given above under (1) for formula (I), and in which G₁ is H or trimethylsilyl, in the presence of a reducing agent, and subsequently cleaving the protecting group by methods known *per* se; or
(B) for the preparation of a compound of the formula 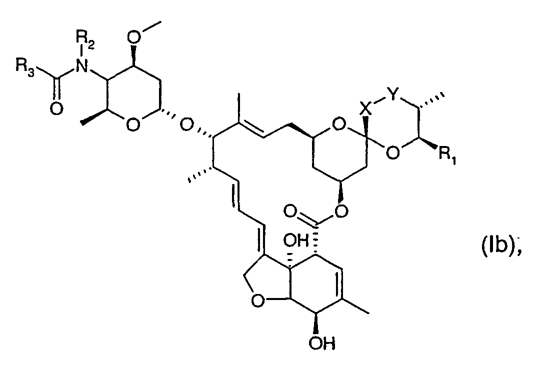 in which X-Y, R₁, R₂ and R₃ have the same meanings as given above under (1) for formula (I), a compound of the formula (Ia) as defined above under (A) is reacted with a compound R₃-C(=O)-Cl or a compound [R₃-C(=O)-]₂O, in which R₃ has the same meaning as given above under (1) for formula (I); or
(C) for the preparation of a compound of the formula 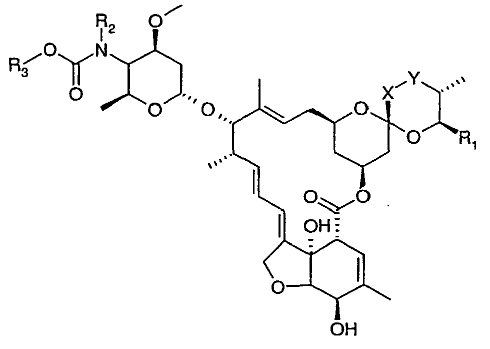 and in which X-Y, R₁, R₂ and R₃ have the same meanings as given above under (1) for formula (I),
   a compound of the formula (Ia) as defined above under (A) is reacted with a compound R₃-O-C(=O)-Cl or a compound [R₃-O-C(=O)-]₂O, in which R₃ has the same meaning as given above under (1) for formula (I); or
(D) for the preparation of a compound of the formula 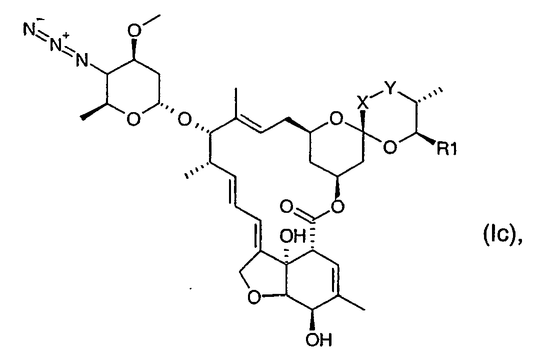 in which X-Y and R₁ have the same meanings as given above under (1) for formula (I), a compound of the formula 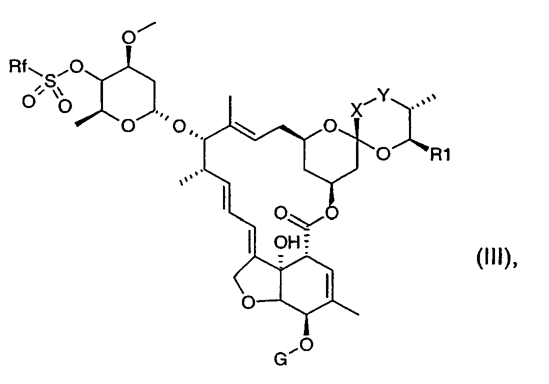 in which X-Y and R₁ have the same meanings as given above under (1) for formula (I), and Rf is C₁-C₁₂alkyl, haloC₁-C₁₂alkyl or aryl, especially trifluoromethyl, which is known and which can be prepared by methods known per se, and in which G is a protecting group, for example a trialkylsilyl group or an ester group, is reacted with an azide, for example with a metal azide, such as an alkali metal azide, or with a tetraalkylammonium azide, and subsequently the protecting group is cleaved by methods known *per se*; or
(E) for the preparation of a compound of the formula 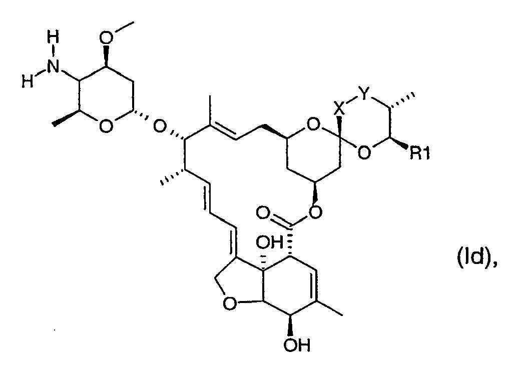 in which X-Y and R₁ have the same meanings as given above under (1) for formula (I), a compound of the formula (Ic) as defined before under (D), is reacted with a reducing agent, for example with a phosphine in the presence of water, such as a trialkylphosphine or a triphenylphosphine, or with a hydride, such as a borohydride, or with a formate in the presence of a hydrogenation catalyst, such as an alkali metal formate or a tetraalkylammonium formate, or with hydrogen in the presence of a hydrogenation catalyst; or
(F) for the preparation of a compound of the formula 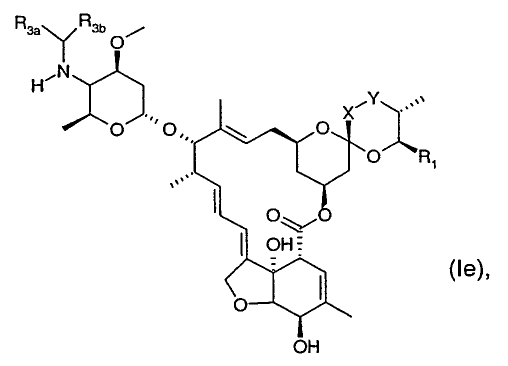 in which X-Y, R₁ and R₃ have the same meanings as given above under (1) for formula (I), is reacted with a phosphine, for example with a trialkylphosphine or with a triphenylphosphine; subsequently reacted with a compound R₃ₐ-C(=O)-R_{3b} in which R₃ₐ and R_{3b} are, independently from each other, H, C₁-C₁₁alkyl, C₂-C₁₁alkenyl or C₂-C₁₁alkynyl, wherein C₁-C₁₁alkyl, C₂-C₁₁alkenyl, C₂-C₁₁alkynyl substituents may be unsubstituted or mono- to pentasubstituted with substituents having the same meaning as given above under (1) for formula (I); and wherein the number of carbon atoms of the substituents R₃ₐ and R_{3b} together is not greater than 11 and then subsequently reacted with a reducing agent, for example a borohydride; or
(G) for the preparation of a compound of the formula (I) 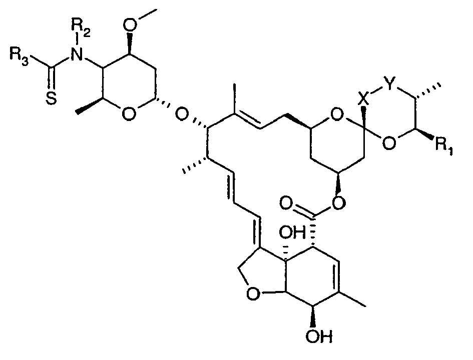 in which X-Y, R₁, R₂ and R₃ have the same meanings as given above under (1) for formula (I),
   a compound of the formula (Ia) as defined above under (A) is reacted with a compound R₃-C(=S)-S-Q₁ or a compound R₃-C(=S)-H_{N}, in which R₃ has the same meaning as given above under (1) for formula (I), H_{N} is a nitrogen containing heterocycle radical, which is connected via a nitrogen atom, for example 1 H-benzotriazol-1-yl or isoindole-1,3-dione-2-yl or 1,3-dihydro-benzoimidazol-2-one-1-yl, and in which Q₁ is H, unsubstituted or mono- to pentasubstituted C₁-C₁₂alkyl, unsubstituted or mono- to pentasubstituted C₂-C₁₂alkenyl, unsubstituted or mono- to pentasubstituted C₂-C₁₂alkynyl, unsubstituted or mono- to pentasubstituted C₃-C₁₂cycloalkyl, unsubstituted or mono- to pentasubstituted C₅-C₁₂cycloalkenyl, unsubstituted or mono- to pentasubstituted aryl, or unsubstituted or mono- to pentasubstituted heterocyclyl, wherein the substituents of the alkyl, alkenyl, alkynyl, cycloalkyl, cycloalkenyl, aryl and heterocyclyl radicals mentioned have the same meaning as given above under (1) for formula (I); or
(H) for the preparation of a compound of the formula 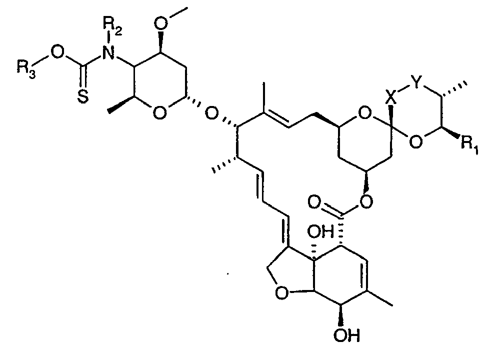 in which X-Y, R₁, R₂ and R₃ have the same meanings as given above under (1) for formula (I),
   a compound of the formula (Ia) as defined above under (A) is reacted with a compound R₃-OH and CS₂, or with a compound R₃-O-C(=S)-S-Q₁, in which R₃ has the same meaning as given above under (1) for formula (I) and Q₁ has the same meaning as given above under (G); or
(J) for the preparation of a compound of the formula 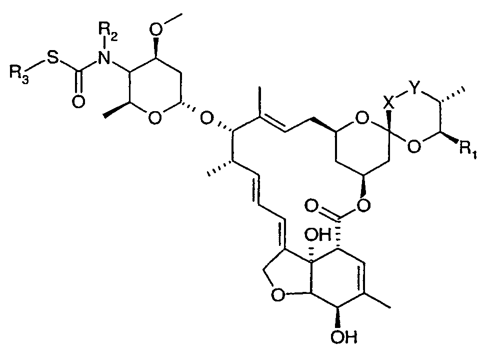 in which X-Y, R₁, R₂ and R₃ have the same meanings as given above under (1) for formula (I), a compound of the formula (Ia) as defined above under (A) is reacted with a compound R₃-S-C(=O)-Cl or [R₃-S-C(=O)-]₂O, in which R₃ has the same meaning as given above under (1) for formula (I); or
(K) for the preparation of a compound of the formula 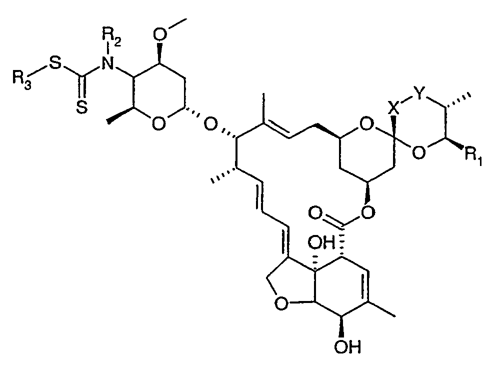 in which X-Y, R₁, R₂ and R₃ have the same meanings as given above under (1) for formula (I),
   a compound of the formula (Ia), as defined under (A), is reacted with CS₂ and a compound R₃-X₁, or with a compound R₃-S-C(=S)-H_{N}, in which R₃ has the same meaning as given above under (1) for formula (I), X₁ is chlorine, bromine or iodine, and H_{N} has the same meaning as given above under (G); or
(L) for the preparation of a compound of the formula 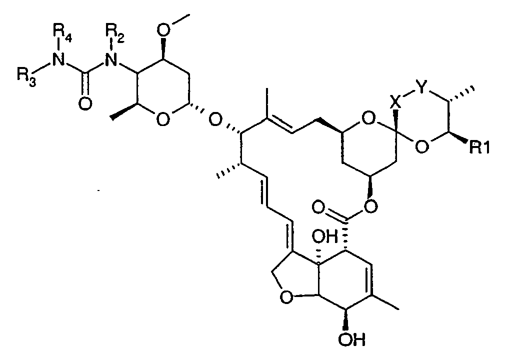 in which X-Y, R₁, R₂, R₃ and R₄ have the same meanings as given above under (1) for formula (I), a compound of the formula (Ia) as defined under (A), is reacted with a compound R₃-NR₄-C(=O)-Cl or with a compound R₃-N=C=O in which R₃ and R₄ have the same meanings as given above under (1) for formula (I); or
(M) for the preparation of a compound of the formula 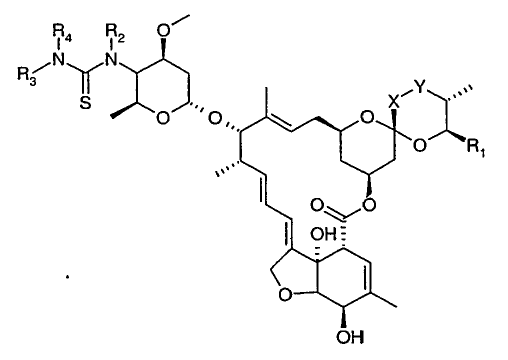 in which X-Y, R₁, R₂, R₃ and R₄ have the same meanings as given above under (1) for formula (I),
   a compound of the formula (Ia) as defined above under (A) is reacted with a compound R₃-N=C=S, or with a compound R₃-NR₄-H and CS₂, or with a compound R₃-NR₄-C(=S)-S-M₁, in which R₃ and R₄ have the same meanings as given above under (1) for formula (I), and M₁ is a metal, for example zinc; or
(N) for the preparation of a compound of the formula 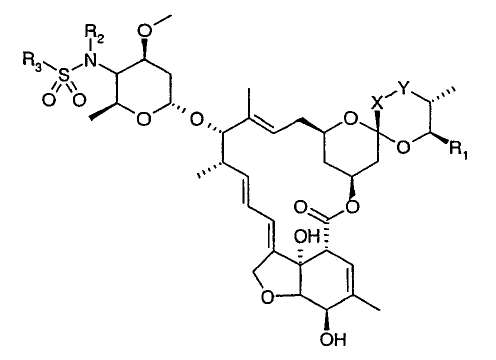 in which X-Y, R₁, R₂ and R₃ have the same meanings as given above under (1) for formula (I), a compound of the formula (Ia) as defined above under (A) is reacted with a compound R₃-SO₂-Cl or [R₃-SO₂-]₂O, in which R₃ has the same meaning as given above under (1) for formula (I); or
(O) for the preparation of a compound of the formula 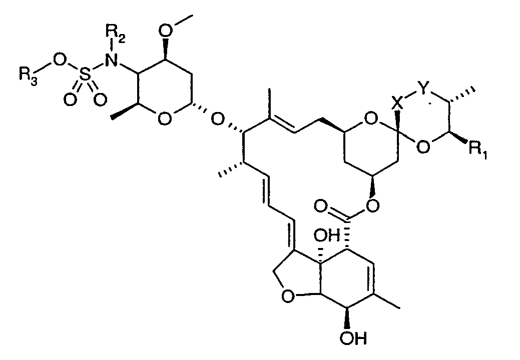 in which X-Y, R₁, R₂ and R₃ have the same meanings as given above under (1) for formula (I), a compound of the formula (Ia) as defined above under (A) is reacted with SO₃ and a compound R₃-OH, or with a compound R₃-O-SO₂-O-Q₁, in which R₃ has the same meaning as given above under (1) for formula (I), and Q₁ has the same meaning as given above under (G); or
(P) for the preparation of a compound of the formula 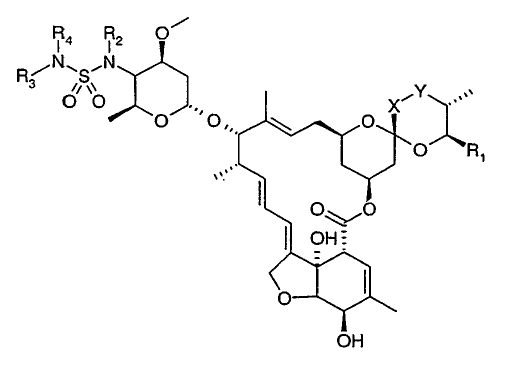 in which X-Y, R₁, R₂, R₃ and R₄ have the same meanings as given above under (1) for formula (I), a compound of the formula (Ia) as defined above under (A) is reacted with a compound R₃-NR₄-SO₂-O-Q₁ or with a compound R₃-NR₄-SO₂-Cl, in which R₃ has the same meaning as given above under (1) for formula (I), and Q₁ has the same meaning as given above under (G); or
(Q) for the preparation of a compound of the formula (I) wherein R₁, R₂, R₃, X and Y have the meanings as defined under (1) for formula (I), and A is a bond,
   a compound of the formula (Ia) as defined above under (A) is reacted with a compound R₃-X₁, in which R₃ has the same meaning as given above under (1) for formula (I), and X₁ has the same meaning as given above under (K); or
(R) for the preparation of a compound of the formula (I) wherein R₁, R₂, R₃, X and Y have the meanings as defined under (1) for formula (I), and A is a bond,
   a compound of the formula (Ia) as defined above under (A) is reacted with a compound R₃ₐ-C(=O)-R_{3b}, in which R₃ₐ and R_{3b} have the same meanings as given above under (F), in the presence of a reducing agent, for example a borohydride.

The comments made above in connection with isomers and tautomers of compounds of formula (I) apply analogously to the starting materials and intermediates mentioned hereinabove and hereinbelow in respect of their isomers and tautomers.

Furthermore compounds of formula (I) bearing a functional group in its free or protected form can be used as starting materials for the preparation of further compounds of formula (I). For such manipulations methods known to the person skilled in the art can be applied.

For example a compound of formula (I) wherein R₂ is CH₃C(=O)OC₁-C₁₂alky can be converted to a compound of formula (I) wherein R₂ is hydroxy-C₁-C₁₂alkyl, Further standard reactions can deliver compounds of formula (I) wherein R₂ is C₁-C₁₂alkyl-OCH₂O-C₁-C₁₂alkyl, R₆C(=O)OC₁-C₁₂alkyl, R₆ZC(=O)OC₁-C₁₂alkyl, and N₃-C₁-C₁₂alkyl, where n is an integer and Z and R₆ are as defined in formula (I). A compound of formula (I) wherein R₂ is N₃-C₁-C₁₂alkyl can be converted to a compound of formula (I) wherein R₂ is NH₂-C₁-C₁₂alkyl. Treatment of such a compound of formula (I) with Hal-C(=O)R₆ gives compounds of formula (I) wherein R₂ is R₄C(=O)NHC₁-C₁₂alkyl.

The reactions described hereinabove and hereinbelow are carried out in a manner known *per se*, for example in the absence or, customarily, in the presence of a suitable solvent or diluent or of a mixture thereof, the reactions being carried out, as required, with cooling, at room temperature or with heating, for example in a temperature range of approximately from -80°C to the boiling temperature of the reaction medium, preferably from approximately 0°C to approximately +150°C, and, if necessary, in a closed vessel, under pressure, under an inert gas atmosphere and/or under anhydrous conditions. Especially advantageous reaction conditions can be found in the Examples.

The reaction time is not critical; a reaction time of from about 0.1 to about 24 hours, especially from about 0.5 to about 10 hours, is preferred.

The product is isolated by customary methods, for example by means of filtration, crystallisation, distillation or chromatography, or any suitable combination of such methods.

The starting materials mentioned hereinabove and hereinbelow that are used for the preparation of the compounds of formula (I) and, where applicable, their isomers and tautomers are known or can be prepared by methods known *per se*, e.g. as indicated below.

### Process variant (A):

Examples of solvents and diluents include: aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethyl ether, dimethoxydiethyl ether, tetrahydrofuran or dioxane; alcohols, such as methanol, ethanol, propanol, isopropanol, butanol, ethylene glycol or glycerol; carboxylic acids, such as acetic acid, pivalic acid or formic acid; ketones, such as acetone, methyl ethyl ketone or methyl isobutyl ketone; carboxylic acid esters, such as methyl acetate, ethyl acetate, or esters of benzoic acid; amides, such as N,N-dimethylformamide, N,N-diethylformamide, N,N-dimethylacetamide, N-methylpyrrolidone or hexamethylphosphoric acid triamide; nitriles, such as acetonitrile or propionitrile; and sulfoxides, such as dimethyl sulfoxide; and also water; or mixtures of the mentioned solvents; especially suitable are esters, ethers, alcohols, water, carboxylic acids, or mixtures thereof, more especially ethyl acetate, isopropyl acetate, tetrahydrofuran, pivalic acid or water.

The reactions are advantageously carried out in a temperature range of from about room temperature to the boiling point of the solvent used; preference being given to reaction at 10 to 50°C.

Examples of reducing agents are known to a person skilled in the art, they include hydrides; especially suitable are borohydrides, for example sodium borohydride or sodium cyanoborohydride.

In a preferred embodiment of Variant (A) the reaction is carried out with sodium cyanoborohydride at room temperature, in tetrahydrofuran in the presence of pivalic acid and water at ambient temperature.

In another embodiment of Variant (A) the reaction is carried out in the presence of a Lewis acid. In another preferred embodiment of Variant (A) the reaction is carried out in isopropyl acetate, in the presence of zinc bromide, with sodium borohydride at 40 °C.

Especially preferred conditions for this Process variant are described in Example A3.1.

### Process variant (B):

Examples of solvents and diluents include those listed above under Process variant (A); especially suitable are esters, for example ethyl acetate, or water, or mixtures thereof; or halogenated hydrocarbons, for example dichloromethane, trichloromethane; or ethers, for example tetrahydrofuran.

The reactions are advantageously carried out in a temperature range of from about room temperature to the boiling point of the solvent used; preference being given to reaction at 10 to 30°C.

The reaction is carried out in the presence or in the absence of a base. Preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine.

In a preferred embodiment of Variant (B) the reaction is carried out in a mixture of ethyl acetate and water, in the presence of sodium bicarbonate, at ambient temperature. In another preferred embodiment of Variant (B) the reaction is carried out in tetrahydrofuran, in the presence of N,N-diisopropyl-ethylamine, at ambient temperature.

Especially preferred conditions for this Process variant are described, for example, in Example A1.1.

### Process variant (C):

Examples of solvents and diluents include those listed above under Process variant (A); especially suitable are esters, for example ethyl acetate, or water, or mixtures thereof; or halogenated hydrocarbons, for example dichloromethane, trichloromethane; or ethers, for example tetrahydrofuran.

The reactions are advantageously carried out in a temperature range of from about room temperature to the boiling point of the solvent used; preference being given to reaction at 10 to 30°C.

The reaction is carried out in the presence or in the absence of a base. Preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine.

In a preferred embodiment of Variant (C) the reaction is carried out in a mixture of ethyl acetate and water, in the presence of sodium bicarbonate, at ambient temperature. In another preferred embodiment of Variant (C) the reaction is carried out in tetrahydrofuran, in the presence of N,N-diisopropyl-ethylamine, at ambient temperature.

Especially preferred conditions for this Process variant are described, for example, in Example A2.1.

### Process variant (D):

Examples of solvents and diluents include those listed above under Process variant (A); especially suitable are amides, for example N,N-dimethylformamide.

The reactions are advantageously carried out in a temperature range of from about -10 °C to the boiling point of the solvent used; preference being given to reaction at 0 °C to 30°C.

In a preferred embodiment of Variant (D) the reaction is carried out with sodium azide, at 0 to 5 °C in N,N-dimethylformamide as the solvent.

Especially preferred conditions for this Process variant are described, for example, in Example A4.1.

### Process variant (E):

Examples of solvents and diluents include those listed above under Process variant (A); especially suitable are alcohols, ethers, water, or mixtures thereof, more especially methanol, or tetrahydrofuran and water.

The reactions are advantageously carried out in a temperature range of from about room temperature to the boiling point of the solvent used; preference being given to reaction at 10 to 40°C.

The reaction is carried out in the presence or in the absence of a base. Preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine.

Especially suitable phosphines include trialkylphosphines, for example trimethylphosphine. Especially suitable hydrides include, for example, sodium borohydride. Especially suitable hydrogenation catalysts include palladium catalysts, for example, palladium hydroxide on carbon. Especially suitable formates include sodium formate and ammonium formate.

In a preferred embodiment of Variant (E) the reaction is carried out with trimethylphosphine at 30 °C, in tetrahydrofuran as the solvent, and the subsequent addition of aqueous sodium hydroxide.

In another preferred embodiment of Variant (E) the reaction is carried out with ammonium formate, in the presence of palladium hydroxide on carbon, in methanol as a solvent, at ambient temperature.

Especially preferred conditions for this Process variant are described, for example, in Example A4.2.

### Process variant (F):

Examples of solvents and diluents include those listed above under Process variant (A); especially suitable are alcohols, ethers, carboxylic acids, water, or mixtures thereof, more especially methanol, tetrahydrofuran, pivalic acid and water.

The reactions are advantageously carried out in a temperature range of from 0°C to the boiling point of the solvent used, preferably between ambient temperature and 30 °C.

Especially suitable phosphines include trialkylphosphines, for example trimethylphosphine.

Suitable reducing agents are borohydrides, for example sodium borohydride or sodium cyanoborohydride, especially suitable are sodium borohydride, or sodium cyanoborohydride in the presence of pivalic acid.

In a preferred embodiment of Variant (F) the reaction is carried out with trimethylphosphine in tetrahydrofuran at 30 °C, followed by addition of R₃ₐ-C(=O)-R_{3b} in tetrahydrofuran, and subsequent addition of sodium borohydride.

Especially preferred conditions for this Process variant are described, for example, in Example A4.3.

### Process variant (G):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

The reaction is carried out in the presence or in the absence of a base.

In a preferred embodiment of Variant (G) the reaction is carried out in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (G) the reaction is carried in the absence of a base.

### Process variant (H):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

The reaction is carried out in the presence or in the absence of a base.

In a preferred embodiment of Variant (H) the reaction is carried out in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (H) the reaction is carried out in the absence of a base.

### Process variant (J):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

The reaction is carried out in the presence or in the absence of a base.

In a preferred embodiment of Variant (J) the reaction is carried out in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (J) the reaction is carried out in the absence of a base.

### Process variant (K):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

The reaction is carried out in the presence or in the absence of a base.

In a preferred embodiment of Variant (K) preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (K) the reaction is carried out in the absence of a base.

### Process variant (L):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

In a preferred embodiment of Variant (L) the reaction is carried out in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (L) the reaction is carried out in the absence of a base.

### Process variant (M):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

In a preferred embodiment of Variant (M) the reaction is carried out in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (M) the reaction is carried out in the absence of a base.

### Process variant (N):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

In a preferred embodiment of Variant (N) preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (N) preference is being given to carrying out the reaction in the absence of a base.

### Process variant (O):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

In a preferred embodiment of Variant (O) preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (O) the reaction is carried out in the absence of a base.

### Process variant (P):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

In a preferred embodiment of Variant (P) the reaction is carried out in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine. In another preferred embodiment of Variant (P) preference is being given to carrying out the reaction in the absence of a base.

### Process variant (Q):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

In a preferred embodiment of Variant (Q) preference is being given to carrying out the reaction in the presence of a base. Suitable bases include, for example, inorganic salts, for example sodium bicarbonate, sodium hydroxide or potassium carbonate, and organic bases, such as amines, for example triethylamine, pyridine, imidazole, 4-(N,N-dimethylamino)-pyridine or N,N-diisopropyl-ethylamine.

In an especially preferred embodiment of Variant (Q) the reaction is carried out in a mixture of water and ethyl acetate as solvents, in the presence of sodium bicarbonate as a base, at ambient temperature.

### Process variant (R):

Examples of solvents and diluents include those listed above under Process variant (A); especially suitable are aromatic, aliphatic and alicyclic hydrocarbons and halogenated hydrocarbons, ethers, amides, nitrile and water; more especially toluene, hexane, cyclohexane, dichloromethane, tetrahydrofuran, acetonitrile, water, or mixtures thereof.

The reactions are advantageously carried out in a temperature range of from 0°C to the boiling point of the solvent used, preferably at ambient temperature.

Suitable reducing agents are, for example, borohydrides, for example sodium borohydride or sodium cyanoborohydride, especially suitable are sodium borohydride, or sodium cyanoborohydride in the presence of pivalic acid.

In a preferred embodiment of variant (R) the reaction is carried out in tetrahydrofuran, with sodium cyanoborohydride, in the presence of pivalic acid and water, at ambient temperature.

The compounds of formula (I) may be in the form of one of the possible isomers or in the form of a mixture thereof, in the form of pure isomers or in the form of an isomeric mixture, i.e. in the form of a diastereomeric mixture; the invention relates both to the pure isomers and to the diastereomeric mixtures and is to be interpreted accordingly hereinabove and hereinbelow, even if stereochemical details are not mentioned specifically in every case.

The diastereomeric mixtures can be resolved into the pure isomers by known methods, for example by recrystallisation from a solvent, by chromatography, for example high pressure liquid chromatography (HPLC) on acetylcellulose, with the aid of suitable microorganisms, by cleavage with specific, immobilised enzymes, or *via* the formation of inclusion compounds, for example using crown ethers, only one isomer being complexed.

Apart from by separation of corresponding mixtures of isomers, pure diastereoisomers can be obtained according to the invention also by generally known methods of stereoselective synthesis, for example by carrying out the process according to the invention using starting materials having correspondingly suitable stereochemistry.

In each case it may be advantageous to isolate or synthesise the biologically more active isomer, where the individual components have different biological activity.

The compounds of formula (I) may also be obtained in the form of their hydrates and/or may include other solvents, for example solvents which may have been used for the crystallisation of compounds in solid form.

The invention relates to all those embodiments of the process according to which a compound obtainable as starting material or intermediate at any stage of the process is used as starting material and all or some of the remaining steps are carried out, or in which a starting material is used in the form of a derivative and/or a salt and/or its diastereomers, or, especially, is formed under the reaction conditions. For instance compounds of formula (I) bearing a functional group in its free or protected form can be used as starting materials for the preparation of further compounds of formula (I). For such manipulations methods known to the person skilled in the art can be applied.

In the processes of the present invention it is preferable to use those starting materials and intermediates which result in the compounds of formula (I) that are especially preferred.

The invention relates especially to the preparation processes described in Examples A1.1 to A4.3.

In the area of pest control, the compounds of formula (I) according to the invention are active ingredients exhibiting valuable preventive and/or curative activity with a very advantageous biocidal spectrum and a very broad spectrum, even at low rates of concentration, while being well tolerated by warm-blooded animals, fish and plants. They are, surprisingly, equally suitable for controlling both plant pests and ecto- and endo-parasites in humans and more especially in productive livestock, domestic animals and pets. They are effective against all or individual development stages of normally sensitive animal pests, but also of resistant animal pests, such as insects and representatives of the order Acarina, nematodes, cestodes and trematodes, while at the same time protecting useful organisms. The insecticidal or acaricidal activity of the active ingredients according to the invention may manifest itself directly, i.e. in the mortality of the pests, which occurs immediately or only after some time, for example during moulting, or indirectly, for example in reduced oviposition and/or hatching rate. Good activity corresponds to a mortality of at least 50 to 60 %.

Successful control within the scope of the subject of the invention is possible, in particular, of pests from the orders Lepidoptera, Coleoptera, Orthoptera, Isoptera, Psocoptera, Anoplura, Mallophaga, Thysanoptera, Heteroptera, Homoptera, Hymenoptera, Diptera, Siphonaptera, Thysanura and Acarina, mainly Acarina, Diptera, Thysanoptera, Lepidoptera and Coleoptera.

Very especially good control is possible of the following pests: Abagrotis spp., Abraxas spp., Acantholeucania spp., Acanthoplusia spp., Acarus spp., Acarus siro, Aceria spp., Aceria sheldoni, Acleris spp., Acoloithus spp., Acompsia spp., Acossus spp., Acria spp., Acrobasis spp., Acrocercops spp., Acrolepia spp., Acrolepiopsis spp., Acronicta spp., Acropolitis spp., Actebia spp., Aculus spp., Aculus schlechtendali, Adoxophyes spp., Adoxophyes reticulana, Aedes spp., Aegeria spp., Aethes spp., Agapeta spp., Agonopterix spp., Agriopis spp., Agriotes spp., Agriphila spp., Agrochola spp., Agroperina spp., Alabama spp., Alabama argillaceae, Agrotis spp., Albuna spp., Alcathoe spp., Alcis spp., Aleimma spp., Aletia spp., Aleurothrixus spp., Aleurothrixus floccosus, Aleyrodes spp., Aleyrodes brassicae, Allophyes spp., Alsophila spp., Amata spp., Amathes spp., Amblyomma spp., Amblyptilia spp., Ammoconia spp., Amorbia spp., Amphion spp., Amphipoea spp., Amphipyra spp., Amyelois spp., Anacamptodes spp., Anagrapha spp., Anarsia spp., Anatrychyntis spp., Anavitrinella spp., Ancylis spp., Andropolia spp., Anhimella spp., Antheraea spp., Antherigona spp., Antherigona soccata, Anthonomus spp., Anthonomus grandis, Anticarsia spp., Anticarsia gemmatalis, Aonidiella spp., Apamea spp., Aphania spp., Aphelia spp., Aphididae, Aphis spp., Apotomis spp., Aproaerema spp., Archippus spp., Archips spp., Acromyrmex, Arctia spp., Argas spp., Argolamprotes spp., Argyresthia spp., Argyrogramma spp., Argyroploce spp., Argyrotaenia spp., Arotrophora spp., Ascotis spp., Aspidiotus spp., Aspilapteryx spp., Asthenoptycha spp., Aterpia spp., Athetis spp., Atomaria spp., Atomaria linearis, Atta spp., Atypha spp., Autographa spp., Axylia spp., Bactra spp., Barbara spp., Batrachedra spp., Battaristis spp., Bembecia spp., Bemisia spp., Bemisia tabaci, Bibio spp., Bibio hortulanis, Bisigna spp., Blastesthia spp., Blatta spp., Blatella spp., Blepharosis spp., Bleptina spp., Boarmia spp., Bombyx spp., Bomolocha spp., Boophilus spp., Brachmia spp., Bradina spp., Brevipalpus spp., Brithys spp., Bryobia spp., Bryobia praetiosa, Bryotropha spp., Bupalus spp., Busseola spp., Busseola fusca, Cabera spp., Cacoecimorpha spp., Cadra spp., Cadra cautella, Caenurgina spp., Calipitrimerus spp., Callierges spp., Callophpora spp., Callophpora erythrocephala, Calophasia spp., Caloptilia spp., Calybites spp., Capnoptycha spp., Capua spp., Caradrina spp., Caripeta spp., Carmenta spp., Carposina spp., Carposina nipponensis, Catamacta spp., Catelaphris spp., Catoptria spp., Caustoloma spp., Celaena spp., Celypha spp., Cenopis spp., Cephus spp., Ceramica spp., Cerapteryx spp., Ceratitis spp, Ceratophyllus spp., Ceroplaster spp., Chaetocnema spp., Chaetocnema tibialis, Chamaesphecia spp., Charanvca spp., Cheimophila spp., Chersotis spp., Chiasmia spp., Chilo spp., Chionodes spp., Chorioptes spp., Choristoneura spp., Chrysaspidia spp., Chrysodeixis spp., Chrysomya spp., Chrysomphalus spp., Chrysomphalus dictyospermi, Chrysomphalus aonidium, Chrysoteuchia spp., Cilix spp., Cimex spp., Clysia spp., Clysia ambiguella, Clepsis spp., Cnaemidophorus spp., Cnaphalocrocis spp., Cnephasia spp., Coccus spp., Coccus hesperidum, Cochylis spp., Coleophora spp., Colotois spp., Commophila spp., Conistra spp., Conopomorpha spp., Corcyra spp., Cornutiplusia spp., Cosmia spp., Cosmopolites spp., Cosmopterix spp., Cossus spp., Costaeonvexa spp., Crambus spp., Creatonotos spp., Crocidolomia spp., Crocidolomia binotalis, Croesia spp., Crymodes spp., Cryptaspasma spp., Cryptoblabes spp., Cryptocala spp., Cryptophlebia spp., Cryptophlebia Ieucotreta, Cryptoptila spp., Ctenopseustis spp., Ctenocephalides spp., Cucullia spp., Curculio spp., Culex spp., Cuterebra spp., Cydia spp., Cydia pomonella, Cymbalophora spp., Dactylethra spp., Dacus spp., Dadica spp., Damalinea spp., Dasychira spp., Decadarchis spp., Decodes spp., Deilephila spp., Deltodes spp., Dendrolimus spp., Depressaria spp., Dermestes spp., Dermanyssus spp., Dermanyssus gallinae, Diabrotica spp., Diachrysia spp., Diaphania spp., Diarsia spp., Diasemia spp., Diatraea spp., Diceratura spp., Dichomeris spp., Dichrocrocis spp., Dichrorampha spp., Dicycla spp., Dioryctria spp., Diparopsis spp., Diparopsis castanea, Dipleurina spp., Diprion spp., Diprionidae, Discestra spp., Distantiella spp., Distantiella theobroma, Ditula spp., Diurnea spp., Doratopteryx spp., Drepana spp., Drosphila spp., Drosphila melanogaster, Dysauxes spp., Dysdercus spp., Dysstroma spp., Eana spp., Earias spp., Ecclitica spp., Ecdytolopha spp., Ecpyrrhorrhoe spp., Ectomyelois spp., Eetropis spp., Egira spp., Elasmopalpus spp., Emmelia spp., mpoasca spp., Empyreuma spp., Enargia spp., Enarmonia spp., Endopiza spp., Endothenia spp., Endotricha spp., Eoreuma spp., Eotetranychus spp., Eotetranychus carpini, Epagoge spp., Epelis spp., Ephestia spp., Ephestiodes spp., Epiblema spp., Epiehoristodes spp., Epinotia spp., Epiphyas spp., Epiplema spp., Epipsestis spp., Epirrhoe spp., Episimus spp., Epitymbia spp., Epllachna spp., Erannis spp., Erastria spp., Eremnus spp., Ereunetis spp., Eriophyes spp., Eriosoma spp., Eriosoma lanigerum, Erythroneura spp., Estigmene spp., Ethmia spp., Etiella spp., Euagrotis spp., Eucosma spp., Euehlaena spp., Euelidia spp., Eueosma spp., Euchistus spp., Eucosmomorpha spp., Eudonia spp., Eufidonia spp., Euhyponomeutoides spp., Eulepitodes spp., Eulia spp., Eulithis spp., Eupithecia spp., Euplexia spp., Eupoecilia spp., Eupoecilia ambiguella, Euproctis spp., Eupsilia spp., Eurhodope spp., Eurois spp., Eurygaster spp., Eurythmia spp., Eustrotia spp., Euxoa spp., Euzophera spp., Evergestis spp., Evippe spp., Exartema spp., Fannia spp., Faronta spp., Feltia spp., Filatima spp., Fishia spp., Frankliniella spp., Fumibotys spp., Gaesa spp., Gasgardia spp., Gastrophilus spp., Gelechia spp., Gilpinia spp., Gilpinia polytoma, Glossina spp., Glyphipterix spp., Glyphodes spp., Gnorimoschemini spp., Gonodonta spp., Gortyna spp., Gracillaria spp., Graphania spp., Grapholita spp., Grapholitha spp., Gravitarmata spp., Gretchena spp., Griselda spp., Gryllotalpa spp., Gynaephora spp., Gypsonoma spp., Hada spp., Haematopinus spp., Halisidota spp., Harpipteryx spp., Harrisina spp., Hedya spp., Helicoverpa spp., Heliophobus spp., Heliothis spp., Hellula spp., Helotropa spp., Hemaris spp., Hercinothrips spp., Herculia spp., Hermonassa spp., Heterogenea spp., Holomelina spp., Homadaula spp., Homoeosoma spp., Homoglaea spp., Homohadena spp., Homona spp., Homonopsis spp., Hoplocampa spp., Hoplodrina spp., Hoshinoa spp., Hxalomma spp., Hydraecia spp., Hydriomena spp., Hyles spp., Hyloicus spp., Hypagyrtis spp., Hypatima spp., Hyphantria spp., Hyphantria cunea, Hypocala spp., Hypocoena spp., Hypodema spp., Hyppobosca spp., Hypsipyla spp., Hyssia spp., Hysterosia spp., Idaea spp., Idia spp., Ipimorpha spp., Isia spp., Isochorista spp., Isophrictis spp., Isopolia spp., Isotrias spp., Ixodes spp., Itame spp., Jodia spp., Jodis spp., Kawabea spp., Keiferia spp., Keiferia Iycopersicella, Labdia spp., Lacinipolia spp., Lambdina spp., Lamprothritpa spp., Laodelphax spp., Lasius spp., Laspeyresia spp., Leptinotarsa spp., Leptinotarsa decemlineata, Leptocorisa spp., Leptostales spp., Lecanium spp., Lecanium comi, Lepidosaphes spp., Lepisma spp., Lepisma saccharina , Lesmone spp., Leucania spp., Leucinodes spp., Leucophaea spp., Leucophaea maderae, Leucoptera spp., Leucoptera scitella, Linognathus spp., Liposcelis spp., Lissorhoptrus spp., Lithacodia spp., Lithocolletis spp., Lithomoia spp., Lithophane spp., Lixodessa spp., Lobesia spp., Lobesia botrana, Lobophora spp., Locusta spp., Lomanaltes spp., Lomographa spp., Loxagrotis spp., Loxostege spp., Lucilia spp., Lymantria spp., Lymnaecia spp., Lyonetia spp., Lyriomyza spp., Macdonnoughia spp., Macrauzata spp., Macronoctua spp., Macrosiphus spp., Malacosoma spp., Maliarpha spp., Mamestra spp., Mamestra brassicae, Manduca spp., Manduca sexta, Marasmia spp., Margaritia spp., Matratinea spp., Matsumuraeses spp., Melanagromyza spp., Melipotes spp., Melissopus spp., Melittia spp., Melolontha spp., Meristis spp., Meritastis spp., Merophyas spp., Mesapamea spp., Mesogona spp., Mesoleuca spp., Metanema spp., Metendothenia spp., Metzneria spp., Micardia spp., Microcorses spp., Microleon spp., Mnesictena spp., Mocis spp., Monima spp., Monochroa spp., Monomorium spp., Monomorium pharaonis, Monopsis spp., Morrisonia spp., Musca spp., Mutuuraia spp., Myelois spp., Mythimna spp., Myzus spp., Naranga spp., Nedra spp., Nemapogon spp., Neodiprion spp., Neosphaleroptera spp., Nephelodes spp., Nephotettix spp., Nezara spp., Nilaparvata spp., Niphonympha spp., Nippoptilia spp., Noctua spp., Nola spp., Notocelia spp., Notodonta spp., Nudaurelia spp., Ochropleura spp., Ocnerostoma spp., Oestrus spp., Olethreutes spp., Oligia spp., Olindia spp., Olygonychus spp., Olygonychus gallinae, Oncocnemis spp., Operophtera spp., Ophisma spp., Opogona spp., Oraesia spp., Orniodoros spp., Orgyia spp., Oria spp., Orseolia spp., Orthodes spp., Orthogonia spp., Orthosia spp., Oryzaephilus spp., Oscinella spp., Oscinella frit, Osminia spp., Ostrinia spp., Ostrinia nubilalis, Otiorhynchus spp., Ourapteryx spp., Pachetra spp., Pachysphinx spp., Pagyda spp., Paleacrita spp., Paliga spp., Palthis spp., Pammene spp., Pandemis spp., Panemeria spp., Panolis spp., Panolis flammea, Panonychus spp., Parargyresthia spp., Paradiarsia spp., Paralobesia spp., Paranthrene spp., Parapandemis spp., Parapediasia spp., Parastichtis spp., Parasyndemis spp., Paratoria spp., Pareromeme spp., Pectinophora spp., Pectinophora gossypiella, Pediculus spp., Pegomyia spp., Pegomyia hyoscyami, Pelochrista spp., Pennisetia spp., Penstemonia spp., Pemphigus spp., Peribatodes spp., Peridroma spp., Perileucoptera spp., Periplaneta spp., Perizoma spp., Petrova spp., Pexicopia spp., Phalonia spp., Phalonidia spp., Phaneta spp., Phlyctaenia spp., Phlyctinus spp., Phorbia spp., Phragmatobia spp., Phricanthes spp., Phthorimaea spp., Phthorimaea operculella, Phyllocnistis spp., Phyllocoptruta spp., Phyllocoptruta oleivora, Phyllonorycter spp., Phyllophila spp., Phylloxera spp., Pieris spp., Pieris rapae, Piesma spp., Planococus spp., Planotortrix spp., Platyedra spp., Platynota spp., Platyptilia spp., Platysenta spp., Plodia spp., Plusia spp., Plutella spp., Plutella xylostella, Podosesia spp., Polia spp., Popillia spp., Polymixis spp., Polyphagotarsonemus spp., Polyphagotarsonemus latus, Prays spp., Prionoxystus spp., Probole spp., Proceras spp., Prochoerodes spp., Proeulia spp., Proschistis spp., Proselena spp., Proserpinus spp., Protagrotis spp., Proteoteras spp., Protobathra spp., Protoschinia spp., Pselnophorus spp., Pseudaletia spp., Pseudanthonomus spp., Pseudaternelia spp., Pseudaulacaspis spp., Pseudexentera spp., Pseudococus spp., Pseudohermenias spp., Pseudoplusia spp., Psoroptes spp., Psylla spp., Psylliodes spp., Pterophorus spp., Ptycholoma spp., Pulvinaria spp., Pulvinaria aethiopica, Pyralis spp., Pyrausta spp., Pyrgotis spp., Pyrreferra spp., Pyrrharctia spp., Quadraspidiotus spp., Rancora spp., Raphia spp., Reticultermes spp., Retinia spp., Rhagoletis spp, Rhagoletis pomonella, Rhipicephalus spp., Rhizoglyphus spp., Rhizopertha spp., Rhodnius spp., Rhophalosiphum spp., Rhopobota spp., Rhyacia spp., Rhyacionia spp., Rhynchopacha spp., Rhyzosthenes spp., Rivula spp., Rondotia spp., Rusidrina spp., Rynchaglaea spp., Sabulodes spp., Sahlbergella spp., Sahlbergella singularis, Saissetia spp., Samia spp., Sannina spp., Sanninoidea spp., Saphoideus spp., Sarcoptes spp., Sathrobrota spp., Scarabeidae, Sceliodes spp., Schinia spp., Schistocerca spp., Schizaphis spp., Schizura spp., Schreckensteinia spp., Sciara spp., Scirpophaga spp., Scirthrips auranti, Scoparia spp., Scopula spp., Scotia spp., Scotinophara spp., Scotogramma spp., Scrobipalpa spp., Scrobipalpopsis spp., Semiothisa spp., Sereda spp., Sesamia spp., Sesia spp., Sicya spp., Sideridis spp., Simyra spp., Sineugraphe spp., Sitochroa spp., Sitobion spp., Sitophilus spp., Sitotroga spp., Solenopsis spp., Smerinthus spp., Sophronia spp., Spaelotis spp., Spargaloma spp., Sparganothis spp., Spatalistis spp., Sperchia spp., Sphecia spp., Sphinx spp., Spilonota spp., Spodoptera spp., Spodoptera littoralis, Stagmatophora spp., Staphylinochrous spp., Stathmopoda spp., Stenodes spp., Sterrha spp., Stomoxys spp., Strophedra spp., Sunira spp., Sutyna spp., Swammerdamia spp., Syllomatia spp., Sympistis spp., Synanthedon spp., Synaxis spp., Syncopacma spp., Syndemis spp., Syngrapha spp., Synthomeida spp., Tabanus spp., Taeniarchis spp., Taeniothrips spp., Tannia spp., Tarsonemus spp., Tegulifera spp., Tehama spp., Teleiodes spp., Telorta spp., Tenebrio spp., Tephrina spp., Teratoglaea spp., Terricula spp., Tethea spp., Tetranychus spp., Thalpophila spp., Thaumetopoea spp., Thiodia spp., Thrips spp., Thrips palmi, Thrips tabaci, Thyridopteryx spp., Thyris spp., Tineola spp., Tipula spp., Tortricidia spp., Tortrix spp., Trachea spp., Trialeurodes spp., Trialeurodes vaporariorum, Triatoma spp., Triaxomera spp., Tribolium spp., Tricodectes spp., Trichoplusia spp., Trichoplusia ni, Trichoptilus spp., Trioza spp., Trioza erytreae, Triphaenia spp., Triphosa spp., Trogoderma spp., Tyria spp., Udea spp., Unaspis spp., Unaspis citri, Utetheisa spp., Valeriodes spp., Vespa spp., Vespamima spp., Vitacea spp., Vitula spp., Witlesia spp., Xanthia spp., Xanthorhoe spp., Xanthotype spp., Xenomicta spp., Xenopsylla spp., Xenopsylla cheopsis, Xestia spp., Xylena spp., Xylomyges spp., Xyrosaris spp., Yponomeuta spp., Ypsolopha spp., Zale spp., Zanclognathus spp., Zeiraphera spp., Zenodoxus spp., Zeuzera spp., Zygaena spp.,

It is also possible to control pests of the class Nematoda using the compounds according to the invention. Such pests include, for example,
root knot nematodes, cyst-forming nematodes and also stem and leaf nematodes;
especially of Heterodera spp., e.g. Heterodera schachtii, Heterodora avenae and Heterodora trifolii; Globodera spp., e.g. Globodera rostochiensis; Meloidogyne spp., e.g. Meloidogyne incognita and Meloidogyne javanica; Radopholus spp., e.g. Radopholus similis; Pratylenchus, e.g. Pratylenchus neglectans and Pratylenchus penetrans; Tylenchulus, e.g. Tylenchulus semipenetrans; Longidorus, Trichodorus, Xiphinema, Ditylenchus, Apheenchoides and Anguina; especially Meloidogyne, e.g. Meloidogyne incognita, and Heterodera, e.g. Heterodera glycines.

An especially important aspect of the present invention is the use of the compounds of formula (I) according to the invention in the protection of plants against parasitic feeding pests.

The action of the compounds according to the invention and the compositions comprising them against animal pests can be significantly broadened and adapted to the given circumstances by the addition of other insecticides, acaricides or nematicides. Suitable additives include, for example, representatives of the following classes of active ingredient: organophosphorus compounds, nitrophenols and derivatives, formamidines, ureas, carbamates, pyrethroids, chlorinated hydrocarbons, neonicotinoids and Bacillus thuringiensis preparations.

Examples of especially suitable mixing partners include: azamethiphos; chlorfenvinphos; cypermethrin, cypermethrin high-cis; cyromazine; diafenthiuron; diazinon; dichlorvos; dicrotophos; dicyclanil; fenoxycarb; fluazuron; furathiocarb; isazofos; iodfenphos; kinoprene; lufenuron; methacriphos; methidathion; monocrotophos; phosphamidon; dinotefuran; profenofos; diofenolan; a compound obtainable from the Bacillus thuringiensis strain GC91 or from strain NCTC11821; pymetrozine; bromopropylate; methoprene; disulfoton; quinalphos; tau-fluvalinate; thiocyclam; thiometon; aldicarb; azinphos-methyl; benfuracarb; bifenthrin; buprofezin; carbofuran; dibutylaminothio; cartap; chlorfluazuron; chlorpyrifos; clothianidin; cyfluthrin; lambda-cyhalothrin; alpha-cypermethrin; zeta-cypermethrin; deltamethrin; diflubenzuron; endosulfan; ethiofencarb; fenitrothion; fenobucarb; fenvalerate; formothion; methiocarb; heptenophos; imidacloprid; isoprocarb; methamidophos; methomyl; mevinphos; parathion; parathion-methyl; phosalone; pirimicarb; propoxur; teflubenzuron; terbufos; triazamate; fenobucarb; tebufenozide; fipronil; beta-cyfluthrin; silafluofen; fenpyroximate; pyridaben; pyridalyl; fenazaquin; pyriproxyfen; pyrimidifen; nitenpyram; acetamiprid; emamectin; emamectin-benzoate; spinosad; a plant extract that is active against insects; a preparation that comprises nematodes and is active against insects; a preparation obtainable from Bacillus subtilis; a preparation that comprises fungi and is active against insects; a preparation that comprises viruses and is active against insects; chlorfenapyr; acephate; acrinathrin; alanycarb; alphamethrin; amitraz; AZ 60541; azinphos A; azinphos M; azocyclotin; bendiocarb; bensultap; beta-cyfluthrin; brofenprox; bromophos A; bufencarb; butocarboxin; butylpyridaben; cadusafos; carbaryl; carbophenothion; chloethocarb; chlorethoxyfos; chlormephos; cis-resmethrin; clocythrin; clofentezine; cyanophos; cycloprothrin; cyhexatin; demeton M; demeton S; demeton-S-methyl; dichlofenthion; dicliphos; diethion; dimethoate; dimethylvinphos; dioxathion; edifenphos; esfenvalerate; ethion; ethofenprox; ethoprophos; etrimphos; fenamiphos; fenbutatin oxide; fenothiocarb; fenpropathrin; fenpyrad; fenthion; fluazinam; flucycloxuron; flucythrinate; flufenoxuron; flufenprox; fonophos; fosthiazate; fubfenprox; HCH; hexaflumuron; hexythiazox; flonicamid; iprobenfos; isofenphos; isoxathion; ivermectin; malathion; mecarbam; mesulfenphos; metaldehyde; metolcarb; milbemectin; moxidectin; naled; NC 184; nithiazine; omethoate; oxamyl; oxydemethon M; oxydeprofos; permethrin; phenthoate; phorate; phosmet; phoxim; pirimiphos M; pirimiphos E; promecarb; propaphos; prothiofos; prothoate; pyrachlophos; pyradaphenthion; pyresmethrin; pyrethrum; tebufenozide; salithion; sebufos; sulfotep; sulprofos; tebufenpyrad; tebupirimphos; tefluthrin; ternephos; terbam; tetrachlorvinphos; thiacloprid; thiafenox; thiamethoxam; thiodicarb; thiofanox; thionazin; thuringiensin; tralomethrin; triarathene; triazophos; triazuron; trichlorfon; triflumuron; trimethacarb; vamidothion; xylylcarb; etoxazole; zetamethrin; indoxacarb; methoxyfenozide; bifenazate; XMC (3,5-xylyl methylcarbamate); or the fungus pathogen Metarhizium anisopliae.

The compounds according to the invention can be used to control, i.e. to inhibit or destroy, pests of the mentioned type occurring on plants, especially on useful plants and ornamentals in agriculture, in horticulture and in forestry, or on parts of such plants, such as the fruits, blossoms, leaves, stems, tubers or roots, while in some cases plant parts that grow later are still protected against those pests.

Target crops include especially cereals, such as wheat, barley, rye, oats, rice, maize and sorghum; beet, such as sugar beet and fodder beet; fruit, e.g. pomes, stone fruit and soft fruit, such as apples, pears, plums, peaches, almonds, cherries and berries, e.g. strawberries, raspberries and blackberries; leguminous plants, such as beans, lentils, peas and soybeans; oil plants, such as rape, mustard, poppy, olives, sunflowers, coconut, castor oil, cocoa and groundnuts; cucurbitaceae, such as marrows, cucumbers and melons; fibre plants, such as cotton, flax, hemp and jute; citrus fruits, such as oranges, lemons, grapefruit and mandarins; vegetables, such as spinach, lettuce, asparagus, cabbages, carrots, onions, tomatoes, potatoes and paprika; lauraceae, such as avocado, cinnamon and camphor; and tobacco, nuts, coffee, aubergines, sugar cane, tea, pepper, vines, hops, bananas, natural rubber plants and ornamentals.

Further areas of use of the compounds according to the invention are the protection of stored goods and storerooms and the protection of raw materials, and also in the hygiene sector, especially the protection of domestic animals and productive livestock against pests of the mentioned type, more especially the protection of domestic animals, especially cats and dogs, from infestation by fleas, ticks and nematodes.

The invention therefore relates also to pesticidal compositions, such as emulsifiable concentrates, suspension concentrates, directly sprayable or dilutable solutions, spreadable pastes, dilute emulsions, wettable powders, soluble powders, dispersible powders, wettable powders, dusts, granules and encapsulations of polymer substances, that comprise at least one of the compounds according to the invention, the choice of formulation being made in accordance with the intended objectives and the prevailing circumstances.

The active ingredient is used in those compositions in pure form, a solid active ingredient, for example, in a specific particle size, or preferably together with at least one of the adjuvants customary in formulation technology, such as extenders, e.g. solvents or solid carriers, or surface-active compounds (surfactants). In the area of parasite control in humans, domestic animals, productive livestock and pets it will be self-evident that only physiologically tolerable additives are used.

Solvents are, for example: non-hydrogenated or partly hydrogenated aromatic hydrocarbons, preferably fractions C₈ to C₁₂ of alkylbenzenes, such as xylene mixtures, alkylated naphthalenes or tetrahydronaphthalene, aliphatic or cycloaliphatic hydrocarbons, such as paraffins or cyclohexane, alcohols, such as ethanol, propanol or butanol, glycols and ethers and esters thereof, such as propylene glycol, dipropylene glycol ether, ethylene glycol or ethylene glycol monomethyl or -ethyl ether, ketones, such as cyclohexanone, isophorone or diacetone alcohol, strongly polar solvents, such as N-methylpyrrolid-2-one, dimethyl sulfoxide or N,N-dimethylformamide, water, non-epoxidized or epoxidized plant oils, such as non-epoxidized or epoxidized rapeseed, castor, coconut or soya oil, and silicone oils.

The solid carriers used, for example for dusts and dispersible powders, are as a rule natural rock powders, such as calcite, talc, kaolin, montmorillonite or attapulgite. Highly disperse silicic acids or highly disperse absorbent polymers can also be added to improve the physical properties. Granular adsorptive granule carriers are porous types, such as pumice, crushed brick, sepiolite or bentonite, and non-sorbent carrier materials are calcite or sand. A large number of granular materials of inorganic or organic nature can furthermore be used, in particular dolomite or comminuted plant residues.

Surface-active compounds are, depending on the nature of the active compound to be formulated, nonionic, cationic and/or anionic surfactants or surfactant mixtures with good emulsifying, dispersing and wetting properties. The surfactants listed below are to be regarded only as examples; many other surfactants which are customary in formulation technology and are suitable according to the invention are described in the relevant literature.

Nonionic surfactants are, in particular, polyglycol ether derivatives of aliphatic or cycloaliphatic alcohols, saturated or unsaturated fatty acids and alkylphenols, which can contain 3 to 30 glycol ether groups and 8 to 20 carbon atoms in the (aliphatic) hydrocarbon radical and 6 to 18 carbon atoms in the alkyl radical of the alkylphenols. Substances which are furthermore suitable are water-soluble polyethylene oxide adducts, containing 20 to 250 ethylene glycol ether and 10 to 100 propylene glycol ether groups, on propylene glycol, ethylene diaminopolypropylene glycol and alkyl polypropylene glycol having 1 to 10 carbon atoms in the alkyl chain. The compounds mentioned usually contain 1 to 5 ethylene glycol units per propylene glycol unit. Examples are nonylphenol-polyethoxyethanols, castor oil polyglycol ethers, polypropylene-polyethylene oxide adducts, tributylphenoxypolyethoxyethanol, polyethylene glycol and octylphenoxypolyethoxyethanol. Other substances are fatty acid esters of polyoxyethylene sorbitan, such as polyoxyethylene sorbitan trioleate.

The cationic surfactants are, in particular, quaternary ammonium salts which contain, as substituents, at least one alkyl radical having 8 to 22 C atoms and, as further substituents, lower, non-halogenated or halogenated alkyl, benzyl or lower hydroxyalkyl radicals. The salts are preferably in the form of halides, methyl-sulfates or ethyl-sulfates. Examples are stearyl-trimethyl-ammonium chloride and benzyl-di-(2-chloroethyl)-ethylammonium bromide.

Suitable anionic surfactants can be both water-soluble soaps and water-soluble synthetic surface-active compounds. Suitable soaps are the alkali metal, alkaline earth metal and substituted or unsubstituted ammonium salts of higher fatty acids (C₁₀-C₂₂), such as the sodium or potassium salts of oleic or stearic acid, or of naturally occurring fatty acid mixtures, which can be obtained, for example, from coconut oil or tall oil; and furthermore also the fatty acid methyl-taurine salts. However, synthetic surfactants are more frequently used, in particular fatty sulfonates, fatty sulfates, sulfonated benzimidazole derivatives or alkylarylsulfonates. The fatty sulfonates and sulfates are as a rule in the form of alkali metal, alkaline earth metal or substituted or unsubstituted ammonium salts and in general have an alkyl radical of 8 to 22 C atoms, alkyl also including the alkyl moiety of acyl radicals; examples are the sodium or calcium salt of ligninsulfonic acid, of dodecylsulfuric acid ester or of a fatty alcohol sulfate mixture prepared from naturally occurring fatty acids. These also include the salts of sulfuric acid esters and sulfonic acids of fatty alcohol-ethylene oxide adducts. The sulfonated benzimidazole derivatives preferably contain 2 sulfonic acid groups and a fatty acid radical having about 8 to 22 C atoms. Alkylarylsulfonates are, for example, the sodium, calcium or triethanolammonium salts of dodecylbenzenesulfonic acid, of dibutylnaphthalenesulfonic acid or of a naphthalenesulfonic acid-formaldehyde condensation product. Corresponding phosphates, such as salts of the phosphoric acid ester of a p-nonylphenol-(4-14)-ethylene oxide adduct or phospholipids, can further also be used.

The compositions as a rule comprise 0.1 to 99 %, in particular 0.1 to 95 %, of active compound and 1 to 99.9 %, in particular 5 to 99.9 %, of - at least - one solid or liquid auxiliary, it being possible as a rule for 0 to 25 %, in particular 0.1 to 20 %, of the composition to be surfactants (% is in each case per cent by weight). While concentrated compositions are more preferred as commercial goods, the end user as a rule uses dilute compositions which comprise considerably lower concentrations of active compound. Preferred compositions are composed, in particular, as follows (% = per cent by weight):

| Emulsifiable concentrates: | |
|---|---|
| active ingredient: | 1 to 90%, preferably 5 to 20% |
| surfactant: | 1 to 30%, preferably 10 to 20% |
| solvent: | 5 to 98%, preferably 70 to 85% |
| | |

| Dusts: | |
|---|---|
| active ingredient: | 0.1 to 10%, preferably 0.1 to 1% |
| solid carrier: | 99.9 to 90%, preferably 99.9 to 99% |
| | |

| Suspension concentrates: | |
|---|---|
| active ingredient: | 5 to 75%, preferably 10 to 50% |
| water: | 94 to 24%, preferably 88 to 30% |
| surfactant: | 1 to 40%, preferably 2 to 30% |
| | |

| Wettable powders: | |
|---|---|
| active ingredient: | 0.5 to 90%, preferably 1 to 80% |
| surfactant: | 0.5 to 20%, preferably 1 to 15% |
| solid carrier: | 5 to 99%, preferably 15 to 98% |
| | |

| Granules: | |
|---|---|
| active ingredient: | 0.5 to 30%, preferably 3 to 15% |
| solid carrier: | 99.5 to 70%, preferably 97 to 85% |

The compositions according to the invention may also comprise further solid or liquid adjuvants, such as stabilisers, e.g. vegetable oils or epoxidised vegetable oils (e.g. epoxidised coconut oil, rapeseed oil or soybean oil), antifoams, e.g. silicone oil, preservatives, viscosity regulators, binders and/or tackifiers as well as fertilisers or other active ingredients for obtaining special effects, e.g. acaricides, bactericides, fungicides, nematicides, molluscicides or selective herbicides.

The crop protection products according to the invention are prepared in known manner, in the absence of adjuvants, e.g. by grinding, sieving and/or compressing a solid active ingredient or mixture of active ingredients, for example to a certain particle size, and in the presence of at least one adjuvant, for example by intimately mixing and/or grinding the active ingredient or mixture of active ingredients with the adjuvant(s). The invention relates likewise to those processes for the preparation of the compositions according to the invention and to the use of the compounds of formula (I) in the preparation of those compositions.

The invention relates also to the methods of application of the crop protection products, i.e. the methods of controlling pests of the mentioned type, such as spraying, atomising, dusting, coating, dressing, scattering or pouring, which are selected in accordance with the intended objectives and the prevailing circumstances, and to the use of the compositions for controlling pests of the mentioned type. Typical rates of concentration are from 0.1 to 1000 ppm, preferably from 0.1 to 500 ppm, of active ingredient. The rates of application per hectare are generally from 1 to 2000 g of active ingredient per hectare, especially from 10 to 1000 g/ha, preferably from 20 to 600 g/ha, more especially from 20 to 100 g/ha.

A preferred method of application in the area of crop protection is application to the foliage of the plants (foliar application), the frequency and the rate of application being dependent upon the risk of infestation by the pest in question. However, the active ingredient can also penetrate the plants through the roots (systemic action) when the locus of the plants is impregnated with a liquid formulation or when the active ingredient is incorporated in solid form into the locus of the plants, for example into the soil, e.g. in granular form (soil application). In the case of paddy rice crops, such granules may be applied in metered amounts to the flooded rice field.

The crop protection products according to the invention are also suitable for protecting plant propagation material, e.g. seed, such as fruits, tubers or grains, or plant cuttings, against animal pests. The propagation material can be treated with the composition before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example to the seed furrow during sowing. The invention relates also to such methods of treating plant propagation material and to the plant propagation material so treated, thereby conferring pest-resistance to such material.

The following Examples serve to illustrate the invention. They do not limit the invention. Temperatures are given in degrees Celsius; mixing ratios of solvents are given in parts by volume.

### Preparation Examples:

### Example A1.1: 4'-Desoxy-4'-(R)-(N-acetyl-N-methyl-amino)-avermectin B1 monosaccharide

4 g 4'-desoxy-4'-(R)-(N-methyl-amino)-avermectin B1 monosaccharide are dissolved in a mixture of 30 ml ethyl acetate and 30 ml saturated aqueous sodium bicarbonate. 0.77 ml acetyl chloride are added, and the mixture is stirred vigorously for 3 hours at room temperature. Then the phases are separated; the organic phase is dried over sodium sulfate and the solvents are distilled off. The residue is purified by chromatography on silica gel with hexane/ethyl acetate, yielding 4'-desoxy-4'-(R)-(N-acetyl-N-methyl-amino)-avermectin B1 monosaccharide.

### Example A2.1: 4'-Desoxy-4'-(R)-(N-methoxycarbonyl-N-methyl-amino)-avermectin B1 monosaccharide

4 g 4'-desoxy-4'-(R)-(N-methyl-amino)-avermectin B1 monosaccharide are dissolved in a mixture of 30 ml ethyl acetate and 30 ml saturated aqueous sodium bicarbonate. 0.83 ml methyl chloroformate are added, and the mixture is stirred vigorously for 3 hours at room temperature. Then the phases are separated; the organic phase is dried over sodium sulfate and the solvents are distilled off. The residue is purified by chromatography on silica gel with hexane/ethyl acetate, yielding 4'-desoxy-4'-(R)-(N-methoxycarbonyl-N-methyl-amino)-avermectin B1 monosaccharide.

### Example A3.1: 4'-Desoxy-4'-(R)-(N-methyl-amino)-avermectin B1 monosaccharide

Step 1: 10 g 4'-desoxy-4'-oxo-5-O-t-butyldimethylsilyl-avermectin B1 monosaccharide, 6.5 ml heptamethyldisilazane and 7.5 g zinc bromide are dissolved in 75 ml isopropyl acetate. The mixture is stirred at 50 °C for 3 hours, and subsequently cooled in an ice bath. At a temperature of 0 - 5 °C, 0.68 g sodium borohydride are added, and the mixture is stirred for one hour at 0 - 5 °C, then allowed to warm to room temperature for one more hour. Then about 20 ml of a 10% aqueous solution of acetic acid are added, resulting in a pH of about 6. Then 2N aqueous sodium hydroxide is added until the pH is 8. The resulting suspension is filtered, to remove insoluble material. Then the phases are separated; the organic phase is washed with brine, dried over sodium sulfate and the solvent is distilled off. The residue is purified by chromatography on silica gel with hexane/ethyl acetate, yielding 4'-desoxy-4'-(R)-(N-methyl-amino)-5-O-t-butyldimethylsilyl-avermectin B1 monosaccharide.

Step 2: 7.1 g 4'-desoxy-4'-(R)-(N-methyl-amino)-5-O-t-butyldimethylsilyl-avermectin B1 monosaccharide are dissolved in 80 ml tetrahydrofuran, then 26 ml of a stock solution are added, which is prepared from 250 g 70% HF-Pyridin, 275 ml tetrahydrofuran and 125 ml pyridine. The mixture is stirred at room temperature for 24 hours, poured into water, and extracted with ethyl acetate. Then the phases are separated; the organic phase is washed with saturated sodium bicarbonate, dried over sodium sulfate and the solvent is distilled off. The residue is purified by chromatography on silica gel with hexane/ethyl acetate, yielding 4'-desoxy-4'-(R)-(N-methyl-amino)-avermectin B1 monosaccharide.

### Example A4.1: 4'-Desoxy-4'-(S)-azido-avermectin B1 monosaccharide

Step1: 9.8 g 4'-(R)-5-O-t-butyldimethylsilyl-avermection B1 monosaccharide are dissolved in 300 ml dichloromethane under an atmosphere of argon and the solution is cooled to -35°C. Subsequently, 7.3 g N,N-dimethyl-4-aminopyridine, 7.8 g ethyl-di-isopropylamine and 11.3 g trifluoromethanesulfonic anhydride are added under vigorous stirring. The mixture is allowed to warm to 0°C, then stirred at 0°C for 2 hours. Then the mixture is poured on ice, extracted with diethylether, the phases are separated; the organic phase is washed with brine, dried over sodium sulphate and the solvent is distilled off. The residue is purified by chromatography on silica gel with hexane/ethyl acetate, yielding 4'-(R)-4'-O-trifluoromethylsulfonyl-5-O-t-butyldimethylsilyl-avermectin B1 monosaccharide.

Step 2: 8.8 g 4'-(R)-4'-O-trifluoromethylsulfonyl-5-O-t-butyldimethylsilyl-avermectin B1 monosaccharide are dissolved in 50 ml N,N-dimethyl-formamide, 1 g sodium azide are added, and the mixture is stirred at room temperature for 15 hours. Then the mixture is extracted with ethyl acetate and water, the phases are separated; the organic phase is washed with brine, dried over sodium sulfate and the solvent is distilled off. The residue is purified by chromatography on silica gel with hexane/ethyl acetate, yielding 4'-desoxy-4'-(S)-azido-5-O-t-butyldimethylsilyl-avermectin B1 monosaccharide.

Step 3: 6.9 g 4'-desoxy-4'-*(S)*-azido-5-O-t-butyldimethylsilyl-avermectin B1 monosaccharide are dissolved in 75 ml tetrahydrofuran, then 25 ml of a stock solution are added, which is prepared from 250 g 70% HF-Pyridin, 275 ml tetrahydrofuran and 125 ml pyridine. The mixture is stirred at room temperature for 24 hours, poured into water, and extracted with ethyl acetate. Then the phases are separated; the organic phase is dried over sodium sulfate and the solvents are distilled off. The residue is purified by chromatography on silica gel with hexane/ethyl acetate, yielding 4'-desoxy-4'-(S)-azido-avermectin B1 monosaccharide.

### Example A4.2: 4'-Desoxy-4'-(S)-amino-avermectin B1 monosaccharide

3 g 4'-desoxy-4'-(S)-azido-avermectin B1 monosaccharide are dissolved in 200 ml tetrahydrofuran, then 15 ml of a 1 M solution of trimethylphosphine in tetrahydrofuran are added. The mixture is stirred at 50 °C for 48 hours. Then 60 ml of a 0.001 M aqueous solution of sodium hydroxide are added, and the mixture is stirred for additional 18 hours at 45 °C. After cooling to room temperature, the mixture is extracted with ethyl acetate and water. Then the phases are separated; the organic phase is washed with brine, dried over sodium sulfate and the solvents are distilled off. The residue is purified by chromatography on silica gel with hexane/ethyl acetate, yielding 4'-desoxy-4'-(S)-amino-avermectin B1 monosaccharide.

### Example A4.3: 4'-Desoxy-4'-(S)-(N-ethyl-amino)-avermectin B1 monosaccharide

5 g 4'-desoxy-4'-(S)-azido-avermectin B1 monosaccharide are dissolved in 250 ml tetrahydrofuran, then 25 ml of a 1 M solution of trimethylphosphine in tetrahydrofuran are added. The mixture is stirred at 65 °C for 4 hours. After cooling to 40 °C, 1.1 ml acetaldehyde are added, and stirring is continued at 40 °C for further 15 hours. Then the solvent is removed by evaporation in vacuo, the residue is dissolved in 150 ml methanol, 0.23 g sodium borohydride are added, and the mixture is stirred for 2 hours at room temperature, and subsequently extracted with ethyl acetate and water. Then the phases are separated; the organic phase is washed with brine, dried over sodium sulfate and the solvents are distilled off. The residue is purified by chromatography on silica gel with hexane/ethyl acetate, yielding 4'-desoxy-4'-(S)-(N-ethyl-amino)-avermectin B1 monosaccharide.

Tables A1 to A9 list compounds that have been prepared and provide their chromatographic characterization. Accordingly, it is considered that each of the compounds in Tables 1 to 144 can also be prepared. In the Tables, where necessary, the symbol 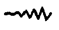 denotes the bond through which the radical in question is attached to the skeleton.

Since in most cases the compounds are present as mixtures of the monosaccharide derivatives of avermectin B1 and B2, characterization by customary physical data such as melting point or refractive index makes little sense. For this reason, the compounds are characterized by the retention times which are determined in an analysis by HPLC (high performance liquid chromatography). Here, the term B1a refers to the main component in which R₁ is sec-butyl, with a content of usually more than 80%. B1b denotes the minor component in which R₁ is isopropyl. The compounds where two retention times are given both for the B1 a and for the B1 b derivative are mixtures of diastereomers which can be separated chromatographically. In the case of compounds where a retention time is given only in column B1a or only in column B1b, the pure B1a or B1b component, respectively, can be obtained during work-up. The correct structures of the B1a and B1b components are assigned by mass spectrometry.
The following method is used for HPLC analysis for compounds listed in Tables A1 to A6:

| HPLC gradient conditions | | | |
|---|---|---|---|
| Solvent A: | 0.01% of trifluoroacetic acid in H₂O | | |
| Solvent B: | 0.01% of trifluoroacetic acid in CH₃CN | | |

| Time [min] | A [%] | B [%] | Flow rate [µl/min] |
|---|---|---|---|
| 0 | 80 | 20 | 500 |
| 0.1 | 50 | 50 | 500 |
| 10 | 5 | 95 | 500 |
| 15 | 0 | 100 | 500 |
| 17 | 0 | 100 | 500 |
| 17.1 | 80 | 20 | 500 |
| 22 | 80 | 20 | 500 |
| Type of column | YMC-Pack ODS-AQ | | |
| Column length | 125 mm | | |
| Internal diameter of column: | 2 mm | | |
| Temperature | 40°C | | |

The YMC-Pack ODS-AQ column used for the chromatography of the compounds is manufactured by YMC, Alte Raesfelderstrasse 6, 46514 Schermbeck, Germany.

**Table A1: Compounds of the formula in which R₁ is sec-butyl or isopropyl**

| | | | | |
|---|---|---|---|---|
| 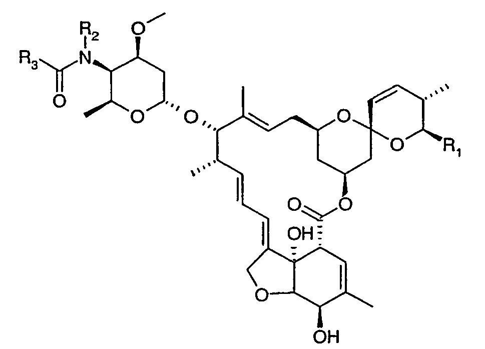 | | | | |

| No. | R₂ | R₃ | Retention time (min) | |
|---|---|---|---|---|
| | | | B1a | B1b |
| A1.1 | CH₃ | CH₃ | 7.85 | 7.13 |
| A1.2 | CH₃ | phenyl | 10.11 | 8.61 |
| A1.3 | CH₃ | benzyl | 10.12 | 9.39 |
| A1.4 | CH₃ | 3-pyridyl | 6.88 | 5.48 |
| A1.5 | H | CH₂OCH₃ | 7.33 | 6.99 |
| A1.6 | H | CH₃ | 6.41 | 5.86 |
| A1.7 | H | CH₂CO₂CH₃ | 6.77 | 6.19 |
| A1.8 | H | SCH₂CH₃ | 9.21 | |
| A1.9 | H | CH₂CH₃ | 7.04 | |
| A1.10 | H | CH(CH₃)₂ | 8.80 | 7.36 |
| A1.11 | H | CHO | 6.29 | 5.76 |
| A1.12 | CH₃ | CH₂OCH₃ | 9.17 | |
| A1.13 | CH₃ | CH₂CH₃ | 10.40 | 9.71 |
| A1.14 | CH₃ | cyclopropyl | 10.99 | 10.19 |
| A1.15 | CH₃ | CH₂CH₂Cl | 11.31 | 10.61 |
| A1.16 | CH₃ | C(CH₃)₂CH₂Cl | 12.75 | |
| A1.17 | CH₃ | H | 9.23 | |
| A1.18 | CH₃ | 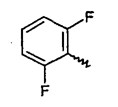 | 12.69 | |

**Table A2: Compounds of the formula**

| | | | | |
|---|---|---|---|---|
| 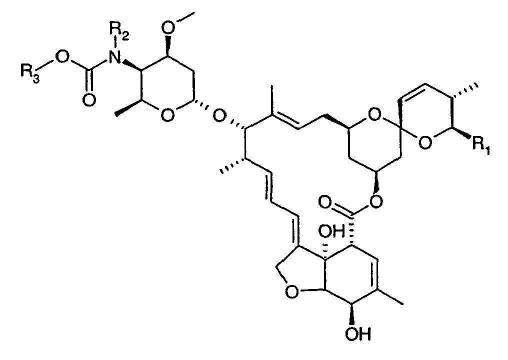 | | | | |
| in which R₁ is sec-butyl or isopropyl | | | | |

| No. | R₂ | R₃ | Retention time (min) | |
|---|---|---|---|---|
| | | | B1a | B1b |
| A2.1 | CH₃ | CH₃ | 9.51 | 8.74 |
| A2.2 | H | CH₃ | 7.79 | 7.15 |
| A2.3 | H | OCH(CH₃)₂ | 9.39 | |
| A2.4 | H | OCH₂CH₃ | 8.59 | |
| A2.5 | CH₃ | OCH₂CH=CH₂ | 12.27 | 11.74 |
| A2.6 | CH₃ | OCH₂CH₂Cl | 12.21 | |
| A2.7 | CH₃ | OCH₂CH₃ | 12.11 | 11.42 |
| A2.8 | CH₃ | OCH(CH₃)₂ | 12.53 | 12.00 |

**Table A3: Compounds of the formula in which R₁ is sec-butyl or isopropyl**

| | | | | |
|---|---|---|---|---|
| 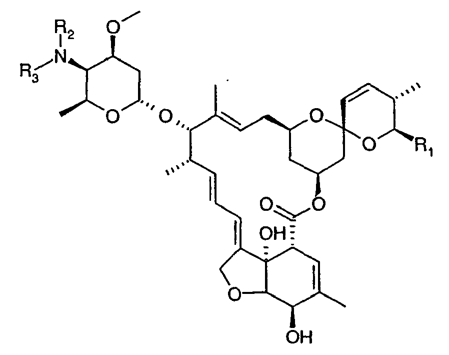 | | | | |

| No. | R₂ | R₃ | Retention time (min) | |
|---|---|---|---|---|
| | | | B1a | B1b |
| A3.1 | H | CH₃ | 3.95 | 3.79 |
| A3.2 | H | CH₂(*p*NO₂C₆H₄) | 4.80 | 4.53 |
| A3.3 | H | CH₂(*p*BrC₆H₄) | 5.17 | 4.91 |
| A3.4 | H | CH₂CH₂CH₂CH₃ | 4.79 | 4.43 |
| A3.5 | H | CHCH=CH₂ | 4.26 | 4.00 |
| A3.6 | H | CH₂CO₂CH₂CH₂OCH₃ | 4.48 | 4.16 |
| A3.7 | CH₂CO₂CH₂CH₂OCH₃ | CH₂CO₂CH₂CH₂OCH₃ | 9.65 | |
| A3.8 | H | CH₂CH=C(CH₃)₂ | 4.82 | |
| A3.9 | H | CH₂CN | 9.57 | 8.75 |
| A3.10 | H | CH₂CO₂CH₃ | 4.37 | 4.11 |

**Table A4: Compounds of the formula in which R₁ is sec-butyl or isopropyl**

| | | | | |
|---|---|---|---|---|
| 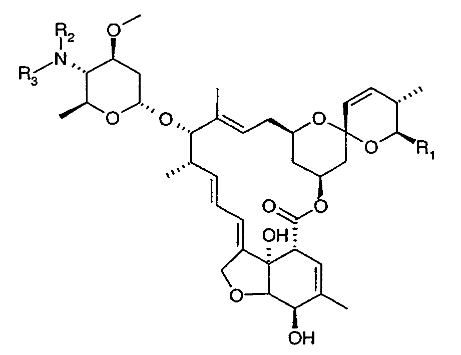 | | | | |

| No. | R₂ R₃ | | Retention time (min) | |
|---|---|---|---|---|
| | | | B1a | B1b |
| A4.1 | -N⁺=N⁻ | | 10.86 | |
| A4.2 | H | H | 4.11 | 3.89 |
| A4.3 | H | ethyl | 4.59 | 4.27 |
| A4.4 | H | n-propyl | 4.69 | 4.43 |
| A4.5 | H | benzyl | 5.44 | 5.07 |
| A4.6 | H | CH₂CH₂CH₂CH₃ | 5.87 | 5.12 |
| A4.7 | CH₂CH₂CH₂CH₃ | CH₂CH₂CH₂CH₃ | 6.93 | 6.51 |
| A4.8 | H | CHCH=CH₂ | 5.87 | 5.39 |
| A4.9 | CHCH=CH₂ | CHCH=CH₂ | 6.93 | 6.40 |
| A4.10 | H | CH₂CH=C(CH₃)₂ | 5.92 | 5.60 |
| A4.11 | H | CH₂(*p*BrC₆H₄) | 7.09 | 6.67 |
| A4.12 | CH₂(*p*BrC₆H₄) | CH₂(*p*BrC₆H₄) | 16.43 | 15.52 |
| A4.13 | H | CH₂(*p*NO₂C₆H₄) | 6.30 | 5.92 |
| A4.14 | CH₂(*p*NO₂C₆H₄) | CH₂(*p*NO₂C₆H₄) | 13.02 | 12.49 |
| A4.15 | H | CH₂CO₂CH₃ | 5.60 | 5.12 |
| A4.16 | H | CH₂CO₂CH₂CH₂OCH₃ | 5.33 | 4.96 |
| A4.17 | H | CH₂CN | 8.64 | 7.95 |
| A4.18 | H | 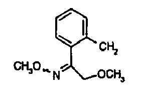 | 6.35 | 5.92 |

**Table A5: Compounds of the formula in which R₁ is sec-butyl or isopropyl**

| | | | | |
|---|---|---|---|---|
| 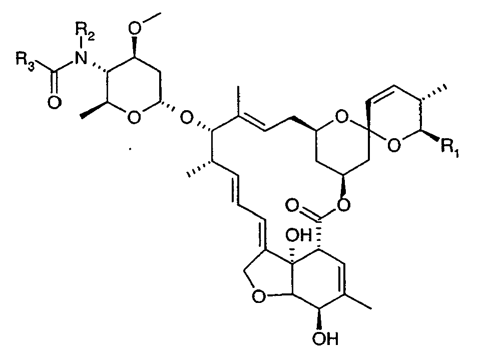 | | | | |

| No. | R₂ | R₃ | Retention time (min) | |
|---|---|---|---|---|
| | | | B1a | B1b |
| A5.1 | H | H | 7.31 | 6.56 |
| A5.2 | H | CH₃ | 6.93 | 6.29 |
| A5.3 | H | CH₂CH₃ | 7.52 | |
| A5.4 | H | CH(CH₃)₂ | 8.06 | 7.36 |
| A5.5 | H | CH₂OCH₃ | 7.41 | 6.67 |
| A5.6 | H | CH₂CO₂CH₃ | 7.48 | 6.78 |
| A5.7 | H | cyclopropyl | 7.79 | 7.09 |
| A5.8 | H | 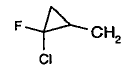 | 8.49 | 7.90 |

**Table A6: Compounds of the formula in which R₁ is sec-butyl or isopropyl**

| | | | | |
|---|---|---|---|---|
| 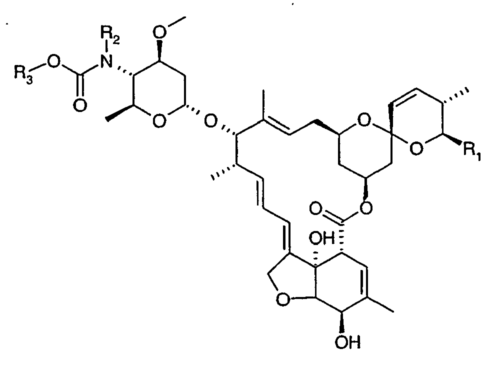 | | | | |

| No. | R₂ | R₃ | Retention time (min) | |
|---|---|---|---|---|
| | | | B1a | B1b |
| A6.1 | H | CH₃ | 8.48 | 7.79 |
| A6.2 | H | OCH(CH₃)₂ | 9.71 | 8.96 |
| A6.3 | H | OCH₂CH₃ | 9.07 | 8.37 |

**Table A7: Compounds of the formula in which R₁ is sec-butyl or isopropyl**

| | | | | | |
|---|---|---|---|---|---|
| 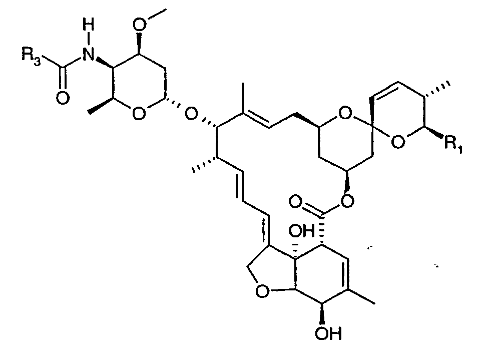 | | | | | |
| and wherein the following method is used for the HPLC analysis: | | | | | |

| HPLC gradient conditions | | | | | |
|---|---|---|---|---|---|
| solvent A (%): | 45.0 H₂O (containing 0.1 % HCO₂H) | | | | |
| solvent B (%): | 45.0 H₂O (containing 0.1 % HCO₂H) | | | | |
| solvent C (%): | CH₃CN (containing 0.1 % HCO₂H) | | | | |
| solvent D (%): | CH₃CN (containing 0.1 % HCO₂H) | | | | |

| Time [min] | A [%] | B [%] | C [%] | D [%] | flow rate [ml/min] |
|---|---|---|---|---|---|
| 0.00 | 45 | 45 | 5 | 5 | 3.50 |
| 2.50 | 0 | 0 | 50 | 50 | 3.50 |
| 2.80 | 0 | 0 | 50 | 50 | 3.50 |
| 3.00 | 45 | 0 | 5 | 5 | 3.50 |
| Column*: | Waters ATLANTIS^{™} | | | | |
| column length: | 20 mm | | | | |
| column internal diameter: | 4.6 mm | | | | |
| temperature: | 40 °C | | | | |

| | | | | | |
|---|---|---|---|---|---|
| The Waters ATLANTIS^{™} column used for chromatography of the compounds is available from Water AG, Dorfstrasse, 5102 Rupperswill, Switzerland (Column serial number: W32021 C09) | | | | | |

| No. | R₃ | Retention time (min) | |
|---|---|---|---|
| | | B1a | B1b |
| A7.1 | 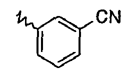 | 2.08 | |
| A7.2 | 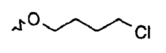 | 2.20 | |
| A7.3 | 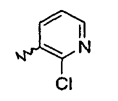 | 1.94 | |
| A7.4 | 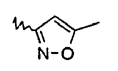 | 2.16 | |
| A7.5 | 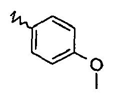 | 2.09 | |
| A7.6 | 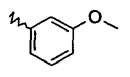 | 2.16 | |
| A7.7 | 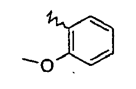 | 2.24 | |
| A7.8 | 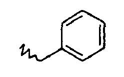 | 2.10 | |
| A7.9 | 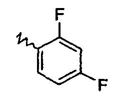 | 2.22 | |
| A7.10 | 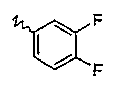 | 2.20 | |
| A7.11 | 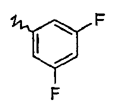 | 2.24 | |
| A7.12 | 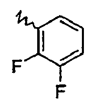 | 2.21 | |
| A7.13 | 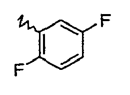 | 2.20 | |
| A7.14 | 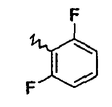 | 2.04 | |
| A7.15 | 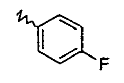 | 2.12 | |
| A7.16 | 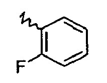 | 2.21 | |
| A7.17 | 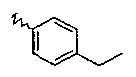 | 2.28 | |
| A7.18 | 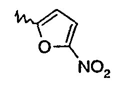 | 2.10 | |
| A7.19 | 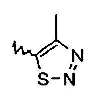 | 2.00 | |
| A7.20 | 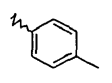 | 2.22 | |
| A7.21 | 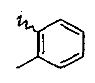 | 2.20 | |
| A7.22 | 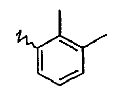 | 2.20 | |
| A7.23 | 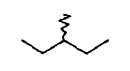 | 2.19 | |
| A7.24 | 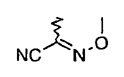 | 2.12 | |
| A7.25 | 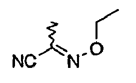 | 2.20 | |
| A7.26 | 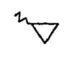 | 1.89 | |
| A7.27 | 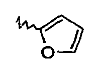 | 2.05 | |
| A7.28 | 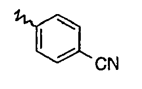 | 2.03 | |
| A7.29 | 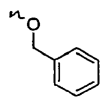 | 2.24 | |
| A7.30 | 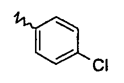 | 2.24 | |
| A7.31 | 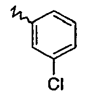 | 2.26 | |
| A7.32 | 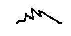 | 1.85 | |
| A7.33 | 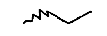 | 2.00 | |
| A7.34 | 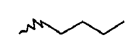 | 2.16 | |
| A7.35 | 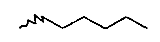 | 2.28 | |
| A7.36 | 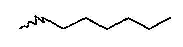 | 2.40 | |
| A7.37 | 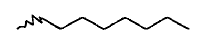 | 2.44 | |
| A7.38 | 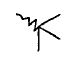 | 2.21 | |
| A7.39 | 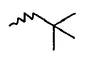 | 2.17 | |
| A7.40 | 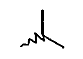 | 2.00 | |
| A7.41 | 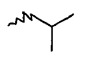 | 2.10 | |
| A7.42 | 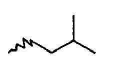 | 2.16 | |
| A7.43 | 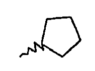 | 2.14 | |
| A7.44 | 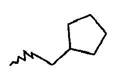 | 2.27 | |
| A7.45 | 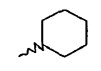 | 2.20 | |
| A7.46 | 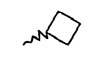 | 2.06 | |
| A7.47 | 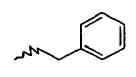 | 2.15 | |
| A7.48 | 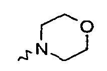 | 1.80 | |
| A7.49 | 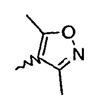 | 2.01 | |
| A7.50 | 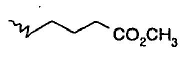 | 1.92 | |
| A7.51 | 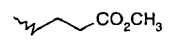 | 1.90 | |
| A7.52 | 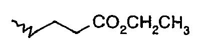 | 1.96 | |
| A7.53 | 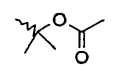 | 2.07 | |
| A7.54 | 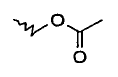 | 1.84 | |
| A7.55 | 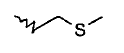 | 1.93 | |
| A7.56 | 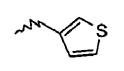 | 2.05 | |
| A7.57 | 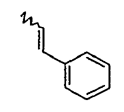 | 2.15 | |
| A7.58 | 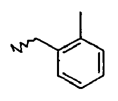 | 2.20 | |
| A7.59 | 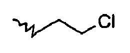 | 1.59 | |
| A7.60 | 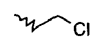 | 2.07 | |
| A7.61 | 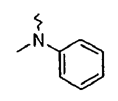 | 2.26 | |
| A7.62 | 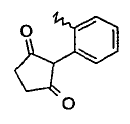 | 1.90 | |
| A7.63 | 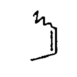 | 1.94 | |
| A7.64 | 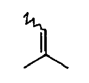 | 2.10 | |
| A7.65 | 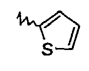 | 2.08 | |

**Table A8: Compounds of the formula in which R₁ is sec-butyl or isopropyl**

| | | | | | |
|---|---|---|---|---|---|
| 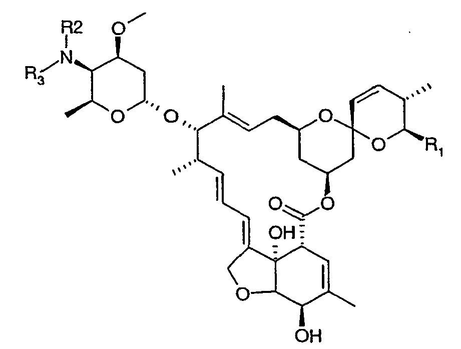 | | | | | |
| and wherein the following method is used for the HPLC analysis: | | | | | |

| HPLC gradient conditions | | | | | |
|---|---|---|---|---|---|
| solvent A (%): | 45.0 H₂O (containing 0.1 % HCO₂H) | | | | |
| solvent B (%): | 45.0 H₂O (containing 0.1 % HCO₂H) | | | | |
| solvent C (%): | CH₃CN (containing 0.1 % HCO₂H) | | | | |
| solvent D (%): | CH₃CN (containing 0.1 % HCO₂H) | | | | |

| Time [min] | A [%] | B [%] | C [%] | D [%] | flow rate [ml/min] |
|---|---|---|---|---|---|
| 0.00 | 45 | 45 | 5 | 5 | 3.50 |
| 2.50 | 0 | 0 | 50 | 50 | 3.50 |
| 2.80 | 0 | 0 | 50 | 50 | 3.50 |
| 3.00 | 45 | 0 | 5 | 5 | 3.50 |
| Column*: | Waters ATLANTIS^{™} | | | | |
| column length: | 20 mm | | | | |
| column internal diameter: | 4.6 mm | | | | |
| temperature: | 40 °C | | | | |

| | | | | | |
|---|---|---|---|---|---|
| The Waters ATLANTIS^{™} column used for chromatography of the compounds is available from Water AG, Dorfstrasse, 5102 Rupperswill, Switzerland (Column serial number: W32021 C09) | | | | | |

| No. | R₂ | R₃ | Retention time (min) | |
|---|---|---|---|---|
| | | | B1a | B1b |
| A8.1 | H | 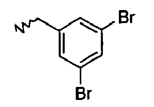 | 1.67 | |
| A8.2 | H | 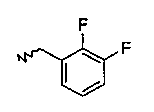 | 1.48 | |
| A8.3 | H | 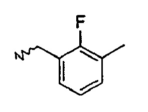 | 1.51 | |
| A8.4 | H | 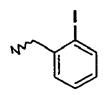 | 1.59 | |
| A8.5 | H | 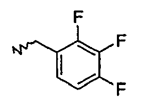 | 1.53 | |
| A8.6 | H | 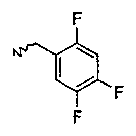 | 1.52 | |
| A8.7 | H | 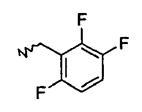 | 1.58 | |
| A8.8 | H | 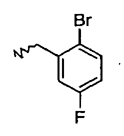 | 1.67 | |
| A8.9 | H | 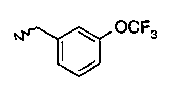 | 1.55 | |
| A8.10 | H | 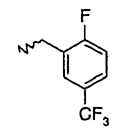 | 1.76 | |
| A8.11 | H | 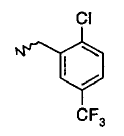 | 1.79 | |
| A8.12 | H | 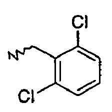 | 1.70 | |
| A8.13 | H | 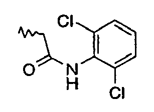 | 2.15 | |
| A8.14 | H | 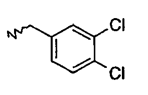 | 1.62 | |
| A8.15 | H | 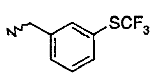 | 1.59 | |
| A8.16 | H | 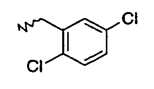 | 1.69 | |
| A8.17 | H | 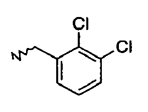 | 1.67 | |
| A8.18 | H | 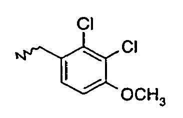 | 1.62 | |
| A8.19 | H | 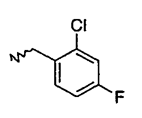 | 1.50 | |
| A8.20 | H | 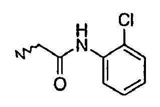 | 1.93 | |
| A8.21 | H | 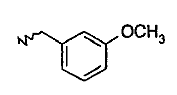 | 1.50 | |
| A8.22 | H | 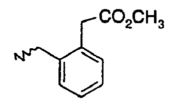 | 1.57 | |
| A8.23 | H | 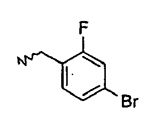 | 1.57 | |
| A8.24 | H | 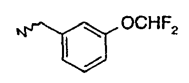 | 1.51 | |
| A8.25 | H | 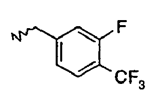 | 1.60 | |
| A8.26 | H | 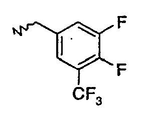 | 1.69 | |
| A8.27 | H | 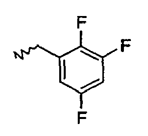 | 1.62 | |
| A8.28 | H | 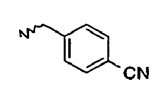 | 1.43 | |
| A8.29 | H | 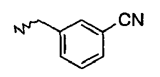 | 1.46 | |
| A8.30 | H | 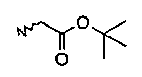 | 1.55 | |
| A8.31 | H | 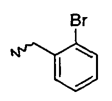 | 1.56 | |
| A8.32 | H | 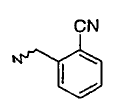 | 1.60 | |
| A8.33 | H | 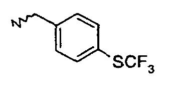 | 1.61 | |
| A8.34 | H | 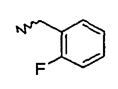 | 1.45 | |
| A8.35 | H | 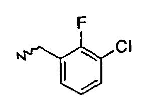 | 1.53 | |
| A8.36 | H | 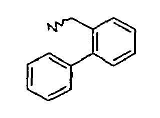 | 1.59 | |
| A8.37 | H | 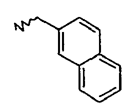 | 1.56 | |
| A8.38 | H | 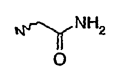 | 1.32 | |
| A8.39 | H | 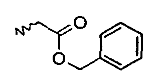 | 1.65 | |
| A8.40 | H | 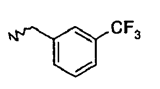 | 1.60 | |
| A8.41 | H | 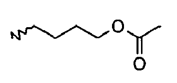 | 1.35 | |
| A8.42 | H | 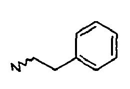 | 1.54 | |
| A8.43 | H | 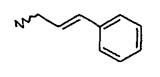 | 1.51 | |
| A8.44 | H | 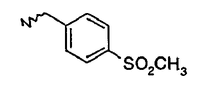 | 1.40 | |
| A8.45 | H | 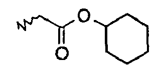 | 1.70 | |
| A8.46 | 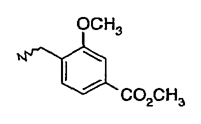 | 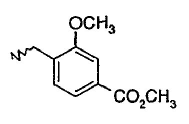 | 1.77 | |
| A8.47 | 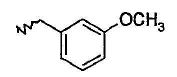 | 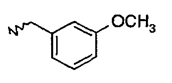 | 2.59 | |
| A8.48 | 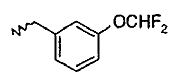 | 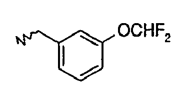 | 2.65 | |
| A8.49 | 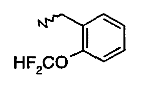 | 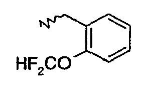 | 2.16 | |
| A8.50 | 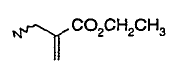 | 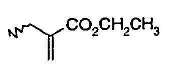 | 1.92 | |
| A8.51 | 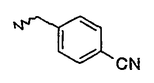 | 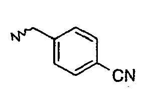 | 2.57 | |
| A8.52 | 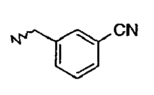 | 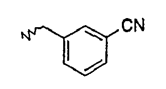 | 2.57 | |
| A8.53 | 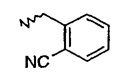 | 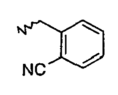 | 2.55 | |
| A8.54 | 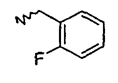 | 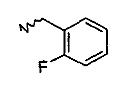 | 2.71 | |
| A8.55 | 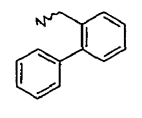 | 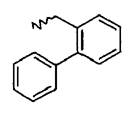 | 2.75 | |
| A8.56 | 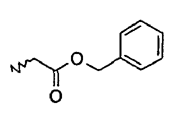 | 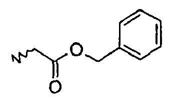 | 2.60 | |
| A8.57 | 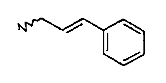 | 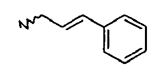 | 1.97 | |
| A8.58 | 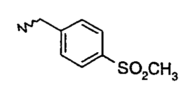 | 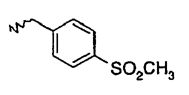 | 2.24 | |
| A8.59 | 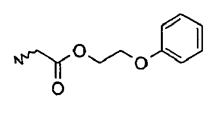 | 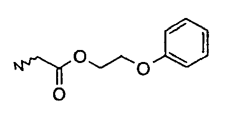 | 2.53 | |

**Table A9: Compounds of the formula in which R₁ is sec-butyl or isopropyl**

| | | | | | |
|---|---|---|---|---|---|
| 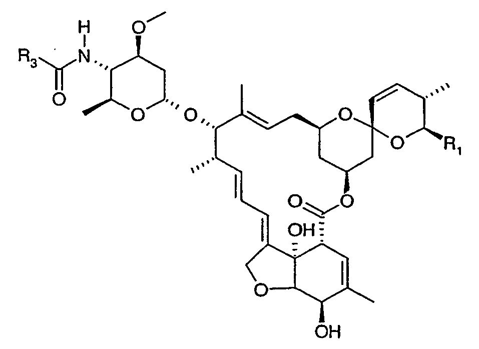 | | | | | |
| and wherein the following method is used for the HPLC analysis: | | | | | |

| HPLC gradient conditions | | | | | |
|---|---|---|---|---|---|
| solvent A (%): | 30.0 H₂O (containing 0.1 % HCO₂H + 10% CH₃CN) | | | | |
| solvent B (%): | 10.0 CH₃CN (containing 0.1% HCO₂H) | | | | |
| solvent C (%): | 10.0 H₂O (containing 0.1% HCO₂H) | | | | |
| solvent D (%): | 30.0 CH₃CN (containing 0.1% HCO₂H) | | | | |

| Time [min] | A [%] | B [%] | C [%] | D [%] | flow rate [ml/min] |
|---|---|---|---|---|---|
| 0.00 | 30 | 10 | 30 | 30 | 1.50 |
| 3.50 | 0 | 100 | 0 | 0 | 1.50 |
| 3.80 | 0 | 100 | 0 | 0 | 1.50 |
| 4.00 | 30 | 10 | 30 | 30 | 1.50 |
| column: | YMC ODS-AQ | | | | |
| column length: | 33 mm | | | | |
| column internal diameter: | 3.0 mm | | | | |
| temperature: | 40 °C | | | | |

| | | | | | |
|---|---|---|---|---|---|
| The YMC ODS-AQ column used for chromatography of the compounds is available from Stragroma AG, Chr. Merian-Ring31a, CH, Reinach (Catalogue number: AQ12S030303QT) | | | | | |

| No. | R₃ | Retention time (min) | |
|---|---|---|---|
| | | B1a | B1b |
| A9.1 | 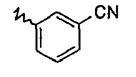 | 2.12 | |
| A9.2 | 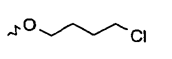 | 2.43 | |
| A9.3 | 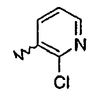 | 1.82 | |
| A9.4 | 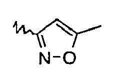 | 2.00 | |
| A9.5 | 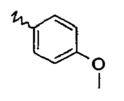 | 2.00 | |
| A9.6 | 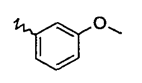 | 2.06 | |
| A9.7 | 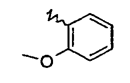 | 2.10 | |
| A9.8 | 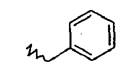 | 1.94 | |
| A9.9 | 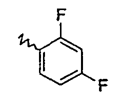 | 2.17 | |
| A9.10 | 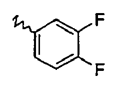 | 2.28 | |
| A9.11 | 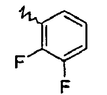 | 2.19 | |
| A9.12 | 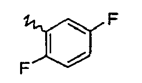 | 2.20 | |
| A9.13 | 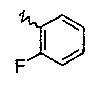 | 2.10 | |
| A9.14 | 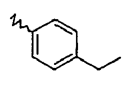 | 2.30 | |
| A9.15 | 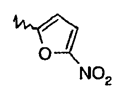 | 2.10 | |
| A9.16 | 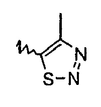 | 2.10 | |
| A9.17 | 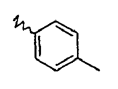 | 2.12 | |
| A9.18 | 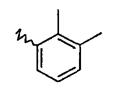 | 2.21 | |
| A9.19 | 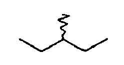 | 2.03 | |
| A9.20 | 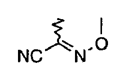 | 2.20 | |
| A9.21 | 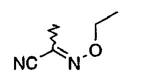 | 2.30 | |
| A9.22 | 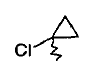 | 2.18 | |
| A9.23 | 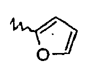 | 1.79 | |
| A9.24 | 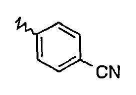 | 2.05 | |
| A9.25 | 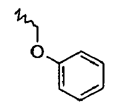 | 2.10 | |
| A9.26 | 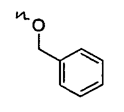 | 2.50 | |
| A9.27 | 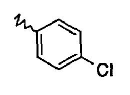 | 2.29 | |
| A9.28 | 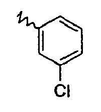 | 2.33 | |
| A9.29 | 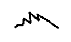 | 1.50 | |
| A9.30 | 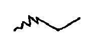 | 1.70 | |
| A9.31 | 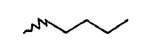 | 2.12 | |
| A9.32 | 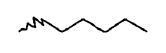 | 2.33 | |
| A9.33 | 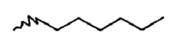 | 2.55 | |
| A9.34 | 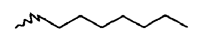 | 2.80 | |
| A9.35 | 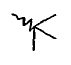 | 1.90 | |
| A9.36 | 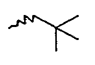 | 2.10 | |
| A9.37 | 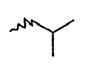 | 1.88 | |
| A9.38 | 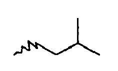 | 2.08 | |
| A9.39 | 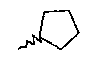 | 1.98 | |
| A9.40 | 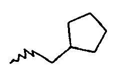 | 2.35 | |
| A9.41 | 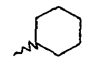 | 2.12 | |
| A9.42 | 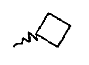 | 1.80 | |
| A9.43 | 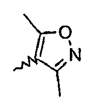 | 1.89 | |
| A9.44 | 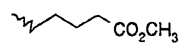 | 1.67 | |
| A9.45 | 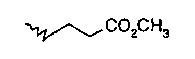 | 1.60 | |
| A9.46 | 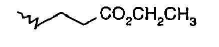 | 1.80 | |
| A9.47 | 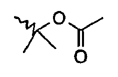 | 1.77 | |
| A9.48 | 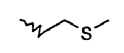 | 1.73 | |
| A9.49 | 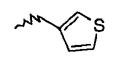 | 1.92 | |
| A9.50 | 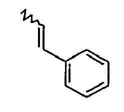 | 2.13 | |
| A9.51 | 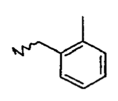 | 2.11 | |
| A9.52 | 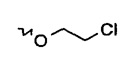 | 2.14 | |
| A9.53 | 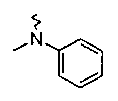 | 1.92 | |
| A9.54 | 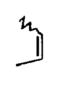 | 1.67 | |
| A9.55 | 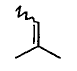 | 2.01 | |
| A9.56 | 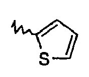 | 2.01 | |
| A9.57 | 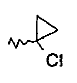 | 2.18 | |
| A9.58 | 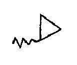 | 1.70 | |

Table 1: Compounds of the formula (I) in which the carbon atom 4' has the configuration *(R)*, A is -C(=O)-, X-Y is -CH=CH-, R₁ is sec-butyl or isopropyl and the combination of R₂ and R₃ for each compound corresponds to a line B.1 to B.293 of Table B below.
Table 2: Compounds of the formula (I) in which the carbon atom 4' has the configuration *(R)*, A is -C(=S)-, X-Y is -CH=CH-, R₁ is sec-butyl or isopropyl and the combination of R₂ and R₃ for each compound corresponds to a line B.1 to B.293 of Table B below.
Table 3: Compounds of the formula (I) in which the carbon atom 4' has the configuration *(R)*, A is -O-C(=O)-, X-Y is -CH=CH-, R₁ is sec-butyl or isopropyl and the combination of R₂ and R₃ for each compound corresponds to a lineB.1 to B.293 of Table B below.
Table 4: Compounds of the formula (I) in which the carbon atom 4' has the configuration *(R)*, A is -O-C(=S)-, X-Y is -CH=CH-, R₁ is sec-butyl or isopropyl and the combination of R₂ and R₃ for each compound corresponds to a line B.1 to B.293 of Table B below.
Table 5: Compounds of the formula (I) in which the carbon atom 4' has the configuration *(R)*, A is -S-C(=O)-, X-Y is -CH=CH-, R₁ is sec-butyl or isopropyl and the combination of R₂ and R₃ for each compound corresponds to a line B.1 to B.293 of Table B below.
Table 6: Compounds of the formula (I) in which the carbon atom 4' has the configuration *(R)*, A is -S-C(=S)-, X-Y is -CH=CH-, R₁ is sec-butyl or isopropyl and the combination of R₂ and R₃ for each compound corresponds to a line B.1 to B.293 of Table B below.
Table 7: Compounds of the formula (I) in which the carbon atom 4' has the configuration *(R)*, A is -NR₄-C(=O)-, X-Y is -CH=CH-, R₁ is sec-butyl or isopropyl and the combination of R₂ and R₃ for each compound corresponds to a line B.1 to B.293 of Table B below.
Table 8: Compounds of the formula (I) in which the carbon atom 4' has the configuration *(R)*, A is -NR₄-C(=S)-, X-Y is -CH=CH-, R₁ is sec-butyl or isopropyl and the combination of R₂ and R₃ for each compound corresponds to a line B.1 to B.293 of Table B below.
Table 9: Compounds of the formula (I) in which the carbon atom 4' has the configuration *(R)*, A is -SO₂-, X-Y is -CH=CH-, R₁ is sec-butyl or isopropyl and the combination of R₂ and R₃ for each compound corresponds to a line B.1 to B.293 of Table B below.
Table 10: Compounds of the formula (I) in which the carbon atom 4' has the configuration *(R)*, A is -O-SO₂-, X-Y is -CH=CH-, R₁ is sec-butyl or isopropyl and the combination of R₂ and R₃ for each compound corresponds to a line B.1 to B.293 of Table B below.
Table 11: Compounds of the formula (I) in which the carbon atom 4' has the configuration *(R)*, A is -NR₄-SO₂-, X-Y is -CH=CH-, R₁ is sec-butyl or isopropyl and the combination of R₂ and R₃ for each compound corresponds to a line B.1 to B.293 of Table B below.
Table 12: Compounds of the formula (I) in which the carbon atom 4' has the configuration *(R),* A is a bond, X-Y is -CH=CH-, R₁ is sec-butyl or isopropyl and the combination of R₂ and R₃ for each compound corresponds to a line B.1 to B.293 of Table B below.
Table 13: Compounds of the formula (I) in which the carbon atom 4' has the configuration *(S)*, A is -C(=O)-, X-Y is -CH=CH-, R₁ is sec-butyl or isopropyl and the combination of R₂ and R₃ for each compound corresponds to a line B.1 to B.293 of Table B below.
Table 14: Compounds of the formula (I) in which the carbon atom 4' has the configuration *(S),* A is -C(=S)-, X-Y is -CH=CH-, R₁ is sec-butyl or isopropyl and the combination of R₂ and R₃ for each compound corresponds to a line B.1 to B.293 of Table B below.
Table 15: Compounds of the formula (I) in which the carbon atom 4' has the configuration *(S)*, A is -O-C(=O)-, X-Y is -CH=CH-, R₁ is sec-butyl or isopropyl and the combination of R₂ and R₃ for each compound corresponds to a line B.1 to B.293 of Table B below.
Table 16: Compounds of the formula (I) in which the carbon atom 4' has the configuration *(S)*, A is -O-C(=S)-, X-Y is -CH=CH-, R₁ is sec-butyl or isopropyl and the combination of R₂ and R₃ for each compound corresponds to a line B.1 to B.293 of Table B below.
Table 17: Compounds of the formula (I) in which the carbon atom 4' has the configuration *(S),* A is -S-C(=O)-, X-Y is -CH=CH-, R₁ is sec-butyl or isopropyl and the combination of R₂ and R₃ for each compound corresponds to a line B.1 to B.293 of Table B below.
Table 18: Compounds of the formula (I) in which the carbon atom 4' has the configuration *(S)*, A is -S-C(=S)-, X-Y is -CH=CH-, R₁ is sec-butyl or isopropyl and the combination of R₂ and R₃ for each compound corresponds to a line B.1 to B.293 of Table B below.
Table 19: Compounds of the formula (I) in which the carbon atom 4' has the configuration *(S)*, A is -NR₄-C(=O)-, X-Y is -CH=CH-, R₁ is sec-butyl or isopropyl and the combination of R₂ and R₃ for each compound corresponds to a line B.1 to B.293 of Table B below.
Table 20: Compounds of the formula (I) in which the carbon atom 4' has the configuration *(S),* A is -NR₄-C(=S)-, X-Y is -CH=CH-, R₁ is sec-butyl or isopropyl and the combination of R₂ and R₃ for each compound corresponds to a line B.1 to B.293 of Table B below.
Table 21: Compounds of the formula (I) in which the carbon atom 4' has the configuration *(S)*, A is -SO₂-, X-Y is -CH=CH-, R₁ is sec-butyl or isopropyl and the combination of R₂ and R₃ for each compound corresponds to a line B.1 to B.293 of Table B below.
Table 22: Compounds of the formula (I) in which the carbon atom 4' has the configuration *(S)*, A is -O-SO₂-, X-Y is -CH=CH-, R₁ is sec-butyl or isopropyl and the combination of R₂ and R₃ for each compound corresponds to a line B.1 to B.293 of Table B below.
Table 23: Compounds of the formula (I) in which the carbon atom 4' has the configuration *(S),* A is -NR₄-SO₂-, X-Y is -CH=CH-, R₁ is sec-butyl or isopropyl and the combination of R₂ and R₃ for each compound corresponds to a line B.1 to B.293 of Table B below.
Table 24: Compounds of the formula (I) in which the carbon atom 4' has the configuration *(S),* A is a bond, X-Y is -CH=CH-, R₁ is sec-butyl or isopropyl and the combination of R₂ and R₃ for each compound corresponds to a line B.1 to B.293 of Table B below.
Table 25: Compounds of the formula (I) in which the carbon atom 4' has the configuration *(R),* A is -C(=O)-, X-Y is -CH₂-CH₂-, R₁ is sec-butyl or isopropyl and the combination of R₂ and R₃ for each compound corresponds to a line B.1 and B.3 to B.293 of Table B below.
Table 26: Compounds of the formula (I) in which the carbon atom 4' has the configuration *(R)*, A is -C(=S)-, X-Y is -CH₂-CH₂-, R₁ is sec-butyl or isopropyl and the combination of R₂ and R₃ for each compound corresponds to a line B.1 to B.293 of Table B below.
Table 27: Compounds of the formula (I) in which the carbon atom 4' has the configuration *(R)*, A is -O-C(=O)-, X-Y is -CH₂-CH₂-, R₁ is sec-butyl or isopropyl and the combination of R₂ and R₃ for each compound corresponds to a line B.1 to B.293 of Table B below.
Table 28: Compounds of the formula (I) in which the carbon atom 4' has the configuration *(R)*, A is -O-C(=S)-, X-Y is -CH₂-CH₂-, R₁ is sec-butyl or isopropyl and the combination of R₂ and R₃ for each compound corresponds to a line B.1 to B.293 of Table B below.
Table 29: Compounds of the formula (I) in which the carbon atom 4' has the configuration *(R)*, A is -S-C(=O)-, X-Y is -CH₂-CH₂-, R₁ is sec-butyl or isopropyl and the combination of R₂ and R₃ for each compound corresponds to a line B.1 to B.293 of Table B below.
Table 30: Compounds of the formula (I) in which the carbon atom 4' has the configuration *(R)*, A is -S-C(=S)-, X-Y is -CH₂-CH₂-, R₁ is sec-butyl or isopropyl and the combination of R₂ and R₃ for each compound corresponds to a line B.1 to B.293 of Table B below.
Table 31: Compounds of the formula (I) in which the carbon atom 4' has the configuration *(R)*, A is -NR₄-C(=O)-, X-Y is -CH₂-CH₂-, R₁ is sec-butyl or isopropyl and the combination of R₂ and R₃ for each compound corresponds to a line B.1 to B.293 of Table B below.
Table 32: Compounds of the formula (I) wherein the carbon atom 4' has the configuration *(R)*, A is -NR₄-C(=S)-, X-Y is -CH₂-CH₂-, R₁ is sec-butyl or isopropyl and the combination of R₂ and R₃ for each compound corresponds to a line B.1 to B.293 of Table B below.
Table 33: Compounds of the formula (I) in which the carbon atom 4' has the configuration *(R)*, A is -SO₂-, X-Y is -CH₂-CH₂-, R₁ is sec-butyl or isopropyl and the combination of R₂ and R₃ for each compound corresponds to a line B.1 to B.293 of Table B below.
Table 34: Compounds of the formula (I) in which the carbon atom 4' has the configuration *(R),* A is -O-SO₂-, X-Y is -CH₂-CH₂-, R₁ is sec-butyl or isopropyl and the combination of R₂ and R₃ for each compound corresponds to a line B.1 to B.293 of Table B below.
Table 35: Compounds of the formula (I) in which the carbon atom 4' has the configuration *(R),* A is -NR₄-SO₂-, X-Y is -CH₂-CH₂-, R₁ is sec-butyl or isopropyl and the combination of R₂ and R₃ for each compound corresponds to a line B.1 to B.293 of Table B below.
Table 36: Compounds of the formula (I) in which the carbon atom 4' has the configuration *(R)*, A is a bond, X-Y is -CH₂-CH₂-, R₁ is sec-butyl or isopropyl and the combination of R₂ and R₃ for each compound corresponds to a line B.2 to B.293 of Table B below.
Table 37: Compounds of the formula (I) in which the carbon atom 4' has the configuration *(S)*, A is -C(=O)-, X-Y is -CH₂-CH₂-, R₁ is sec-butyl or isopropyl and the combination of R₂ and R₃ for each compound corresponds to a line B.1 to B.293 of Table B below.
Table 38: Compounds of the formula (I) in which the carbon atom 4' has the configuration *(S)*, A is -C(=S)-, X-Y is -CH₂-CH₂-, R₁ is sec-butyl or isopropyl and the combination of R₂ and R₃ for each compound corresponds to a line B.1 to B.293 of Table B below.
Table 39: Compounds of the formula (I) in which the carbon atom 4' has the configuration *(S)*, A is -O-C(=O)-, X-Y is -CH₂-CH₂-, R₁ is sec-butyl or isopropyl and the combination of R₂ and R₃ for each compound corresponds to a line B.1 to B.293 of Table B below.
Table 40: Compounds of the formula (I) wherein the carbon atom 4' has the configuration *(S)*, A is -O-C(=S)-, X-Y is -CH₂-CH₂-, R₁ is sec-butyl or isopropyl and the combination of R₂ and R₃ for each compound corresponds to a line B.1 to B.293 of Table B below.
Table 41: Compounds of the formula (I) wherein the carbon atom 4' has the configuration *(S)*, A is -S-C(=O)-, X-Y is -CH₂-CH₂-, R₁ is sec-butyl or isopropyl and the combination of R₂ and R₃ for each compound corresponds to a line B.1 to B.293 of Table B below.
Table 42: Compounds of the formula (I) in wherein the carbon atom 4' has the configuration *(S)*, A is -S-C(=S)-, X-Y is -CH₂-CH₂-, R₁ is sec-butyl or isopropyl and the combination of R₂ and R₃ for each compound corresponds to a line B.1 to B.293 of Table B below.
Table 43: Compounds of the formula (I) in which the carbon atom 4' has the configuration *(S)*, A is -NR₄-C(=O)-, X-Y is -CH₂-CH₂-, R₁ is sec-butyl or isopropyl and the combination of R₂ and R₃ for each compound corresponds to a line B.1 to B.293 of Table B below.
Table 44: Compounds of the formula (I) in which the carbon atom 4' has the configuration *(S)*, A is -NR₄-C(=S)-, X-Y is -CH₂-CH₂-, R₁ is sec-butyl or isopropyl and the combination of R₂ and R₃ for each compound corresponds to a line B.1 to B.293 of Table B below.
Table 45: Compounds of the formula (I) in which the carbon atom 4' has the configuration *(S),* A is -SO₂-, X-Y is -CH₂-CH₂-, R₁ is sec-butyl or isopropyl and the combination of R₂ and R₃ for each compound corresponds to a line B.1 to B.293 of Table B below.
Table 46: Compounds of the formula (I) in which the carbon atom 4' has the configuration *(S)*, A is -O-SO₂-, X-Y is -CH₂-CH₂-, R₁ is sec-butyl or isopropyl and the combination of R₂ and R₃ for each compound corresponds to a line B.1 to B.293 of Table B below.
Table 47: Compounds of the formula (I) in which the carbon atom 4' has the configuration *(S)*, A is -NR₄-SO₂-, X-Y is -CH₂-CH₂-, R₁ is sec-butyl or isopropyl and the combination of R₂ and R₃ for each compound corresponds to a line B.1 to B.293 of Table B below.
Table 48: Compounds of the formula (I) in which the carbon atom 4' has the configuration *(S)*, A is a bond, X-Y is -CH₂-CH₂-, R₁ is sec-butyl or isopropyl and the combination of R₂ and R₃ for each compound corresponds to a line B.1 to B.293 of Table B below.
Table 49: Compounds of the formula (I) in which the carbon atom 4' has the configuration *(R)*, A is -C(=O)-, X-Y is -CH=CH-, R₁ is cyclohexyl and the combination of R₂ and R₃ for each compound corresponds to a line B.1 to B.293 of Table B below.
Table 50: Compounds of the formula (I) in which the carbon atom 4' has the configuration *(R)*, A is -C(=S)-, X-Y is -CH=CH-, R₁ is cyclohexyl and the combination of R₂ and R₃ for each compound corresponds to a line B.1 to B.293 of Table B below.
Table 51: Compounds of the formula (I) in which the carbon atom 4' has the configuration *(R)*, A is -O-C(=O)-, X-Y is -CH=CH-, R₁ is cyclohexyl and the combination of R₂ and R₃ for each compound corresponds to a line B.1 to B.293 of Table B below.
Table 52: Compounds of the formula (I) in which the carbon atom 4' has the configuration *(R)*, A is -O-C(=S)-, X-Y is -CH=CH-, R₁ is cyclohexyl and the combination of R₂ and R₃ for each compound corresponds to a line B.1 to B.293 of Table B below.
Table 53: Compounds of the formula (I) in which the carbon atom 4' has the configuration *(R),* A is -S-C(=O)-, X-Y is -CH=CH-, R₁ is cyclohexyl and the combination of R₂ and R₃ for each compound corresponds to a line B.1 to B.293 of Table B below.
Table 54: Compounds of the formula (I) in which the carbon atom 4' has the configuration *(R)*, A is -S-C(=S)-, X-Y is -CH=CH-, R₁ is cyclohexyl and the combination of R₂ and R₃ for each compound corresponds to a line B.1 to B.293 of Table B below.
Table 55: Compounds of the formula (I) in which the carbon atom 4' has the configuration *(R)*, A is -NR₄-C(=O)-, X-Y is -CH=CH-, R₁ is cyclohexyl and the combination of R₂ and R₃ for each compound corresponds to a line B.1 to B.293 of Table B below.
Table 56: Compounds of the formula (I) in which the carbon atom 4' has the configuration *(R),* A is -NR₄-C(=S)-, X-Y is -CH=CH-, R₁ is cyclohexyl and the combination of R₂ and R₃ for each compound corresponds to a line B.1 to B.293 of Table B below.
Table 57: Compounds of the formula (I) in which the carbon atom 4' has the configuration *(R)*, A is -SO₂-, X-Y is -CH=CH-, R₁ is cyclohexyl and the combination of R₂ and R₃ for each compound corresponds to a line B.1 to B.293 of Table B below.
Table 58: Compounds of the formula (I) in which the carbon atom 4' has the configuration *(R)*, A is -O-SO₂-, X-Y is -CH=CH-, R₁ is cyclohexyl and the combination of R₂ and R₃ for each compound corresponds to a line B.1 to B.293 of Table B below.
Table 59: Compounds of the formula (I) in which the carbon atom 4' has the configuration *(R)*, A is -NR₄-SO₂-, X-Y is -CH=CH-, R₁ is cyclohexyl and the combination of R₂ and R₃ for each compound corresponds to a line B.1 to B.293 of Table B below.
Table 60: Compounds of the formula (I) in which the carbon atom 4' has the configuration *(R)*, A is a bond, X-Y is -CH=CH-, R₁ is cyclohexyl and the combination of R₂ and R₃ for each compound corresponds to a line B.1 to B.293 of Table B below.
Table 61: Compounds of the formula (I) in which the carbon atom 4' has the configuration *(S)*, A is -C(=O)-, X-Y is -CH=CH-, R₁ is cyclohexyl and the combination of R₂ and R₃ for each compound corresponds to a line B.1 to B.293 of Table B below.
Table 62: Compounds of the formula (I) in which the carbon atom 4' has the configuration *(S)*, A is -C(=S)-, X-Y is -CH=CH-, R₁ is cyclohexyl and the combination of R₂ and R₃ for each compound corresponds to a line B.1 to B.293 of Table B below.
Table 63: Compounds of the formula (I) in which the carbon atom 4' has the configuration *(S)*, A is -O-C(=O)-, X-Y is -CH=CH-, R₁ is cyclohexyl and the combination of R₂ and R₃ for each compound corresponds to a line B.1 to B.293 of Table B below.
Table 64: Compounds of the formula (I) in which the carbon atom 4' has the configuration *(S),* A is -O-C(=S)-, X-Y is -CH=CH-, R₁ is cyclohexyl and the combination of R₂ and R₃ for each compound corresponds to a line B.1 to B.293 of Table B below.
Table 65: Compounds of the formula (I) in which the carbon atom 4' has the configuration *(S)*, A is -S-C(=O)-, X-Y is -CH=CH-, R₁ is cyclohexyl and the combination of R₂ and R₃ for each compound corresponds to a line B.1 to B.293 of Table B below.
Table 66: Compounds of the formula (I) in which the carbon atom 4' has the configuration *(S),* A is -S-C(=S)-, X-Y is -CH=CH-, R₁ is cyclohexyl and the combination of R₂ and R₃ for each compound corresponds to a line B.1 to B.293 of Table B below.
Table 67: Compounds of the formula (I) in which the carbon atom 4' has the configuration *(S),* A is -NR₄-C(=O)-, X-Y is -CH=CH-, R₁ is cyclohexyl and the combination of R₂ and R₃ for each compound corresponds to a line B.1 to B.293 of Table B below.
Table 68: Compounds of the formula (I) in which the carbon atom 4' has the configuration *(S)*, A is -NR₄-C(=S)-, X-Y is -CH=CH-, R₁ is cyclohexyl and the combination of R₂ and R₃ for each compound corresponds to a line B.1 to B.293 of Table B below.
Table 69: Compounds of the formula (I) in which the carbon atom 4' has the configuration *(S),* A is -SO₂-, X-Y is -CH=CH-, R₁ is cyclohexyl and the combination of R₂ and R₃ for each compound corresponds to a line B.1 to B.293 of Table B below.
Table 70: Compounds of the formula (I) in which the carbon atom 4' has the configuration *(S),* A is -O-SO₂-, X-Y is -CH=CH-, R₁ is cyclohexyl and the combination of R₂ and R₃ for each compound corresponds to a line B.1 to B.293 of Table B below.
Table 71: Compounds of the formula (I) in which the carbon atom 4' has the configuration *(S)*, A is -NR₄-SO₂-, X-Y is -CH=CH-, R₁ is cyclohexyl and the combination of R₂ and R₃ for each compound corresponds to a line B.1 to B.293 of Table B below.
Table 72: Compounds of the formula (I) in which the carbon atom 4' has the configuration *(S),* A is a bond, X-Y is -CH=CH-, R₁ is cyclohexyl and the combination of R₂ and R₃ for each compound corresponds to a line B.1 to B.293 of Table B below.
Table 73: Compounds of the formula (I) in which the carbon atom 4' has the configuration *(R)*, A is -C(=O)-, X-Y is -CH₂-CH₂-, R₁ is cyclohexyl and the combination of R₂ and R₃ for each compound corresponds to a line B.1 to B.293 of Table B below.
Table 74: Compounds of the formula (I) in which the carbon atom 4' has the configuration *(R)*, A is -C(=S)-, X-Y is -CH₂-CH₂-, R₁ is cyclohexyl and the combination of R₂ and R₃ for each compound corresponds to a line B.1 to B.293 of Table B below.
Table 75: Compounds of the formula (I) in which the carbon atom 4' has the configuration *(R)*, A is -O-C(=O)-, X-Y is -CH₂-CH₂-, R₁ is cyclohexyl and the combination of R₂ and R₃ for each compound corresponds to a line B.1 to B.293 of Table B below.
Table 76: Compounds of the formula (I) in which the carbon atom 4' has the configuration *(R)*, A is -O-C(=S)-, X-Y is -CH₂-CH₂-, R₁ is cyclohexyl and the combination of R₂ and R₃ for each compound corresponds to a line B.1 to B.293 of Table B below.
Table 77: Compounds of the formula (I) in which the carbon atom 4' has the configuration *(R)*, A is -S-C(=O)-, X-Y is -CH₂-CH₂-, R₁ is cyclohexyl and the combination of R₂ and R₃ for each compound corresponds to a line B.1 to B.293 of Table B below.
Table 78: Compounds of the formula (I) in which the carbon atom 4' has the configuration *(R)*, A is -S-C(=S)-, X-Y is -CH₂-CH₂-, R₁ is cyclohexyl and the combination of R₂ and R₃ for each compound corresponds to a line B.1 to B.293 of Table B below.
Table 79: Compounds of the formula (I) in which the carbon atom 4' has the configuration *(R)*, A is -NR₄-C(=O)-, X-Y is -CH₂-CH₂-, R₁ is cyclohexyl and the combination of R₂ and R₃ for each compound corresponds to a line B.1 to B.293 of Table B below.
Table 80: Compounds of the formula (I) in which the carbon atom 4' has the configuration *(R)*, A is -NR₄-C(=S)-, X-Y is -CH₂-CH₂-, R₁ is cyclohexyl and the combination of R₂ and R₃ for each compound corresponds to a line B.1 to B.293 of Table B below.
Table 81: Compounds of the formula (I) in which the carbon atom 4' has the configuration *(R)*, A is -SO₂-, X-Y is -CH₂-CH₂-, R₁ is cyclohexyl and the combination of R₂ and R₃ for each compound corresponds to a line B.1 to B.293 of Table B below.
Table 82: Compounds of the formula (I) in which the carbon atom 4' has the configuration *(R)*, A is -O-SO₂-, X-Y is -CH₂-CH₂-, R₁ is cyclohexyl and the combination of R₂ and R₃ for each compound corresponds to a line B.1 to B.293 of Table B below.
Table 83: Compounds of the formula (I) in which the carbon atom 4' has the configuration *(R)*, A is -NR₄-SO₂-, X-Y is -CH₂-CH₂-, R₁ is cyclohexyl and the combination of R₂ and R₃ for each compound corresponds to a line B.1 to B.293 of Table B below.
Table 84: Compounds of the formula (I) in which the carbon atom 4' has the configuration *(R)*, A is a bond, X-Y is -CH₂-CH₂-, R₁ is cyclohexyl and the combination of R₂ and R₃ for each compound corresponds to a line B.1 to B.293 of Table B below.
Table 85: Compounds of the formula (I) in which the carbon atom 4' has the configuration *(S)*, A is -C(=O)-, X-Y is -CH₂-CH₂-, R₁ is cyclohexyl and the combination of R₂ and R₃ for each compound corresponds to a line B.1 to B.293 of Table B below.
Table 86: Compounds of the formula (I) in which the carbon atom 4' has the configuration *(S),* A is -C(=S)-, X-Y is -CH₂-CH₂-, R₁ is cyclohexyl and the combination of R₂ and R₃ for each compound corresponds to a line B.1 to B.293 of Table B below.
Table 87: Compounds of the formula (I) in which the carbon atom 4' has the configuration *(S)*, A is -O-C(=O)-, X-Y is -CH₂-CH₂-, R₁ is cyclohexyl and the combination of R₂ and R₃ for each compound corresponds to a line B.1 to B.293 of Table B below.
Table 88: Compounds of the formula (I) in which the carbon atom 4' has the configuration *(S)*, A is -O-C(=S)-, X-Y is -CH₂-CH₂-, R₁ is cyclohexyl and the combination of R₂ and R₃ for each compound corresponds to a line B.1 to B.293 of Table B below.
Table 89: Compounds of the formula (I) in which the carbon atom 4' has the configuration *(S),* A is -S-C(=O)-, X-Y is -CH₂-CH₂-, R₁ is cyclohexyl and the combination of R₂ and R₃ for each compound corresponds to a line B.1 to B.293 of Table B below.
Table 90: Compounds of the formula (I) in which the carbon atom 4' has the configuration *(S)*, A is -S-C(=S)-, X-Y is -CH₂-CH₂-, R₁ is cyclohexyl and the combination of R₂ and R₃ for each compound corresponds to a line B.1 to B.293 of Table B below.
Table 91: Compounds of the formula (I) in which the carbon atom 4' has the configuration *(S)*, A is -NR₄-C(=O)-, X-Y is -CH₂-CH₂-, R₁ is cyclohexyl and the combination of R₂ and R₃ for each compound corresponds to a line B.1 to B.293 of Table B below.
Table 92: Compounds of the formula (I) in which the carbon atom 4' has the configuration *(S)*, A is -NR₄-C(=S)-, X-Y is -CH₂-CH₂-, R₁ is cyclohexyl and the combination of R₂ and R₃ for each compound corresponds to a line B.1 to B.293 of Table B below.
Table 93: Compounds of the formula (I) in which the carbon atom 4' has the configuration *(S)*, A is -SO₂-, X-Y is -CH₂-CH₂-, R₁ is cyclohexyl and the combination of R₂ and R₃ for each compound corresponds to a line B.1 to B.293 of Table B below.
Table 94: Compounds of the formula (I) in which the carbon atom 4' has the configuration *(S),* A is -O-SO₂-, X-Y is -CH₂-CH₂-, R₁ is cyclohexyl and the combination of R₂ and R₃ for each compound corresponds to a line B.1 to B.293 of Table B below.
Table 95: Compounds of the formula (I) in which the carbon atom 4' has the configuration *(S)*, A is -NR₄-SO₂-, X-Y is -CH₂-CH₂-, R₁ is cyclohexyl and the combination of R₂ and R₃ for each compound corresponds to a line B.1 to B.293 of Table B below.
Table 96: Compounds of the formula (I) in which the carbon atom 4' has the configuration *(S),* A is a bond, X-Y is -CH₂-CH₂-, R₁ is cyclohexyl and the combination of R₂ and R₃ for each compound corresponds to a line B.1 to B.293 of Table B below.
Table 97: Compounds of the formula (I) in which the carbon atom 4' has the configuration *(R)*, A is -C(=O)-, X-Y is -CH=CH-, R₁ is 1-methyl-butyl and the combination of R₂ and R₃ for each compound corresponds to a line B.1 to B.293 of Table B below.
Table 98: Compounds of the formula (I) in which the carbon atom 4' has the configuration *(R)*, A is -C(=S)-, X-Y is -CH=CH-, R₁ is 1-methyl-butyl and the combination of R₂ and R₃ for each compound corresponds to a line B.1 to B.293 of Table B below.
Table 99: Compounds of the formula (I) in which the carbon atom 4' has the configuration *(R)*, A is -O-C(=O)-, X-Y is -CH=CH-, R₁ is 1-methyl-butyl and the combination of R₂ and R₃ for each compound corresponds to a line B.1 to B.293 of Table B below.
Table 100: Compounds of the formula (I) in which the carbon atom 4' has the configuration *(R),* A is -O-C(=S)-, X-Y is -CH=CH-, R₁ is 1-methyl-butyl and the combination of R₂ and R₃ for each compound corresponds to a line B.1 to B.293 of Table B below.
Table 101: Compounds of the formula (I) in which the carbon atom 4' has the configuration *(R),* A is -S-C(=O)-, X-Y is -CH=CH-, R₁ is 1-methyl-butyl and the combination of R₂ and R₃ for each compound corresponds to a line B.1 to B.293 of Table B below.
Table 102: Compounds of the formula (I) in which the carbon atom 4' has the configuration *(R),* A is -S-C(=S)-, X-Y is -CH=CH-, R₁ is 1-methyl-butyl and the combination of R₂ and R₃ for each compound corresponds to a line B.1 to B.293 of Table B below.
Table 103: Compounds of the formula (I) in which the carbon atom 4' has the configuration *(R)*, A is -NR₄-C(=O)-, X-Y is -CH=CH-, R₁ is 1-methyl-butyl and the combination of R₂ and R₃ for each compound corresponds to a line B.1 to B.293 of Table B below.
Table 104: Compounds of the formula (I) in which the carbon atom 4' has the configuration *(R)*, A is -NR₄-C(=S)-, X-Y is -CH=CH-, R₁ is 1-methyl-butyl and the combination of R₂ and R₃ for each compound corresponds to a line B.1 to B.293 of Table B below.
Table 105: Compounds of the formula (I) in which the carbon atom 4' has the configuration *(R)*, A is -SO₂-, X-Y is -CH=CH-, R₁ is 1-methyl-butyl and the combination of R₂ and R₃ for each compound corresponds to a line B.1 to B.293 of Table B below.
Table 106: Compounds of the formula (I) in which the carbon atom 4' has the configuration *(R)*, A is -O-SO₂-, X-Y is -CH=CH-, R₁ is 1-methyl-butyl and the combination of R₂ and R₃ for each compound corresponds to a line B.1 to B.293 of Table B below.
Table 107: Compounds of the formula (I) in which the carbon atom 4' has the configuration *(R),* A is -NR₄-SO₂-, X-Y is -CH=CH-, R₁ is 1-methyl-butyl and the combination of R₂ and R₃ for each compound corresponds to a line B.1 to B.293 of Table B below.
Table 108: Compounds of the formula (I) in which the carbon atom 4' has the configuration *(R)*, A is a bond, X-Y is -CH=CH-, R₁ is 1-methyl-butyl and the combination of R₂ and R₃ for each compound corresponds to a line B.1 to B.293 of Table B below.
Table 109: Compounds of the formula (I) in which the carbon atom 4' has the configuration *(S)*, A is -C(=O)-, X-Y is -CH=CH-, R₁ is 1-methyl-butyl and the combination of R₂ and R₃ for each compound corresponds to a line B.1 to B.293 of Table B below.
Table 110: Compounds of the formula (I) in which the carbon atom 4' has the configuration *(S)*, A is -C(=S)-, X-Y is -CH=CH-, R₁ is 1-methyl-butyl and the combination of R₂ and R₃ for each compound corresponds to a line B.1 to B.293 of Table B below.
Table 111: Compounds of the formula (I) in which the carbon atom 4' has the configuration *(S),* A is -O-C(=O)-, X-Y is -CH=CH-, R₁ is 1-methyl-butyl and the combination of R₂ and R₃ for each compound corresponds to a line B.1 to B.293 of Table B below.
Table 112: Compounds of the formula (I) in which the carbon atom 4' has the configuration *(S)*, A is -O-C(=S)-, X-Y is -CH=CH-, R₁ is 1-methyl-butyl and the combination of R₂ and R₃ for each compound corresponds to a line B.1 to B.293 of Table B below.
Table 113: Compounds of the formula (I) in which the carbon atom 4' has the configuration *(S),* A is -S-C(=O)-, X-Y is -CH=CH-, R₁ is 1-methyl-butyl and the combination of R₂ and R₃ for each compound corresponds to a line B.1 to B.293 of Table B below.
Table 114: Compounds of the formula (I) in which the carbon atom 4' has the configuration *(S),* A is -S-C(=S)-, X-Y is -CH=CH-, R₁ is 1-methyl-butyl and the combination of R₂ and R₃ for each compound corresponds to a line B.1 to B.293 of Table B below.
Table 115: Compounds of the formula (I) in which the carbon atom 4' has the configuration *(S)*, A is -NR₄-C(=O)-, X-Y is -CH=CH-, R₁ is 1-methyl-butyl and the combination of R₂ and R₃ for each compound corresponds to a line B.1 to B.293 of Table B below.
Table 116: Compounds of the formula (I) in which the carbon atom 4' has the configuration *(S)*, A is -NR₄-C(=S)-, X-Y is -CH=CH-, R₁ is 1-methyl-butyl and the combination of R₂ and R₃ for each compound corresponds to a line B.1 to B.293 of Table B below.
Table 117: Compounds of the formula (I) in which the carbon atom 4' has the configuration *(S)*, A is -SO₂-, X-Y is -CH=CH-, R₁ is 1-methyl-butyl and the combination of R₂ and R₃ for each compound corresponds to a line B.1 to B.293 of Table B below.
Table 118: Compounds of the formula (I) in which the carbon atom 4' has the configuration *(S)*, A is -O-SO₂-, X-Y is -CH=CH-, R₁ is 1-methyl-butyl and the combination of R₂ and R₃ for each compound corresponds to a line B.1 to B.293 of Table B below.
Table 119: Compounds of the formula (I) in which the carbon atom 4' has the configuration *(S)*, A is -NR₄-SO₂-, X-Y is -CH=CH-, R₁ is 1-methyl-butyl and the combination of R₂ and R₃ for each compound corresponds to a line B.1 to B.293 of Table B below.
Table 120: Compounds of the formula (I) in which the carbon atom 4' has the configuration *(S)*, A is a bond, X-Y is -CH=CH-, R₁ is 1-methyl-butyl and the combination of R₂ and R₃ for each compound corresponds to a line B.1 to B.293 of Table B below.
Table 121: Compounds of the formula (I) in which the carbon atom 4' has the configuration *(R)*, A is -C(=O)-, X-Y is -CH₂-CH₂-, R₁ is 1-methyl-butyl and the combination of R₂ and R₃ for each compound corresponds to a line B.1 to B.293 of Table B below.
Table 122: Compounds of the formula (I) in which the carbon atom 4' has the configuration *(R),* A is -C(=S)-, X-Y is -CH₂-CH₂-, R₁ is 1-methyl-butyl and the combination of R₂ and R₃ for each compound corresponds to a line B.1 to B.293 of Table B below.
Table 123: Compounds of the formula (I) in which the carbon atom 4' has the configuration *(R)*, A is -O-C(=O)-, X-Y is -CH₂-CH₂-, R₁ is 1-methyl-butyl and the combination of R₂ and R₃ for each compound corresponds to a line B.1 to B.293 of Table B below.
Table 124: Compounds of the formula (I) in which the carbon atom 4' has the configuration *(R),* A is -O-C(=S)-, X-Y is -CH₂-CH₂-, R₁ is 1-methyl-butyl and the combination of R₂ and R₃ for each compound corresponds to a line B.1 to B.293 of Table B below.
Table 125: Compounds of the formula (I) in which the carbon atom 4' has the configuration *(R)*, A is -S-C(=O)-, X-Y is -CH₂-CH₂-, R₁ is 1-methyl-butyl and the combination of R₂ and R₃ for each compound corresponds to a line B.1 to B.293 of Table B below.
Table 126: Compounds of the formula (I) in which the carbon atom 4' has the configuration *(R)*, A is -S-C(=S)-, X-Y is -CH₂-CH₂-, R₁ is 1-methyl-butyl and the combination of R₂ and R₃ for each compound corresponds to a line B.1 to B.293 of Table B below.
Table 127: Compounds of the formula (I) in which the carbon atom 4' has the configuration *(R)*, A is -NR₄-C(=O)-, X-Y is -CH₂-CH₂-, R₁ is 1-methyl-butyl and the combination of R₂ and R₃ for each compound corresponds to a line B.1 to B.293 of Table B below.
Table 128: Compounds of the formula (I) in which the carbon atom 4' has the configuration *(R)*, A is -NR₄-C(=S)-, X-Y is -CH₂-CH₂-, R₁ is 1-methyl-butyl and the combination of R₂ and R₃ for each compound corresponds to a line B.1 to B.293 of Table B below.
Table 129: Compounds of the formula (I) in which the carbon atom 4' has the configuration *(R)*, A is -SO₂-, X-Y is -CH₂-CH₂-, R₁ is 1-methyl-butyl and the combination of R₂ and R₃ for each compound corresponds to a line B.1 to B.293 of Table B below.
Table 130: Compounds of the formula (I) in which the carbon atom 4' has the configuration *(R),* A is -O-SO₂-, X-Y is -CH₂-CH₂-, R₁ is 1-methyl-butyl and the combination of R₂ and R₃ for each compound corresponds to a line B.1 to B.293 of Table B below.
Table 131: Compounds of the formula (I) in which the carbon atom 4' has the configuration *(R)*, A is -NR₄-SO₂-, X-Y is -CH₂-CH₂-, R₁ is 1-methyl-butyl and the combination of R₂ and R₃ for each compound corresponds to a line B.1 to B.293 of Table B below.
Table 132: Compounds of the formula (I) in which the carbon atom 4' has the configuration *(R),* A is a bond, X-Y is -CH₂-CH₂-, R₁ is 1-methyl-butyl and the combination of R₂ and R₃ for each compound corresponds to a line B.1 to B.293 of Table B below.
Table 133: Compounds of the formula (I) in which the carbon atom 4' has the configuration *(S),* A is -C(=O)-, X-Y is -CH₂-CH₂-, R₁ is 1-methyl-butyl and the combination of R₂ and R₃ for each compound corresponds to a line B.1 to B.293 of Table B below.
Table 134: Compounds of the formula (I) in which the carbon atom 4' has the configuration *(S)*, A is -C(=S)-, X-Y is -CH₂-CH₂-, R₁ is 1-methyl-butyl and the combination of R₂ and R₃ for each compound corresponds to a line B.1 to B.293 of Table B below.
Table 135: Compounds of the formula (I) in which the carbon atom 4' has the configuration *(S)*, A is -O-C(=O)-, X-Y is -CH₂-CH₂-, R₁ is 1-methyl-butyl and the combination of R₂ and R₃ for each compound corresponds to a line B.1 to B.293 of Table B below.
Table 136: Compounds of the formula (I) in which the carbon atom 4' has the configuration *(S),* A is -O-C(=S)-, X-Y is -CH₂-CH₂-, R₁ is 1-methyl-butyl and the combination of R₂ and R₃ for each compound corresponds to a line B.1 to B.293 of Table B below.
Table 137: Compounds of the formula (I) in which the carbon atom 4' has the configuration *(S)*, A is -S-C(=O)-, X-Y is -CH₂-CH₂-, R₁ is 1-methyl-butyl and the combination of R₂ and R₃ for each compound corresponds to a line B.1 to B.293 of Table B below.
Table 138: Compounds of the formula (I) in which the carbon atom 4' has the configuration *(S),* A is -S-C(=S)-, X-Y is -CH₂-CH₂-, R₁ is 1-methyl-butyl and the combination of R₂ and R₃ for each compound corresponds to a line B.1 to B.293 of Table B below.
Table 139: Compounds of the formula (I) in which the carbon atom 4' has the configuration *(S),* A is -NR₄-C(=O)-, X-Y is -CH₂-CH₂-, R₁ is 1-methyl-butyl and the combination of R₂ and R₃ for each compound corresponds to a line B.1 to B.293 of Table B below.
Table 140: Compounds of the formula (I) in which the carbon atom 4' has the configuration *(S)*, A is -NR₄-C(=S)-, X-Y is -CH₂-CH₂-, R₁ is 1-methyl-butyl and the combination of R₂ and R₃ for each compound corresponds to a line B.1 to B.293 of Table B below.
Table 141: Compounds of the formula (I) in which the carbon atom 4' has the configuration *(S),* A is -SO₂-, X-Y is -CH₂-CH₂-, R₁ is 1-methyl-butyl and the combination of R₂ and R₃ for each compound corresponds to a line B.1 to B.293 of Table B below.
Table 142: Compounds of the formula (I) in which the carbon atom 4' has the configuration *(S)*, A is -O-SO₂-, X-Y is -CH₂-CH₂-, R₁ is 1-methyl-butyl and the combination of R₂ and R₃ for each compound corresponds to a line B.1 to B.293 of Table B below.
Table 143: Compounds of the formula (I) in which the carbon atom 4' has the configuration *(S)*, A is -NR₄-SO₂-, X-Y is -CH₂-CH₂-, R₁ is 1-methyl-butyl and the combination of R₂ and R₃ for each compound corresponds to a line B.1 to B.293 of Table B below.
Table 144: Compounds of the formula (I) in which the carbon atom 4' has the configuration (*S*), A is a bond, X-Y is -CH₂-CH₂-, R₁ is 1-methyl-butyl and the combination of R₂ and R₃ for each compound corresponds to a line B.1 to B.293 of Table B below.

**Table B: Compounds of the formula (I)**

| No. | R₂ | R₃ |
|---|---|---|
| B.1 | H | H |
| B.2 | H | Methyl |
| B.3 | H | Ethyl |
| B.4 | H | n-propyl |
| B.5 | H | Iso-propyl |
| B.6 | H | n-butyl |
| B.7 | H | s-butyl |
| B.8 | H | iso-butyl |
| B.9 | H | t-butyl |
| B.10 | H | CH₂=CH-CH₂- |
| B.11 | H | CH₃-CH=CH-CH₂- |
| B.12 | H | HO-CH₂-CH₂- |
| B.13 | H | phenyl |
| B.14 | H | benzyl |
| B.15 | H | 2-Cl-phenyl |
| B.16 | H | 3-Cl-phenyl |
| B.17 | H | 4-Cl-phenyl |
| B.18 | H | 4-F-phenyl |
| B.19 | H | 4-Br-phenyl |
| B.20 | H | 4-NO₂-phenyl |
| B.21 | H | 4-CN-phenyl |
| B.22 | H | 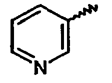 |
| B.23 | H | 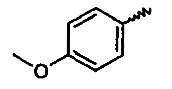 |
| B.24 | H | 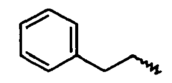 |
| B.25 | H | 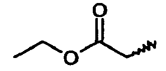 |
| B.26 | H | 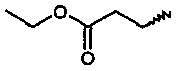 |
| B.27 | H | 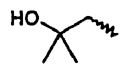 |
| B.28 | H | 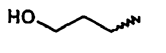 |
| B.29 | H | 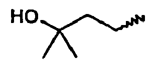 |
| B.30 | H | CH₂=CH- |
| B.31 | H | 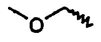 |
| B.32 | H | 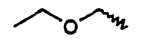 |
| B.33 | H | 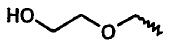 |
| B.34 | H | 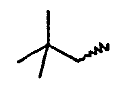 |
| B.35 | H | 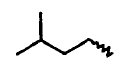 |
| B.36 | H | n-pentyl |
| B.37 | H | n-hexyl |
| B.38 | H | n-heptyl |
| B.39 | H | n-octyl |
| B.40 | H | 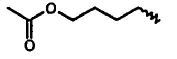 |
| B.41 | H | 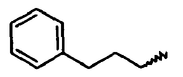 |
| B.42 | H | CF₃CH₂CH₂ |
| B.43 | H | (CH₃)₃SiCH₂ |
| B.44 | H | 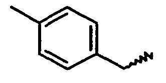 |
| B.45 | H | 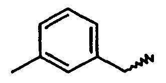 |
| B.46 | H | 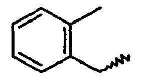 |
| B.47 | H | 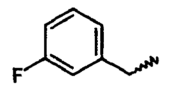 |
| B.48 | H | 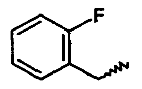 |
| B.49 | H | 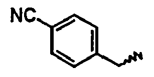 |
| B.50 | H | 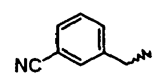 |
| B.51 | H | 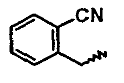 |
| B.52 | H | 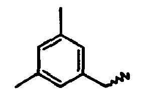 |
| B.53 | H | 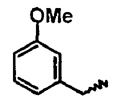 |
| B.54 | H | 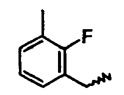 |
| B.55 | H | 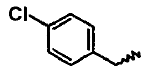 |
| B.56 | H | 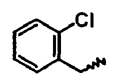 |
| B.57 | H | 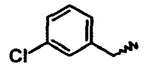 |
| B.58 | H | 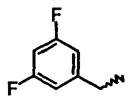 |
| B.59 | H | 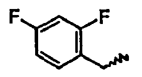 |
| B.60 | H | 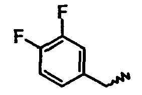 |
| B.61 | H | 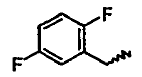 |
| B.62 | H | 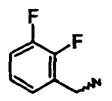 |
| B.63 | H | 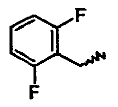 |
| B.64 | H | 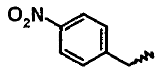 |
| B.65 | H | 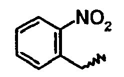 |
| B.66 | H | 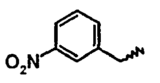 |
| B.67 | H | 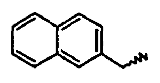 |
| B.68 | H | 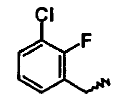 |
| B.69 | H | 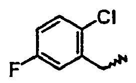 |
| B.70 | H | 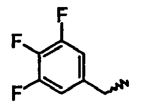 |
| B.71 | H | 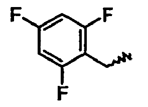 |
| B.72 | H | 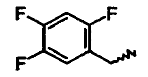 |
| B.73 | H | 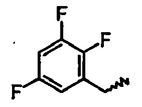 |
| B.74 | H | 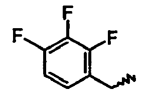 |
| B.75 | H | 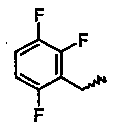 |
| B.76 | H | 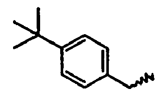 |
| B.77 | H | 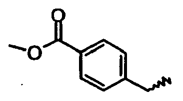 |
| B.78 | H | 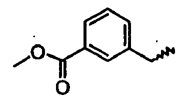 |
| B.79 | H | 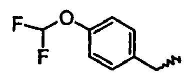 |
| B.80 | H | 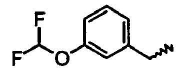 |
| B.81 | H | 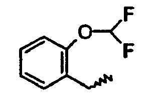 |
| B.82 | H | 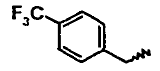 |
| B.83 | H | 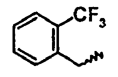 |
| B.84 | H | 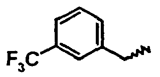 |
| B.85 | H | 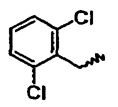 |
| B.86 | H | 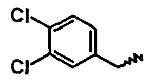 |
| B.87 | H | 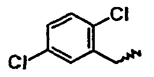 |
| B.88 | H | 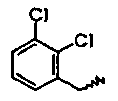 |
| B.89 | H | 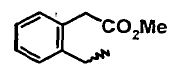 |
| B.90 | H | 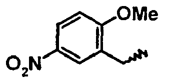 |
| B.91 | H | 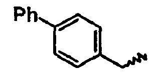 |
| B.92 | H | 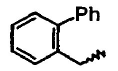 |
| B.93 | H | 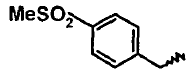 |
| B.94 | H | 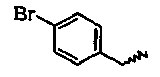 |
| B.95 | H | 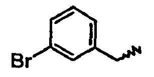 |
| B.96 | H | 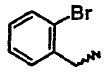 |
| B.97 | H | 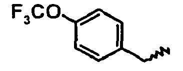 |
| B.98 | H | 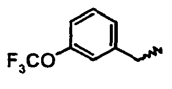 |
| B.99 | H | 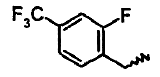 |
| B.100 | H | 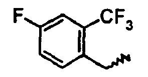 |
| B.101 | H | 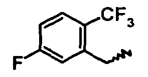 |
| B.102 | H | 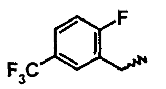 |
| B.103 | H | 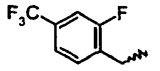 |
| B.104 | H | 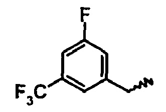 |
| B.105 | H | 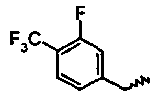 |
| B.106 | H | 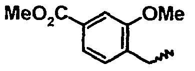 |
| B.107 | H | 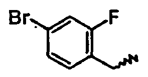 |
| B.108 | H | 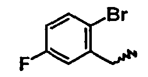 |
| B.109 | H | 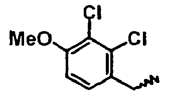 |
| B.110 | H | 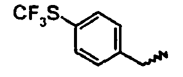 |
| B.111 | H | 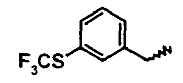 |
| B.112 | H | 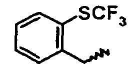 |
| B.113 | H | 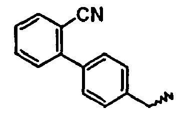 |
| B.114 | H | 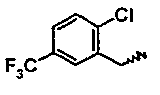 |
| B.115 | H | 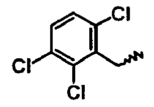 |
| B.116 | H | 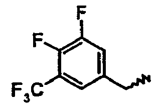 |
| B.117 | H | 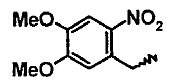 |
| B.118 | H | 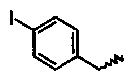 |
| B.119 | H | 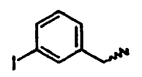 |
| B.120 | H | 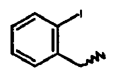 |
| B.121 | H | 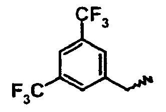 |
| B.122 | H | 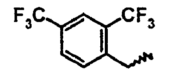 |
| B.123 | H | 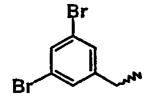 |
| B.124 | H | 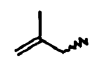 |
| B.125 | H | 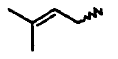 |
| B.126 | H | 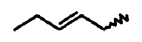 |
| B.127 | H | 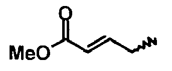 |
| B.128 | H | 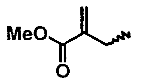 |
| B.129 | H | 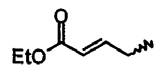 |
| B.130 | H | 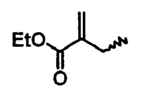 |
| B.131 | H | 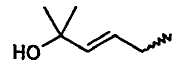 |
| B.132 | H | 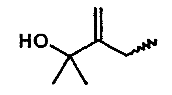 |
| B.133 | H | 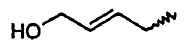 |
| B.134 | H | 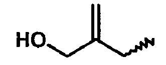 |
| B.135 | H | -CH₂-O-CH₃ |
| B.136 | H | 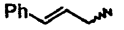 |
| B.137 | H | 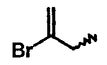 |
| B.138 | H | 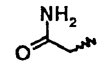 |
| B.139 | H | 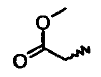 |
| B.140 | H | 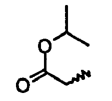 |
| B.141 | H | 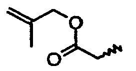 |
| B.142 | H | 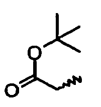 |
| B.143 | H | 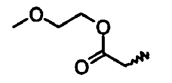 |
| B.144 | H | 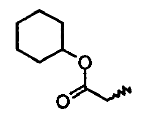 |
| B.145 | H | 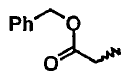 |
| B.146 | H | 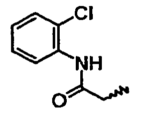 |
| B.147 | H | 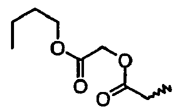 |
| B.148 | H | 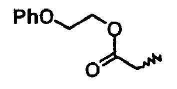 |
| B.149 | H | 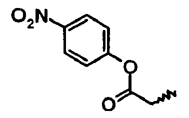 |
| B.150 | H | 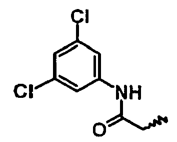 |
| B.151 | H | 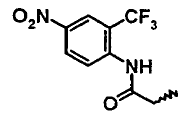 |
| B.152 | methyl | H |
| B.153 | methyl | methyl |
| B.154 | methyl | ethyl |
| B.155 | methyl | n-propyl |
| B.156 | methyl | iso-propyl |
| B.157 | methyl | n-butyl |
| B.158 | methyl | s-butyl |
| B.159 | methyl | iso-butyl |
| B.160 | methyl | t-butyl |
| B.161 | methyl | CH₂=CH-CH₂- |
| B.162 | methyl | CH₃-CH=CH-CH₂- |
| B.163 | methyl | HO-CH₂-CH₂- |
| B.164 | methyl | phenyl |
| B.165 | methyl | benzyl |
| B.166 | methyl | 2-Cl-phenyl |
| B.167 | methyl | 3-Cl-phenyl |
| B.168 | methyl | 4-Cl-phenyl |
| B.169 | methyl | 4-F-phenyl |
| B.170 | methyl | 4-Br-phenyl |
| B.171 | methyl | 4-NO₂-phenyl |
| B.172 | methyl | 4-CN-phenyl |
| B.173 | methyl | 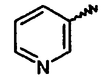 |
| B.174 | methyl | 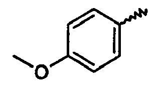 |
| B.175 | methyl | 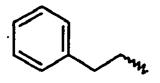 |
| B.176 | methyl | 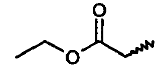 |
| B.177 | methyl | 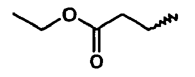 |
| B.178 | methyl | 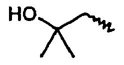 |
| B.179 | methyl | 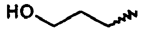 |
| B.180 | methyl | 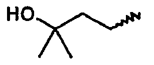 |
| B.181 | methyl | CH₂=CH- |
| B.182 | methyl | 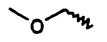 |
| B.183 | methyl | 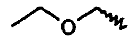 |
| B.184 | methyl | 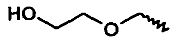 |
| B.185 | methyl | CH=CH₂ |
| B.186 | methyl | (Z)-CH=CHCH₃ |
| B.187 | methyl | OCH₃ |
| B.188 | methyl | C(CH₃)=CH₂ |
| B.189 | methyl | Cyclopropyl |
| B.190 | methyl | N(CH₃)₂ |
| B.191 | methyl | OCH₂CH₃ |
| B.192 | methyl | CH₂OCH₃ |
| B.193 | methyl | CH=C(CH₃)₂ |
| B.194 | methyl | Cyclobutyl |
| B.195 | methyl | OCH₂CH=CH₂ |
| B.196 | methyl | OCH(CH₃)₂ |
| B.197 | methyl | OCH₂CH₂CH₃ |
| B.198 | methyl | SCH₂CH₃ |
| B.199 | methyl | CH₂CH₂Cl |
| B.200 | methyl | 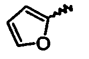 |
| B.201 | methyl | Cyclopentyl |
| B.202 | methyl | CH₂CH₂CH=CH₂ |
| B.203 | methyl | CH₂C(CH₃)₃ |
| B.204 | methyl | CH₂CH₂CH(CH₃)₂ |
| B.205 | methyl | CH(CH₂CH₃)₂ |
| B.206 | methyl | n-pentyl |
| B.207 | methyl | CH₂O(C=O)CH₃ |
| B.208 | methyl | CH₂(C=O)OCH₃ |
| B.209 | methyl | OCH₂CH(CH₃)₂ |
| B.210 | methyl | O-*n*-butyl |
| B.211 | methyl | CH₂CH₂SCH₃ |
| B.212 | methyl | CH₂CH₂CH₂Cl |
| B.213 | methyl | CH₂CH₂Cl |
| B.214 | methyl | N(CH₃)CH₂CH₂CN |
| B.215 | methyl | 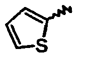 |
| B.216 | methyl | Cyclohexyl |
| B.217 | methyl | CH₂-cyclopentyl |
| B.218 | methyl | 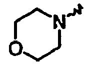 |
| B.219 | methyl | CH₂CO₂CH₂CH₃ |
| B.220 | methyl | CH₂CO₂CH₃ |
| B.221 | methyl | 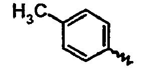 |
| B.222 | methyl | 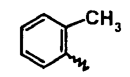 |
| B.223 | methyl | 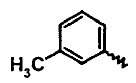 |
| B.224 | methyl | -Ophenyl |
| B.225 | methyl | 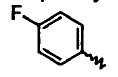 |
| B.226 | methyl | 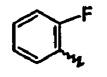 |
| B.227 | methyl | 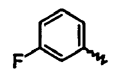 |
| B.228 | methyl | 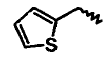 |
| B.229 | methyl | CH₂CH₂-cyclopentyl |
| B.230 | methyl | (CH₂)₂CO₂CH₂CH₃ |
| B.231 | methyl | (CH₂)₃CO₂CH₂CH₃ |
| B.232 | methyl | 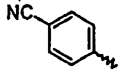 |
| B.233 | methyl | 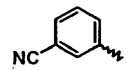 |
| B.234 | methyl | (*E*)-CH=CH-phenyl |
| B.235 | methyl | (*Z*)-CH=CH-phenyl |
| B.236 | methyl | 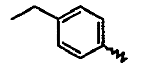 |
| B.237 | methyl | 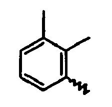 |
| B.238 | methyl | 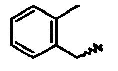 |
| B.239 | methyl | 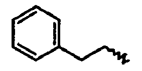 |
| B.240 | methyl | -N(CH₃)-phenyl |
| B.241 | methyl | 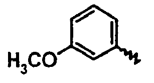 |
| B.242 | methyl | 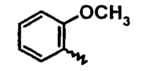 |
| B.243 | methyl | 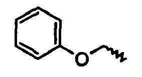 |
| B.244 | methyl | 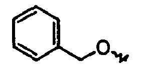 |
| B.245 | methyl | 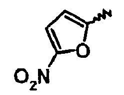 |
| B.246 | methyl | 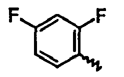 |
| B.247 | methyl | 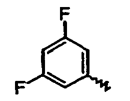 |
| B.248 | methyl | 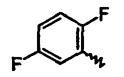 |
| B.249 | methyl | 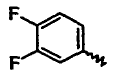 |
| B.250 | methyl | 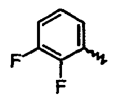 |
| B.251 | methyl | 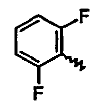 |
| B.252 | methyl | 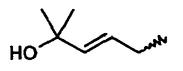 |
| B.253 | methyl | 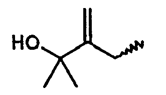 |
| B.254 | methyl | 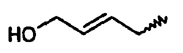 |
| B.255 | methyl | 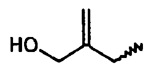 |
| B.256 | methyl | -CH₂-O-CH₃ |
| B.257 | methyl | (CH₂)₄CO₂CH₃ |
| B.258 | =N⁺=N⁻ | |
| B.259 | -CH₂-CH₂-CH₂- | |
| B.260 | -CH₂-CH=CH- | |
| B.261 | 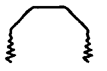 | |
| B.262 | 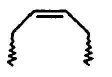 | |
| B.263 | 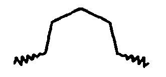 | |
| B.264 | 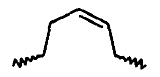 | |
| B.265 | 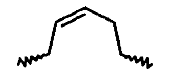 | |
| B.266 | 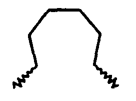 | |
| B.267 | 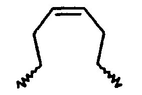 | |
| B.268 | 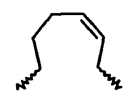 | |
| B.269 | 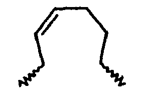 | |
| B.270 | 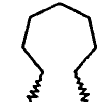 | |
| B.271 | 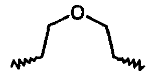 | |
| B.272 | 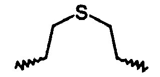 | |
| B.273 | 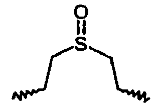 | |
| B.274 | 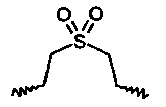 | |
| B.275 | 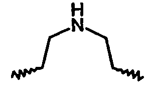 | |
| B.276 | 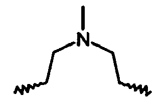 | |
| B.277 | 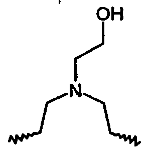 | |
| B.278 | 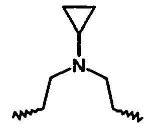 | |
| B.279 | 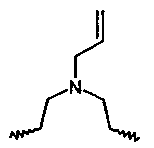 | |
| B.280 | 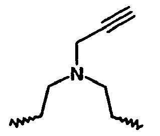 | |
| B.281 | 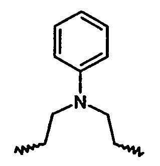 | |
| B.282 | 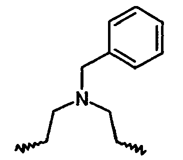 | |
| B.283 | 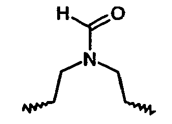 | |
| B.284 | 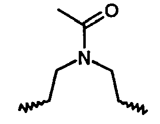 | |
| B.285 | 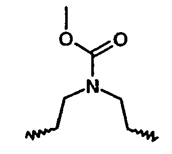 | |
| B.286 | 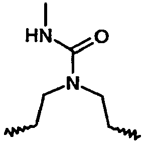 | |
| B.287 | 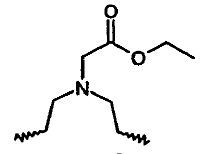 | |
| B.288 | 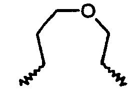 | |
| B.289 | 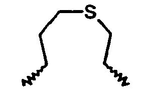 | |
| B.290 | 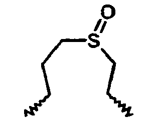 | |
| B.291 | 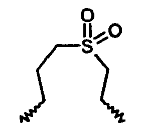 | |
| B.292 | 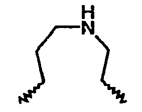 | |
| B.293 | 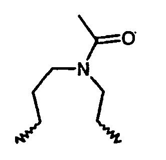 | |

Typical examples of formulation compositions and their preparations are given below:

### Formulation examples for use in crop protection (% = per cent by weight)

| Example F1: Emulsion concentrates | a) | b) | c) |
|---|---|---|---|
| Active compound | 25% | 40% | 50% |
| Calcium dodecylbenzenesulphonate | 5% | 8% | 6% |
| Castor oil polyethylene glycol ether (36 mol of EO) | 5% | - | - |
| Tributylphenol polyethylene glycol ether (30 mol of EO) | - | 12% | 4% |
| Cyclohexanone | - | 15% | 20% |
| Xylene mixture | 65% | 25% | 20% |

Mixing of finely ground active compound and additives gives an emulsion concentrate which, by dilution with water, affords emulsions of the desired concentration.

| Example F2: Solutions | a) | b) | c) | d) |
|---|---|---|---|---|
| Active compound | 80% | 10% | 5% | 95% |
| Ethylene glycol monomethyl ether | - | 20% | - | - |
| Polyethylene glycol (MW 400) | - | 70% | - | - |
| N-methylpyrrolid-2-one | 20% | - | - | - |
| Epoxidized coconut oil | - | - | 1 % | 5% |
| Petroleum ether (boiling range: 160-190°) | - | - | 94% | - |

Mixing of finely ground active compound and additives gives a solution suitable for use in the form of microdrops.

| Example F3: Granules | a) | b) | c) | d) |
|---|---|---|---|---|
| Active compound | 5% | 10% | 8% | 21% |
| Kaolin | 94% | - | 79% | 54% |
| Finely divided silicic acid | 1% | - | 13% | 7% |
| Attapulgite | - | 90% | - | 18% |

The active compound is dissolved in dichloromethane, the solution is sprayed onto the mixture of carriers and the solvent is evaporated under reduced pressure.

| Example F4: Wettable powder | a) | b) | c) |
|---|---|---|---|
| Active compound | 25% | 50% | 75% |
| Sodium lignosulphonate | 5% | 5% | - |
| Sodium lauryl sulphate | 3% | - | 5% |
| Sodium diisobutylnaphthalene sulphonate | - | 6% | 10% |
| Octylphenol polyethylene glycol ether (7-8 mol of EO) | - | 2% | - |
| Finely divided silicic acid | 5% | 10% | 10% |
| Kaolin | 62% | 27% | - |

Active compound and additives are mixed and the mixture is ground in a suitable mill. This gives wettable powders which can be diluted with water to give suspensions of the desired concentration.

| Example F5: Emulsion concentrate | |
|---|---|
| Active compound | 10% |
| Octylphenol polyethylene glycol ether (4-5 mol of EO) | 3% |
| Calcium dodecylbenzenesulphonate | 3% |
| Castor oil polyethylene glycol ether (36 mol of EO) | 4% |
| Cyclohexanone | 30% |
| Xylene mixture | 50% |

Mixing of finely ground active compound and additives gives an emulsion concentrate which, by dilution with water, affords emulsions of the desired concentration.

| Example F6: Extruder granules | |
|---|---|
| Active compound | 10% |
| Sodium lignosulphonate | 2% |
| Carboxymethylcellulose | 1% |
| Kaolin | 87% |

Active compound and additives are mixed, the mixture is ground, moistened with water, extruded and granulated, and the granules are dried in a stream of air.

| Example 7: Coated granules | |
|---|---|
| Active compound | 3% |
| Polyethylene glycol (MW 200) | 3% |
| Kaolin | 94% |

In a mixer, the finely ground active compound is applied uniformly to the kaolin which has been moistened with polyethylene glycol. This gives dust-free coated granules.

| Example F8: Suspension concentrate | |
|---|---|
| Active compound | 40% |
| Ethylene glycol | 10% |
| Nonylphenol polyethylene glycol ether (15 mol of EO) | 6% |
| Sodium lignosulphonate | 10% |
| Carboxymethylcellulose | 1% |
| Aqueous formaldehyde solution (37%) | 0.2% |
| Aqueous silicone oil emulsion (75%) | 0.8% |
| Water | 32% |

Mixing of finely ground active compound and additives gives a suspension concentrate which, by dilution with water, affords suspensions of the desired concentration.

Compounds of formula (I) show good activity against crop pests, in particular compounds A1.1 to A9.58 are more than 80 % effective in biological testing, such as:

### Biological examples:

### Example B1: Activity against Spodoptera littoralis

Young soya bean plants are sprayed with an aqueous emulsion spray liquor which comprises 12.5 ppm of active compound, and, after the spray coating has dried on, populated with 10 caterpillars of the first stage of Spodoptera littoralis and introduced into a plastic container. 3 days later, the reduction in the population in per cent and the reduction in the feeding damage in per cent (% activity) are determined by comparing the number of dead caterpillars and the feeding damage between the treated and the untreated plants.

### Example B2: Activity against Spodoptera littoralis, systemic:

Maize seedlings are placed into the test solution which comprises 12.5 ppm of active compound. After 6 days, the leaves are cut off, placed onto moist filter paper in a Petri dish and populated with 12 to 15 Spodoptera littoralis larvae of the L₁ stage. 4 days later, the reduction of the population in per cent (% activity) is determined by comparing the number of dead caterpillars between the treated and the untreated plants.

### Example B3: Activity against Heliothis virescens

30-35 0- to 24-hour-old eggs of Heliothis virescens are placed onto filter paper in a Petri dish on a layer of synthetic feed. 0.8 ml of the test solution which comprises 12.5 ppm of active compound is then pipetted onto the filter papers. Evaluation is carried out after 6 days. The reduction in the population in per cent (% activity) is determined by comparing the number of dead eggs and larvae on the treated and the untreated filter papers.

### Example B4: Activity against Plutella xylostella caterpillars

Young cabbage plants are sprayed with an aqueous emulsion spray liquor which comprises 12.5 ppm of the active compound. After the spray coating has dried on, the cabbage plants are populated with 10 caterpillars of the first stage of Plutella xylostella and introduced into a plastic container. Evaluation is carried out after 3 days. The reduction in the population in per cent and the reduction in the feeding damage in per cent (% activity) are determined by comparing the number of dead caterpillars and the feeding damage on the treated and the untreated plants.

### Example B5: Activity against Frankliniella occidentalis

In Petri dishes, discs of the leaves of beans are placed onto agar and sprayed with test solution which comprises 12.5 ppm of active compound in a spraying chamber. The leaves are then populated with a mixed population of Frankliniella occidentalis. Evaluation is carried out after 10 days. The reduction in per cent (% activity) is determined by comparing the population on the treated leaves with that of the untreated leaves.

### Example B6: Activity against Diabrotica balteata

Maize seedlings are sprayed with an aqueous emulsion spray liquor which comprises 12.5 ppm of active compound and, after the spray coating has dried on, populated with 10 larvae of the second stage of Diabrotica balteata and then introduced into a plastic container. After 6 days, the reduction in the population in per cent (% activity) is determined by comparing the dead larvae between the treated and the untreated plants.

### Example B7: Activity against Tetranychus urticae

Young bean plants are populated with a mixed population of Tetranychus urticae and, after 1 day, sprayed with an aqueous emulsion spray liquor which comprises 12.5 ppm of active compound, incubated at 25°C for 6 days and then evaluated. The reduction in the population in per cent (% activity) is determined by comparing the number of dead eggs, larvae and adults on the treated and on the untreated plants.

## Claims

1. A compound of the general formula (I) 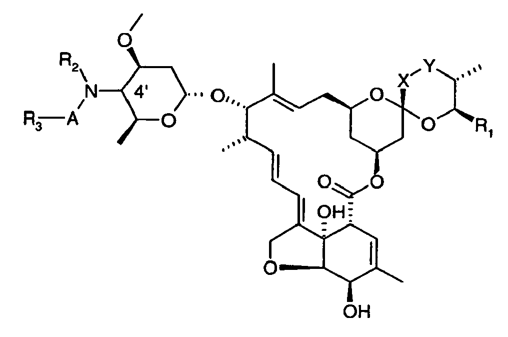 in which
A is -C(=Z)-, -O-C(=Z)-, -S-C(=Z)-, -NR₄-C(=Z)-, -SO₂-, -O-SO₂-, -NR₄-SO₂- or a bond,
X-Y is -CH=CH- or -CH₂-CH₂-;
Z is O or S;
R₁ is C₁-C₁₂alkyl, C₃-C₈cycloalkyl or C₂-C₁₂alkenyl;
R₂ and R₃:
(a) are independently from each other, selected from H, C₁-C₁₂alkyl, C₂-C₁₂alkenyl, C₂-C₁₂alkynyl, C₃-C₁₂cycloalkyl, C₅-C₁₂cycloalkenyl, aryl, heterocyclyl and 2-cyano-2-C₁-C₁₂alkoxyimino; wherein the C₁-C₁₂alkyl, C₂-C₁₂alkenyl, C₂-C₁₂alkynyl, C₃-C₁₂cycloalkyl, C₅-C₁₂cycloalkenyl, aryl, heterocyclyl and C₁-C₁₂alkoxy radicals may be unsubstituted or mono- to pentasubstituted, depending on the substitution possibilities; or
(b) together are a three- to seven membered alkylene or alkenylene bridge, which is unsubstituted or mono to tri-substituted; and wherein, optionally, one of the methylene groups of the three- to seven membered alkylene or alkenylene bridge is replaced by O, NR₄, S, S(=O) or SO₂; or
(c) and A together are =N⁺=N⁻;
R₄ is H, C₁-C₈alkyl, hydroxy-C₁-C₈alkyl, C₃-C₈cycloalkyl, C₂-C₈alkenyl, C₂-C₈alkynyl, phenyl, benzyl -C(=O)R₅, or -CH₂-C(=O)-R₅;
in which the substituents of the alkyl, alkenyl, alkynyl, alkylene, alkenylene, cycloalkyl, cycloalkenyl, aryl, heterocyclyl and alkoxy radicals as defined for R₁, R₂, R₃ and R₄ are selected from the group consisting of OH; =O; halogen; C₁-C₂haloalkyl; -N₃; CN; SCN; NO₂; C₃-C₈cycloalkyl that is unsubstituted or substituted by one to three of any of methyl groups, =O, OH, =S, or SH; norbornylenyl; C₃-C₈halocycloalkyl; C₁-C₁₂alkoxy, which may be substituted with a substituent selected from hydroxy, -N₃, -N(R₈)₂ wherein the two R₈ are independent of each other, and hydroxy; halo-C₁-C₁₂alkoxy; C₃-C₈cycloalkoxy; C₁-C₁₂alkylthio; C₃-C₈cycloalkylthio; C₁-C₁₂haloalkylthio; C₁-C₁₂alkyl-sulfinyl; C₃-C₈cycloalkylsulfinyl; C₁-C₁₂haloalkylsulfinyl; C₃-C₈halocycloalkylsulfinyl; C₁-C₁₂alkylsulfonyl; C₃-C₈cycloalkylsulfonyl; C₁-C₁₂haloalkylsulfonyl; C₃-C₈halocycloalkylsulfonyl; C₂-C₈alkenyl; C₂-C₈alkynyl; -N(R₈)₂, wherein the two R₈ are independent of each other; -C(=O)R₅; -O-C(=O)R₆, -NHC(=O)R₅; -N(CH₃)C(=O)R₅; -S-C(=S)R₆; -P(=O)(OC₁-C₆alkyl)₂; -S(=O)₂R₉; -NH-S(=O)₂R₉; -OC(=O)-C₁-C₆alkyl-S(=O)₂R₉; Si(R₈)₃; aryl; benzyl; heterocyclyl; aryloxy; benzyloxy; heterocyclyloxy; arylthio; benzylthio; and heterocyclylthio; wherein the aryl, heterocyclyl, aryloxy, benzyloxy, heterocyclyloxy, arylthio, benzylthio and heterocyclylthio radicals are unsubstituted or, depending on the possibilities of the substitution on the ring, are mono- to pentasubstituted by substituents selected from the group consisting of OH, halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, dimethylamino-C₁-C₆alkoxy, C₂-C₈alkenyl, C₂-C₈alkynyl, phenoxy, phenyl-C₁-C₆alkyl, methylenedioxy, -C(=O)R₅, -O-C(=O)-R₆, -NH-C(=O)R₆, -N(R₈)₂ wherein the two R₈ are independent of each other, C₁-C₆alkylsulfinyl, C₃-C₈cycloalkylsulfinyl, C₁-C₆haloalkylsulfinyl, C₃-C₈halocycloalkylsulfinyl, C₁-C₆alkylsulfonyl, C₃-C₈cycloalkylsulfonyl, C₁-C₆haloalkylsulfonyl and C₃-C₈halocycloalkylsulfonyl;
R₅ is H, OH, SH, -N(R₈)₂ wherein the two R₈ are independent of each other, C₁-C₂₄alkyl, C₂-C₁₂alkenyl, C₁-C₈hydroxyalkyl, C₁-C₁₂haloalkyl, C₁-C₁₂alkoxy, C₁-C₁₂haloalkoxy, C₁-C₆alkoxy-C₁-C₆alkyl, C₁-C₆alkoxy-C₁-C₆alkoxy, phenoxy-C₁-C₆alkoxy, C₁-C₆alkoxy-C₁-C₆alkoxy-C₁-C₆alkyl, C₁-C₁₂alkylthio, C₂-C₈alkenyloxy, C₂-C₈alkynyloxy, C₁-C₆cycloalkoxy, NH-C₁-C₆alkyl-C(=O)R₇, -N(C₁-C₆alkyl)-C₁-C₆alkyl-C(=O)-R₇, -O-C₁-C₂alkyl-C(=O)R₇, -C₁-C₆alkyl-S(=O)₂R₉, aryl, benzyl, heterocyclyl, aryloxy, benzyloxy, or heterocyclyloxy; or aryl, benzyl, heterocyclyl, aryloxy, benzyloxy or heterocyclyloxy, each of which are mono- to trisubstituted in the ring independently of one another by halogen, nitro, C₁-C₆alkyl, C₁-C₆alkoxy, C₁-C₆haloalkyl or C₁-C₆haloalkoxy;
R₆ is H, C₁-C₂₄alkyl, C₁-C₁₂haloalkyl, C₁-C₁₂hydroxyalkyl, C₂-C₈alkenyl, C₂-C₈alkynyl, C₁-C₆alkoxy-C₁-C₆alkyl, (NR₈)₂ wherein the two R₈ are independent of each other, -C₁-C₆alkyl-C(=O)R₈, -C₁-C₆alkyl-S(=O)₂R₉, aryl, benzyl, and heterocyclyl; or aryl, benzyl or heterocyclyl which, depending on the possibilities of the substitution on the ring, are mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio and C₁-C₁₂haloalkylthio;
R₇ is H, OH, C₁-C₂₄alkyl that is optionally substituted with OH or -S(=O)₂-C₁-C₆alkyl, C₁-C₁₂alkenyl, C₂-C₁₂alkynyl, C₁-C₁₂alkoxy, C₁-C₆alkoxy-C₁-C₆alkyl, C₁-C₆alkoxy-C₁-C₆alkoxy, C₂-C₈alkenyloxy, aryl, aryloxy, benzyloxy, heterocyclyl, heterocyclyloxy or -N(R₈)₂ wherein the two R₈ are independent of each other;
R₈ H, C₁-C₆alkyl that is optionally substituted with one to five substituents selected from the group consisting of halogen, C₁-C₆alkoxy, hydroxy and cyano, C₁-C₈-cycloalkyl, aryl, benzyl or heteroaryl; or aryl, benzyl or heteroaryl, which, depending on the possibilities of the substitution on the ring, are mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio and C₁-C₁₂haloalkylthio; and
R₉ is H, C₁-C₆alkyl that is optionally substituted with one to five substituents selected from the group consisting of halogen, C₁-C₆alkoxy, hydroxy and cyano, aryl, benzyl or heteroaryl; or aryl, benzyl or heteroaryl, which, depending on the possibilities of the substitution on the ring, are mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio and C₁-C₁₂haloalkylthio;
or, if appropriate, an E/Z isomer, E/Z isomer mixture and/or tautomer thereof, in each case in free form or in salt form, provided that R₃ is C₁-C₁₂alkyl, C₂-C₁₂alkenyl, C₂-C₁₂alkynyl, C₃-C₁₂cycloalkyl, C₅-C₁₂cycloalkenyl, aryl, heterocyclyl and 2-cyano-2-C₁-C₁₂alkoxyimino; each of which are unsubstituted or mono- to pentasubstituted, depending on the substitution possibilities, when the compound has a configuration of *(R)* at the 4'-position, X-Y is -CH₂-CH₂-, R₁ is sec-butyl or isopropyl, R₂ is H and A is a bond; or R₃ is H, C₂-C₁₂alkyl, C₂-C₁₂alkenyl, C₂-C₁₂alkynyl, C₃-C₁₂cycloalkyl, C₅-C₁₂cycloalkenyl, aryl, heterocyclyl and 2-cyano-2-C₁-C₁₂alkoxyimino; each of which are unsubstituted or mono- to pentasubstituted, depending on the substitution possibilities, when the compound has a configuration of (R) at the 4'-position, X-Y is -CH₂-CH₂-, R₁ is sec-butyl or isopropyl, R₂ is H and A is -C(=O).

2. A compound according to claim 1 of formula (I) in the free form.

3. A compound according to claim 1 or 2 of formula (I) wherein A is -C(=Z)-.

4. A compound according to anyone of claims 1 to 3 of formula (I) having the *(S)*-configuration at the 4'-position.

5. A pesticidal composition which contains at least one compound of the formula (I) as described in claim 1 as active ingredient and at least one auxiliary.

6. A method for controlling pests wherein a composition as described in claim 5 is applied to the pests or their habitat wherein the method is not a method for the treatment of the human or animal body by surgery or therapy or a diagnostic method practised on the human or animal body.

7. A process for preparing a composition as described in claim 5 which contains at least one auxiliary, wherein the active compound or the active compounds is or are mixed intimately or ground with the auxiliary(s).

8. The use of a composition as described in claim 5 for controlling pests wherein the use is not a method for the treatment of the human or animal body by surgery or therapy or a diagnostic method practised on the human or animal body.

9. A method according to claim 6 for protecting plant propagation material, wherein the propagation material or the location where the propagation material is planted is treated.

10. A pest resistant plant propagation material obtainable, preferably obtained, by the treatment of method claim 9.

## Patentansprüche

1. Verbindung der allgemeinen Formel (I) 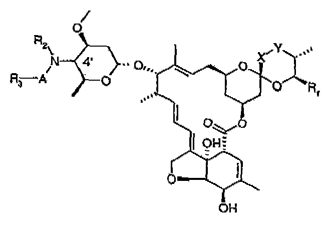 wobei
A für -C(=Z)-, -O-C(=Z)-, -S-C(=Z)-, -NR₄-C(=Z)-, -SO₂-, -O-SO₂-, -NR₄-SO₂- oder eine Bindung steht,
X-Y für -CH=CH- oder -CH₂-CH₂- steht;
Z für O oder S steht;
R₁ für C₁-C₁₂-Alkyl, C₃-C₈-Cycloalkyl oder C₂-C₁₂-Alkenyl steht;
R₂ und R₃:
(a) unabhängig voneinander ausgewählt sind aus H, C₁-C₁₂-Alkyl, C₂-C₁₂-Alkenyl, C₂-C₁₂-Alkinyl, C₃-C₁₂-Cycloalkyl, C₅-C₁₂-Cycloalkenyl, Aryl, Heterocyclyl und 2-Cyano-2-C₁C₁₂-alkoxyimino; wobei die Reste C₁-C₁₂-Alkyl, C₂-C₁₂-Alkenyl, C₂-C₁₂-Alkinyl, C₃-C₁₂-Cycloalkyl, C₅-C₁₂-Cycloalkenyl, Aryl, Heterocyclyl und C₁C₁₂-Alkoxy, abhängig von den Substitutionsmöglichkeiten, unsubstituiert oder mono- bis pentasubstituiert sein können; oder
(b) zusammen für eine drei- bis siebengliedrige Alkylen- oder Alkenylenbrücke stehen, welche unsubstituiert oder mono- bis trisubstituiert ist; und wobei, gegebenenfalls, eine der Methylengruppen der drei- bis siebengliedrigen Alkylen- oder Alkonylenbrücke durch O, NR₄, S, S(=O) oder SO₂ ersetzt ist; oder
(c) und A gemeinsam für =N⁺=N⁻ stehen;
R₄ für H, C₁-C₈-Alkyl, Hydroxy-C₁-C₈-alkyl, C₃-C₈-Cycloalkyl, C₂-C₈-Alkenyl, C₂-C₈-Alkinyl, Phenyl, Benzyl, -C(=O)R₅ oder -CH₂-C(=O)-R₅ steht;
wobei die Substituenten der Reste Alkyl, Alkenyl, Alkinyl, Alkylen, Alkenylen, Cycloalkyl, Cycloalkenyl, Aryl, Heterocyclyl und Alkoxy, wie für R₁, R₂, R₃ und R₄ definiert, ausgewählt sind aus OH; =O; Halogen; C₁-C₂-Halogenalkyl; -N₃; CN; SCN; NO₂; C₃-C₈-Cylcoalkyl, welcher unsubstituiert oder substituiert ist durch eine bis drei aus Methylgruppen, =O, OH, =S oder SH; Norbornylenyl; C₃-C₈-Halogencyaoalkyl; C₁-C₁₂-Alkoxy, welcher substituiert sein kann mit einem Substituenten, ausgewählt aus Hydroxy, -N₃, -N(R₈)₂, wobei die zwei R₈ unabhängig voneinander sind, und Hydroxy; Halogen-C₁-C₁₂-alkoxy; C₃-C₈-Cycloalkoxy; C₁-C₁₂-Alkylthio; C₃-C₈-Cycloalkylthio; C₁-C₁₂-Halogenalkylthio; C₁-C₁₂-Alkylsulfinyl; C₃-C₈-Cycloalkylsulfinyl; C₁-C₁₂-Halogenalkylsulfinyl; C₃-C₈-Halogencyloalkylsulfinyl; C₁-C₁₂-Alkylsulfonyl; C₃-C₈-Cycloalkylsulfonyl; C₁-C₁₂-Halogenalkylsulfonyl; C₃-C₈-Halogencycloalkylsulfonyl; C₂-C₈-Alkenyl; C₂-C₈-Alkinyl; -N(R₈)₂, wobei die zwei R₈ unabhängig voneinander sind; -C(=O)R₅; -O-C(=O)R₆; -NHC(=O)R₅; -N(CH₃)C(=O)R₅; -S-C(=S)R₆; -P(=O)(OC₁-C₆-Alkyl)₂; -S(=O)₂R₉; -NH-S(=O)₂R₉; -OC(=O)-C₁-C₆-Alkyl-S(=O)₂R₉; Si(R₈)₃; Aryl; Benzyl; Heterocyclyl; Aryloxy; Benzyloxy; Heterocyclyloxy; Arylthio; Benzylthio und Heterocyclylthio; wobei die Reste Aryl, Heterocycyl, Aryloxy, Benzyloxy, Heterocyclyloxy, Arylthio, Benzylthio und Heterocyclylthio unsubstituiert sind oder, abhängig von den Möglichkeiten der Substitution am Ring, mono- bis pentasubstituiert sind mit Substituenten, ausgewählt aus OH, Halogen, CN, NO₂, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, Dimethylamino-C₁-C₆-alkoxy, C₂-C₈-Alkenyl, C₂-C₈-Alkinyl, Phenoxy, Phenyl-C₁-C₆-alkyl, Methylendioxy, -C(=O)R₅, -O-C(=O)-R₆, -NH-C(=O)R₆, -N(R₈)₂, wobei die zwei R₈ unabhängig voneinander sind, C₁-C₆-Alkylsulfinyl, C₃-C₈-Cycloalkylsulfinyl, C₁-C₆-Halogenalkylsulfinyl, C₃-C₈-Halogencycloalkylsulfinyl, C₁-C₆-Alkylsulfonyl, C₃-C₈-Cycloalkylsulfonyl, C₁-C₆-Halogenalkylsulfonyl und C₃-C₈-Halogencycloalkylsulfonyl;
R₅ für H, OH, SH, -N(R₈)₂, wobei die zwei R₈ unabhängig voneinander sind, C₁-C₂₄-Alkyl, C₂-C₁₂-Alkenyl, C₁-C₈-Hydroxyalkyl, C₁-C₁₂-Halogenalkyl, C₁-C₁₂-Alkoxy, C₁-C₁₂-Halogenalkoxy, C₁-C₆-Alkoxy-C₁-C₆-alkyl, C₁-C₆-Alkoxy-C₁-C₆-alkoxy, Phenoxy-C₁-C₆-alkoxy, C₁-C₆-Alkoxy-C₁-C₆-alkoxy-C₁-C₆-alkyl, C₁-C₁₂-Alkylthio, C₂-C₈-Alkenyloxy, C₂-C₈-Alkinyloxy, C₁-C₆-Cycloalkoxy, NH-C₁-C₆-Alkyl-C(=O)R₇, -N(C₁-C₆-Alkyl)-C₁-C₆-alkyl-C(=O)-R₇, -O-C₁-C₂-Alkyl-C(=O)R₇, -C₁-C₆-Alkyl-S(=O)₂R₉, Aryl, Benzyl, Heterocyclyl, Aryloxy, Benzyloxy oder Heterocyclyloxy; oder Aryl, Benzyl, Heterocyclyl, Aryloxy, Benzyloxy oder Heterocyclyloxy, von denen jeder unabhängig voneinander am Ring mit Halogen, Nitro, C₁-C₆-Alkyl, C₁-C₆-Alkoxy, C₁-C₆-Halogenalkyl oder C₁-C₆-Halogenalkoxy mono- bis trisubstituiert ist, steht;
R₆ für H, C₁-C₂₄-Alkyl, C₁-C₁₂-Halogenalkyl, C₁-C₁₂-Hydroxyalkyl, C₂-C₈-Alkenyl, C₂-C₈-Alkinyl, C₁-C₆-Alkoxy-C₁-C₆-alkyl, (NR₈)₂, wobei die zwei R₈ unabhängig voneinander sind, -C₁-C₆-Alkyl-C(=O)R₈, -C₁-C₆-Alkyl-S(=O)₂R₉, Aryl, Benzyl und Heterocyclyl; oder Aryl, Benzyl oder Heterocyclyl, welche, abhängig von den Möglichkeiten der Substitution am Ring, mit Substituenten, ausgewählt aus OH, Halogen, CN, NO₂, C₁-C₁₂-Alkyl, C₁-C₁₂-Halogenalkyl, C₁-C₁₂-Alkoxy, C₁-C₁₂-Halogenalkoxy, C₁-C₁₂-Alkylthio und C₁-C₁₂-Halogenalkylthio, mono- bis trisubstituiert sind, steht;
R₇ für H, OH, C₁-C₂₄-Alkyl, welches gegebenenfalls substituiert ist mit OH oder -S(=O)₂-C₁-C₆-Alkyl, C₁-C₁₂-Alkenyl, C₂-C₁₂-Alkinyl, C₁-C₁₂-Alkoxy, C₁-C₆-Alkoxy-C₁-C₆-alkyl, C₁-C₆-Alkoxy-C₁-C₆-alkoxy, C₂-C₈-Alkenyloxy, Aryl, Aryloxy, Benzyloxy, Heterocyclyl, Heterocyclyloxy oder -N(R₈)₂, wobei die zwei R₈ unabhängig voneinander sind, steht;
R₈ für H, C₁-C₆-Alkyl, welcher gegebenenfalls mit einem bis fünf Substituenten, ausgewählt aus Halogen, C₁-C₆-Alkoxy, Hydroxy und Cyano substituiert ist, C₁-C₈-Cycloalkyl, Aryl, Benzyl oder Heteroaryl; oder Aryl, Benzyl oder Heteroaryl, welche, abhängig von den Möglichkeiten der Substitution am Ring, mit Substituenten, ausgewählt aus OH, Halogen, CN, NO₂, C₁-C₁₂-Alkyl, C₁-C₁₂-Halogenalkyl, C₁-C₁₂-Alkoxy, C₁-C₁₂-Halogenalkoxy, C₁-C₁₂-Alkylthio und C₁-C₁₂-Halogenalkylthio, mono- bis trisubstituiert sind, steht; und
R₉ für H, C₁-C₆-Alkyl, welcher gegebenenfalls mit einen bis fünf Substituenten, ausgewählt aus Halogen, C₁-C₆-Alkoxy, Hydroxy und Cyano substituiert ist, Aryl, Benzyl oder Heteroaryl; oder Aryl, Benzyl oder Heteroaryl, welche, abhängig von den Möglichkeiten der Substitution am Ring, mit Substituenten, ausgewählt aus OH, Halogen, CN, NO₂, C₁-C₁₂-Alkyl, C₁-C₁₂-Halogenalkyl, C₁-C₁₂-Alkoxy, C₁-C₁₂-Halogenalkoxy, C₁-C₁₂-Alkylthio und C₁-C₁₂-Halogenalkylthio, mono- bis trisubstituiert sind, steht;
oder, wenn zutreffend, ein E/Z-Isomer, ein E/Z-Isomergemisch und/oder Tautomer davon, jeweils in freier Form oder in Salzform, mit der Maßgabe, dass R₃ für C₁-C₁₂-Alkyl, C₂-C₁₂-Alkenyl, C₂-C₁₂-Alkinyl, C₃-C₁₂-Cycloalkyl, C₅-C₁₂-Cycloalkenyl, Aryl, Heterocyclyl und 2-Cyano-2-C₁-C₁₂-alkoxyimino steht; von denen jeder unsubstituiert oder mono- bis pentasubstituiert ist, abhängig von den Substitutionsmöglichkeiten, wenn die Verbindung eine (*R*)-Konfiguration an der 4'-Position aufweist, X-Y für -CH₂-CH₂- steht, R₁ für sek-Butyl oder Isopropyl steht, R₂ für H steht und A für eine Bindung steht; oder R₃ für H, C₂-C₁₂-Alkyl, C₂-C₁₂-Alkenyl, C₂-C₁₂-Alkinyl, C₃-C₁₂-Cycloalkyl, C₅-C₁₂-Cycloalkenyl, Aryl, Heterocyclyl und 2-Cyano-2-C₁-C₁₂-alkoxyimino steht; von denen jeder unsubstituiert oder mono- bis pentasubstituiert ist, abhängig von den Substitutionsmöglichkeiten, wenn die Verbindung eine (*R*)-Konfiguration an der 4'-Position aufweist, X-Y für -CH₂-CH₂- steht, R₁ für sek-Butyl oder Isopropyl steht, R₂ für H steht und A für -C(=O) steht.

2. Verbindung gemäß Anspruch 1 der Formel (I) in freier Form.

3. Verbindung gemäß Anspruch 1 oder 2 der Formel (I), wobei A für -C(=Z)- steht.

4. Verbindung gemäß einem der Ansprüche 1 bis 3 der Formel (I) mit (*S*)-Konfiguration an der 4'-Position.

5. Schädlingsbekämpfende Zusammensetzung, welche mindestens eine Verbindung der Formel (I) wie in Anspruch 1 beschrieben als wirksamen Bestandteil und mindestens einen Hilfsstoff enthält.

6. Verfahren zur Schädlingsbekämpfung, wobei eine Zusammensetzung wie in Anspruch 5 beschrieben auf Schädlinge oder deren Lebensraum angewendet wird, wobei das Verfahren kein Verfahren zur chirurgischen oder therapeutischen Behandlung des menschlichen oder tierischen Körpers oder ein am menschlichen oder tierischen Körper praktiziertes diagnostisches Verfahren ist.

7. Verfahren zur Herstellung einer Zusammensetzung wie in Anspruch 5 beschrieben, welche mindestens einen Hilfsstoff enthält, wobei die wirksame Verbindung oder die wirksamen Verbindungen innig mit dem/den Hilfsstoff(en) gemischt oder vermahlen werden.

8. Verwendung einer Zusammensetzung wie in Anspruch 5 beschrieben zur Schädlingsbekämpfung, wobei die Verwendung kein Verfahren zur chirurgischen oder therapeutischen Behandlung des menschlichen oder tierischen Körpers oder ein am menschlichen oder tierischen Körper praktiziertes diagnostisches Verfahren ist.

9. Verfahren gemäß Anspruch 6, um Pflanzenvermehrungsgut zu schützen, wobei das Vermehrungsgut oder der Ort, wo das Vermehrungsgut gepflanzt wird, behandelt wird.

10. Schädlingsresistentes Pflanzenvermehrungsgut, erhältlich, vorzugsweise erhalten, durch Behandlung gemäß Verfahrensanspruch 9.

## Revendications

1. Composé de formule générale (I) 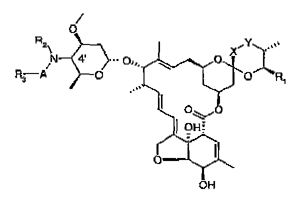 dans laquelle
A représente -C(=Z)-, -O-C(=Z)-, -S-C(=Z)-, -NR₄-C(=Z)-, -SO₂, -O-SO₂-, -NR₄-SO₂- ou une liaison,
X-Y représente -CH=CH- ou -CH₂-CH₂- ;
Z représente O ou S ;
R₁ représente un alkyle en C₁-C₁₂, cycloalkyle en C₃-C₈ ou alcényle en C₂-C₁₂ ;
R₂ et R₃ ;
(a) sont, indépendamment l'un de l'autre, choisis parmi H, alkyle en C₁-C₁₂, alcényle en C₂-C₁₂, alcynyle en C₂-C₁₂, cycloalkyle en C₃-C₁₂, cycloalcényle en C₅-C₁₂, aryle, hétérocyclyle et 2-cyano-2-alcoxyimino en C₁-C₁₂ ; les radicaux alkyle en C₁-C₁₂, alcényle en C₂-C₁₂, alcynyle en C₂-C₁₂, cycloalkyle en C₃-C₁₂, cycloalcényle en C₅-C₁₂, aryle, hétérocyclyle et alcoxy en C₁-C₁₂ pouvant être non substitués ou mono- à penta-substitués, selon les possibilités de substitution ; ou
(b) forment ensemble un pont alkylène ou alcénylène ayant de trois à sept éléments, qui est non substitué ou mono- à tri-substitué ; un des groupements méthylène du pont alkylène ou alcénylène ayant de trois à sept éléments étant éventuellement remplacé par O, NR₄, S, S(=O) ou SO₂ ; ou
(c) forment avec A =N⁺=N⁻ ;
R₄ représente H, un alkyle en C₁-C₈, hydroxy-alkyle en C₁-C₈, cycloalkyle en C₃-C₈, alcényle en C₂-C₈, alcynyle en C₂-C₈, phényle, benzyle, -C(=O)R₅ ou -CH₂-C(=O)-R₅ ;
les substituants des radicaux alkyle, alcényle, alcynyle, alkylène, alcénylène, cycloalkyle, cycloalcényle, aryle, hétérocyclyle et alcoxy tels que définis pour R₁, R₂, R₃ et R₄ étant choisis dans le groupe constitué de OH ; =O, halogène, halogénoalkyle en C₁-C₂, -N₃ ; CN ; SCH ; NO₂ cycloalkyle en C₃-C₈ qui est non substitué ou substitué par un à trois éléments quelconques parmi les groupements méthyle, =O, OH, =S ou SH ; norbomylényle ; halogénocycloalkyle en C₃-C₈ ; alcoxy en C₁-C₁₂, qui peut être substitué par un substituant choisi parmi hydroxy, -N₃, N(R₈)₂, les deux R₈ étant indépendants l'un de l'autre, et hydroxy ; halogénoalcoxy en C₁-C₁₂ ; cycloalcoxy en C₃-C₈; alkylthio en C₁-C₁₂; cycloalkylthio en C₃-C₈ ; halogénoalkylthio en C₁-C₁₂; alkylsulfinyle en C₁-C₁₂; cycloalkylsulfinyle en C₃-C₈ ; halogénoalkylsulfinyle en C₁-C₁₂ ; halogénocycloalkylsulfinyle en C₃-C₈ ; alkylsulfonyle en C₁-C₁₂ ; cycloalkylsulfonyle en C₃-C₈; halogénoalkylsulfonyle en C₁-C₁₂ ; halogénocycloalkylsulfonyle en C₃-C₈; alcényle en C₂-C₈ ; alcynyle en C₂-C₈ ; -N(R₈)₂, les deux R₈ étant indépendants l'un de l'autre ; -C(=O)R₅; -OC(=O)R₆ ; -NHC(=O)R₅ ; -N(CH₃)C(=O)R₅ ; -S-C(=S)R₆; -P(=O)(O-alkyle en C₁-C₆)₂ ; -S(=O)₂R₉ ; -NH-S(=O)₂R₉ ; -OC(=O)-alkyle en C₁-C₆-S(=O)₂R₉ ; Si(R₈)₃ ; aryle ; benzyle ; hétérocyclyle ; aryloxy ; benzyloxy ; hétérocyclyloxy ; arylthio ; benzylthio ; et hétérocyclylthio; les radicaux aryle, hétérocyclyle, aryloxy, benzyloxy, hétérocyclyloxy, arylthio, benzylthio et hétérocyclylthio étant non substitués ou, selon les possibilités de substitution sur le cycle, mono- à penta-substitués par des substituants choisis dans le groupe constitué de OH, halogène, CN, NO₂, alkyle en C₁-C₁₂, cycloalkyle en C₃-C₈ ; halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, alcoxy en C₁-C₆-alkyle en C₁-C₆, diméthylamino-alcoxy en C₁-C₆, alcényle en C₂-C₈, alcynyle en C₂-C₈, phénoxy, phényl-alkyle en C₁-C₆, méthylènedioxy, -C(=O)R₅, -OC(=O)-R₆, -NH-C(=O)R₆, -N(R₈)₂, les deux R₈ étant indépendants l'un de l'autre, alkylsulfinyle en C₁-C₆, cycloalkylsulfinyle en C₃-C₈, halogénoalkylsulfinyle en C₁-C₆, halogénocycloalkylsulfinyle en C₃-C₈, alkylsulfonyle en C_{I}-C₆, cycloalkylsulfonyle en C₃-C₈, halogénoalkylsulfonyle en C₁-C₆ et halogénocycloalkylsulfonyle en C₃-C₈ ;
R₅ représente H, OH, SH, -N(R₈)₂, les deux R₈ étant indépendants l'un de l'autre, alkyle en C₁-C₂₄, alcényle en C₂-C₁₂, hydroxyalkyle en C₁-C₈, halogénolalkyle en C₁-C₁₂, alcoxy en C₁-C₁₂, halogénoalcoxy en C₁-C₁₂, alcoxy en C₁-C₆-alkyle en C₁-C₆, alcoxy en C₁-C₆-alcoxy en C₁-C₆, phénoxy-alcoxy en C₁-C₆ ; alcoxy en C₁-C₆-alcoxy en C₁-C₆-alkyle en C₁-C₆, alkylthio en C₁-C₁₂, alcényloxy en C₂-C₈, alcynyloxy en C₂-C₈, cycloalcoxy en C₁-C₆, NH-alkyle en C₁-C₆-C(=O)R₇, -N(alkyle en C₁-C₆)-alkyle en C₁-C₆-C(=O)-R₇, -O-alkyle en C₁-C₂-C(=O)R₇, alkyle en C₁-C₆-S(=O)₂R₉, aryle, benzyle, hétérocyclyle, aryloxy, benzyloxy ou hétérocyclyloxy ; ou aryle, benzyle, hétérocyclyle, aryloxy, benzyloxy ou hétérocyclyloxy, chacun d'entre eux étant mono- à tri-substitué dans le cycle indépendamment les uns des autres par halogène, nitro, alkyle en C₁-C₆, alcoxy en C₁-C₆, halogénoalkyle en C₁-C₆ ou halogénoalcoxy en C₁-C₆ ;
R₆ représente H, un alkyle en C₁-C₂₄, halogénoalkyle en C₁-C₁₂, hydroxyalkyle en C₁-C₁₂, alcényle en C₂-C₈, alcynyle en C₂-C₈, alcoxy en C₁-C₆-alkyle en C₁-C₆, (NR₈)₂, les deux R₈ étant indépendants l'un de l'autre, alkyle en C₁-C₆-C(=O)R₈, - alkyle en C₁-C₆-S(=O)₂R₉, aryle, benzyle et hétérocyclyle ; ou aryle, benzyle ou hétérocyclyle qui, selon les possibilités de substitution sur le cycle, sont mono- à tri-substitués par des substituants choisis dans le groupe constitué par OH, halogène, CN, NO₂, alkyle en C₁-C₁₂, halogénoalkyle en C₁-C₁₂, alcoxy en C₁-C₁₂, halogénoalcoxy en C₁-C₁₂, alkylthio en C₁-C₁₂ et halogénoalkylthio en C₁-C₁₂ ;
R₇ représente H, OH, alkyle en C₁-C₂₄ qui est éventuellement substitué par OH ou -S(=O)₂-alkyle en C₁-C₆, alcényle en C₁-C₁₂, alcynyle en C₂-C₁₂, alcoxy en C₁-C₁₂, alcoxy en C₁-C₆-alkyle en C₁-C₆, alcoxy en C₁-C₆-alcoxy en C₁-C₆, alcényloxy en C₂-C₈, aryle, aryloxy, benzyloxy, hétérocyclyle, hétéxocyclyloxy ou -N(R₈)₂, les deux R₈ étant indépendants l'un de l'autre ;
R₈ représente H, alkyle en C₁-C₆ qui est éventuellement substitué par un à cinq substituants choisis dans le groupe constitué d'un halogène, alcoxy en C₁-C₆, hydroxy et cyano, cycloalkyle en C₁-C₈, aryle, benzyle ou hétéroaryle ; ou aryle, benzyle ou hétéroaryle, qui, selon les possibilités de substitution sur le cycle, sont mono- à tri-substitués par des substituants choisis dans le groupe constitué de OH, halogène, CN, NO₂, alkyle en C₁-C₁₂, halogénoalkyle en C₁-C₁₂, alcoxy en C₁-C₁₂, halogénoalcoxy en C₁-C₁₂, alkylthio en C₁-C₁₂ et halogénoalkylthio en C₁-C₁₂; et
R₉ représente H, un alkyle en C₁-C₆ qui est éventuellement substitué par un à cinq substituants choisis dans le groupe constitué d'un halogène, alcoxy en C₁-C₆, hydroxy et cyano, aryle, benzyle ou hétéroaryle ; ou aryle, benzyle ou hétéroaryle, qui, selon les possibilités de substitution sur le cycle, sont mono- à tri-substitués par des substituants choisis dans le groupe constitué de OH, halogène, CN, NO₂, alkyle en C₁-C₁₂, halogénoalkyle en C₁-C₁₂, alcoxy en C₁-C₁₂, halogénoalcoxy en C₁-C₁₂, alkylthio en C₁-C₁₂ et halogénoalkylthio en C₁-C₁₂;
ou, le cas échéant, un isomère E/Z, un mélange d'isomères E/Z et/ou leurs tautomères, dans chaque cas sous forme libre ou sous forme de sel, à condition que R₃ représente un alkyle en C₁-C₁₂, alcényle en C₂-C₁₂, alcynyle en C₂-C₁₂, cycloalkyle en C₃-C₁₂, cycloalcényle en C₅-C₁₂, aryle, hétérocyclyle et 2-cyano-2-alcoxyimino en C₁-C₁₂; chacun d'entre eux étant non substitué ou mono- à penta-substitué, selon les possibilités de substitution, lorsque le composé présente une configuration (R) à la position 4', X-Y représente -CH₂-CH₂-, R₁ représente sec-butyle ou isopropyle, R₂ représente H et A est une liaison ; ou R₃ représente H, un alkyle en C₂-C₁₂, alcényle en C₂-C₁₂, alcynyle en C₂-C₁₂, cycloalkyle en C₃-C₁₂, cycloalcényle en C₅-C₁₂, aryle, hétérocyclyle et 2-cyano-2-alcoxyimino en C₁-C₁₂; chacun d'entre eux étant non substitué ou mono- à penta-substitué, selon les possibilités de substitution, lorsque le composé présente une configuration (R) à la position 4', X-Y représente -CH₂-CH₂-, R₁ représente sec-butyle ou isopropyle, R₂ représente H et A représente -C(=O).

2. Composé selon la revendication 1 de formule (I) sous forme libre.

3. Composé selon la revendication 1 ou 2 de formule (I), dans lequel A représente -C(=Z)-.

4. Composé selon l'une quelconque des revendications 1 à 3 de formule (I), présentant la configuration (S) à la position 4'.

5. Composition pesticide contenant au moins un composé de formule (I) selon la revendication 1 en tant qu'ingrédient actif et au moins un auxiliaire.

6. Procédé d'élimination des organismes nuisibles, dans lequel une composition selon la revendication 5 est appliquée aux organismes nuisibles ou à leur habitat, dans lequel le procédé n'est pas un procédé pour le traitement du corps humain ou animal par chirurgie ou thérapie ou un procédé de diagnostic pratiqué sur le corps humain ou animal.

7. Procédé de préparation d'une composition selon la revendication 5, contenant au moins un auxiliaire, dans lequel le composé actif ou les composés actifs est ou sont mélangés intimement ou broyés avec le ou les auxiliaires.

8. Utilisation d'une composition selon la revendication 5 pour éliminer les organismes nuisibles, dans laquelle l'utilisation n'est pas un procédé pour le traitement du corps humain ou animal par chirurgie ou thérapie ou un procédé de diagnostic pratiqué sur le corps humain ou animal.

9. Procédé selon la revendication 6, pour la protection d'un matériau de propagation de plante, dans lequel on traite le matériau de propagation ou l'emplacement où est planté le matériau de propagation.

10. Matériau de propagation de plante résistant aux organismes nuisibles pouvant être obtenu, étant de préférence obtenu par le traitement du procédé selon la revendication 9.
